# EUROPEAN PATENT SPECIFICATION

(11) **EP 3 011 050 B1**
(45) Date of publication and mention of the grant of the patent: **06.09.2017**
(21) Application number: 14738992.8
(22) Date of filing: 19.06.2014
(51) Int. Cl.: C12Q 1/68

(54) **METHODS AND COMPOSITIONS FOR THE DIAGNOSIS, PROGNOSIS AND TREATMENT OF BRAIN METASTASIS**
VERFAHREN UND ZUSAMMENSETZUNGEN ZUR DIAGNOSE, PROGNOSE UND BEHANDLUNG VON HIRNMETASTASEN
MÉTHODES ET COMPOSITIONS POUR LE DIAGNOSTIC, LE PRONOSTIC ET LE TRAITEMENT DE MÉTASTASES CÉRÉBRALES

(30) Priority: 19.06.2013 US 201361836993 P
(43) Date of publication of application: 27.04.2016
(73) Proprietor: Memorial Sloan Kettering Cancer Center, New York, NY 10065 (US)
(72) Inventor: SEVENICH, Lisa Katharina Hildegard, New York, New York 10065 (US); JOYCE, Johanna Alexandra, New York, New York 10065 (US)
(74) Representative: Schlich, George
(86) International application number: PCT/US2014/043291
(87) International publication number: WO 2014/205293

(56) References cited:
- WO-A2-2008/030845
- BOS PAULA D ET AL: "Genes that mediate breast cancer metastasis to the brain", NATURE (LONDON), vol. 459, no. 7249, June 2009 (2009-06), page 1005, XP002732123, ISSN: 0028-0836
- BURTON ET AL: "Prediction of Breast Cancer Metastasis by Gene Expression Profiles: A Comparison of Metagenes and Single Genes", CANCER INFORMATICS, 1 December 2012 (2012-12-01), page 193, XP55150982, DOI: 10.4137/CIN.S10375
- MINN ANDY J ET AL: "Genes that mediate breast cancer metastasis to lung", NATURE, NATURE PUBLISHING GROUP, UNITED KINGDOM, vol. 436, no. 7050, 28 July 2005 (2005-07-28), pages 518-524, XP002454668, ISSN: 0028-0836, DOI: 10.1038/NATURE03799
- SMID MARCEL ET AL: "Genes associated with breast cancer metastatic to bone", UNIX REVIEW, SAN FRANCISCO, CA, US, vol. 24, no. 15, 20 May 2006 (2006-05-20), pages 2261-2267, XP002454667,
- PETTY R D ET AL: "Tumor transcriptome reveals the predictive and prognostic impact of lysosomal protease inhibitors in non-small-cell lung cancer", JOURNAL OF CLINICAL ONCOLOGY 20060410 US, vol. 24, no. 11, 10 April 2006 (2006-04-10), pages 1729-1744, XP002732124, ISSN: 0732-183X
- ROBERTA E BURDEN ET AL: "Inhibition of Cathepsin S by Fsn0503 enhances the efficacy of chemotherapy in colorectal carcinomas", BIOCHIMIE, MASSON, PARIS, FR, vol. 94, no. 2, 24 August 2011 (2011-08-24), pages 487-493, XP028441202, ISSN: 0300-9084, DOI: 10.1016/J.BIOCHI.2011.08.017 [retrieved on 2011-08-31]
- YANG YIXUAN ET AL: "Cathepsin S Mediates Gastric Cancer Cell Migration and Invasion via a Putative Network of Metastasis-Associated Proteins", JOURNAL OF PROTEOME RESEARCH, vol. 9, no. 9, September 2010 (2010-09), pages 4767-4778, XP002732125, ISSN: 1535-3893
- SEVENICH LISA ET AL: "Analysis of tumour-and stroma-supplied proteolytic networks reveals a brain-metastasis-promoting role for cathepsin S", NATURE CELL BIOLOGY, vol. 16, no. 9, September 2014 (2014-09), pages 876-888, XP002732126,

## Description

### Federally-Sponsored Research or Development

This invention was made with Government support under contract CA148967 and CA126518 awarded by the National Cancer Institute/ National Institutes of Health. The U.S. Government has certain rights in the invention.

### Field of the Invention

The invention described herein relates to methods useful in the diagnosis, treatment and management of cancers. In particular, the present invention relates to predicting the likelihood of metastasis of a cancer to the brain, bone and/or lung and its impact on metastasis-free survival.

### Background of the Invention

After cardiovascular disease, cancer is the leading cause of death in the developed world. In the United States alone, over one million people are diagnosed with cancer each year, and over 500,000 people die each year as a result of it. It is estimated that 1 in 3 Americans will develop cancer during their lifetime, and one in five will die from cancer. Further, it is predicted that cancer may surpass cardiovascular diseases as the number one cause of death within 5 years. As such, considerable efforts are directed at improving treatment and diagnosis of this disease.

Most cancer patients are not killed by their primary tumor. They succumb instead to metastases: multiple widespread tumor colonies established by malignant cells that detach themselves from the original tumor and travel through the body, often to distant sites.

Cancer cells in an aggressive primary tumor are adept in exploiting that particular local tissue microenvironment. In contrast, when metastatic cells leave these favorable surroundings, they must possess or acquire traits that will allow them to survive and colonize foreign, potentially hostile tissue environments. The obstacles that metastasizing tumor cells encounter vary from organ to organ, and are influenced by non-cancerous stromal cells of the tumor microenvironment. For example, the blood-brain barrier, composed of endothelial cells, astrocytes and pericytes, presents a far more formidable structure for tumor cells to penetrate, compared to the fenestrated capillaries in the bone marrow. Tumor cells with the capacity to extravasate and seed these different tissue microenvironments then encounter distinct cell types, often with specialized functions, that can positively or negatively regulate subsequent metastatic outgrowth. Indeed, dissemination can occur to multiple organs, yet metastatic tumors may grow in only one or a few sites, indicating critical roles for the microenvironment in this process.

Clinical management of cancer can be aided by prognosis markers and by therapeutic predictive markers. Prognosis markers assess risk of the disease progression independent of therapy. Therapeutic predictive markers indicate sensibility or resistance of a cancer to a specific treatment. For most cancers and cancer treatments, there exist subsets of patients that will respond to a particular treatment and subsets of patients that will fail to respond to the treatment.

The use of therapeutic predictive markers to identify subsets of patients likely to respond to treatment would facilitate the selection of the appropriate treatment and avoid unnecessary delays associated with ineffective treatment. Additionally, because most cancer treatments are associated with adverse side effects inherent to the treatment, said predictive markers eliminate unnecessary risks of adverse side effects by reducing the administration of cancer treatments to individuals for whom treatment is likely to fail.

Metastasis is a complex series of steps in which neoplasic cells leave the original tumor site and migrate to other parts of the body via the blood stream or the lymphatic system and start new tumors that resemble the primary tumor. Breast cancer cells are often transported through the lymphatic pathway to bone or other areas such as liver, lung or brain. It may be life saving to predict whether a primary cancer has the potential to metastasize such that high risk patients can be subject to closer follow up or specific treatment regime that will vary where the cancer has metastasized. Currently there is a need in the art for new and improved means by which to identify when a primary turmor, for example a breast cancer, is going to metastasize and how one can inhibit the metastasis from the primary tumor to, for example, the brain, bone or lung of the patient.

Breast cancer is the most common cancer, and the second leading cause of cancer death, among women in the western world. It is the most common cancer in women and makes up a third of cancer occurrence of women in the US. Common tests that provide information to assists in the diagnosis or prognosis of breast cancer include mammograms and tissue biopsy followed by combinations of histological examination, immune-histochemical detection with antibodies to estrogen receptor (ER), progesterone receptor (PR) and/or HER2/neu proteins.

Currently, the recommended therapeutic predictive markers in oncology are ER (estrogen receptor) and PR (progesterone receptor) status for selecting hormone sensitive breast cancers, and HERB-2 for identifying breast cancer patients who may benefit from trastuzumab treatment.

The incidence of brain metastasis in patients with breast cancer overexpressing HERB-2 treated with tratuzumab is twice that in other breast cancer patients. On the other hand, one-third of the patients with breast cancer will develop CNS metastasis and this often occurs when they are responding to therapy at other sites or have a stable disease. Thus, drugs with a high impact on the clinical outcome of metastatic breast cancer patients, such as taxanes or trastuzumab, play only a limited role in the treatment of brain metastasis.

Cerebral metastases occur in 10-15% of breast cancer patients with advanced disease and have recently become a significant clinical problem. It can be assumed that up to 30% of metastatic breast cancer patients will experience brain metastasis during the course of their disease. The increase in this rate could be linked to greater survival in patients receiving chemotherapy and the fact that it is difficult to overcome the blood brain barrier (BBB) with current systemic treatments. The difficulties in managing brain metastasis therapy result in a median survival of seven months, with brain metastasis being the cause of death or a major contributing factor of it in 68% of patients.

An adequate estimation of independent predictive factors at initial tumor diagnosis is required to enable the clinician to determine whether said tumor can metastasize. This information would be useful for the clinician in order to decide between aggressive treatments, to avoid unnecessary treatment, and to design therapies specifically addressed against differential aspects of each metastatic location. Therefore, there is the need of predictive markers which provides information about the risk of metastasizing a primary tumor to other organs in order to treat efficiently the illness.

A number of strategies have been used to investigate the constituent cell types of different tumor microenvironments, predominantly in primary tumors, including cell sorting or laser capture microdissection followed by mRNA or miRNA expression profiling. These approaches have led to the identification of expression signatures for tumor-associated macrophages, endothelial cells, fibroblasts, Tie2-expressing monocytes, and astrocytes among others. While these studies have been informative in identifying stromal gene signatures, often with prognostic value, they involved manipulation of the tumor to isolate individual cell types, and in most cases the stromal cells were isolated in isolation, without comparative expression information for the tumor cells. Thus, this information is not as informative as one would desire. Accordingly, there is also a need in the art to understand the interplay between cancer cells and the microenvironment in intact tumors at different stages of metastatic seeding and outgrowth, and for better compositions and methods that relate to the manipulation of the metastatic seeding and outgrowth process.
Bos, P et al in "Genes that mediate breast cancer metastasis to the brain", Nature, vol. 459, no. 7249, 18 June 2009 indicated that the molecular basis for breast cancer metastasis to the brain was largely unknown. Brain metastasis to the brain may occur years after the removal of the primary breast tumor, suggesting that free cancer cells must acquire specalizied functions to take over this organ. Gene expression analysis identified the cyclooxygenase COX2 (also known as PTGS2), the epidermal growth factor receptor (EGFR) ligand HBEGF, and the α2,6-sialyltransferase ST6GALNAC5 as mediators of cancer cell passage through the blood-brain barrier. However, this did not result in a method for predicting the likelihood that a patient with breast cancer will develop metastasis to the brain.
Burton et al in "Prediction of Breast Cancer Metastasis by Gene Expression profiles; A comparision of Metagenes and Single Genes", Cancer Informatics, 1 December 2012, p. 193 suggestes that while predictive or prognostic gene expression profiles have been delveloped, the have not generally beeb full validated in independent datasets. From a systematic comparision of metagene (MG) and individual gene (SG) features, prediction of metastasis outcome in lymph node - negative patients by MG- and SG- classifiers showed that SG-classifiers performed significantly better.
WO 2008/030845 in the name of Veridex LLC, describes a method for prediciting distant metastasis of lymph node negative primary breast cancer by obtaining breast cancer cells; isolating nucleic acid and/or protein from the cells; and analyzing the nucleic acid and/or protein to determine the presence, expression lever or status of a Biomarker selected from the top 20 over-represented pathways that have the highest frequencies in the 500 signatures of oestrogen receptor (ER)-positive and ER-negative tumors.
Burden, RE et al in "Inhibition of Cathepsin S by Fsn0503 enhances the efficacy of chemotherapy in colorectal carcinomas", Biochemie, vol. 94, no. 2, 24 August 2011, pages 487 - 493 noted that Capthepsin S is a lysomal cysteine protease implicated in tumorigenesis and in particular in invasion and angiogenesis. A monoclonal antibody (Fsn0503) reduced colorectal carcinoma tumor gowth and angiogenesis by specific inhibition of Cathepsin S. Burden further found that Cathepsin S expression levels are upregulated in HCT116, LoVo, Colo205 cell lines and HUVECs after exposure to CPT-11 in vitro, and the administration of Fsn0503 in combination with CPT-11 significantly attenuated tumour growth in comparison to CPT-11 alone in colorectal HCT116 xenograft models. Additionally tumour vascularisation revealed that this was also significantly disrupted by the combination treatment, indicating that the combination of inhibition of Cathepsin S and use of CPT-11 enhances the therapeutic effect.

### Summary of the Invention

The inventors of the instant application set out to specifically analyze the interplay between cancer cells and the microenvironment in intact tumors at different stages of metastatic seeding and outgrowth. The inventors investigated breast cancer cell interactions with the stroma in three organ sites to which these cells commonly metastasize: the lung, bone and brain.

One aspect of the present disclosure is directed to a method of predicting the likelihood that a patient with breast cancer will develop metastasis to the brain, bone and/or lung, said method comprising: (a) detecting in a sample from the subject the level of expression of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, GDI2, SERPINE2, TPSG1, PSMD2* and *PSME1;* and

### (b)

(i) predicting that the subject will develop metastasis to the brain, bone and lung if expression of *SLPI* is increased over control;
(ii) predicting that the subject will develop metastasis to brain and bone if expression of *PSMD11* is increased over control;
(iii) predicting that the subject will develop metastasis to brain and lung if expression of one or more of *SERPINB3, PI3, ADAMDEC1, ILF2, PSMB4, APP, S100A10, CTSC, CTSL1, CANX, ANXA5, PSMD2* and *CTSB* is increased over control, but will not develop metastasis to the brain if *TPSG1* is increased over control;
(iv) predicting that the subject will develop metastasis to bone and lung if expression of one or more of *MME, PSMB3,* and *PSMD10* is increased over control;
(v) predicting that the subject will develop metastasis to brain only if expression of one or more of *SPOCK2, PSMB6, PRSS22, CTSS, KLK10, GZMK, ELANE, COX4I1,* and *TIMP2* is increased over control, but will not develop metastasis to the brain if *HNRPNPC* and/or *SEPT2* is increased over control;
(vi) predicting that the subject will develop metastasis to bone if expression of *SNRNP200* is increased over control, but will not develop metastasis to the bone if one or more of *EIF3F, RPS6, GZMB, RPS13, RPS10, RPL21, RPL30, OAZ1, SerpinF2, RPL27, PRTN3, RPS5, F2, RPL14, PSMD13, RPL28, RPS27A, TIMP1, RPS11, USP4, 1RPS24, CELA2B,* and *RPL11* is increased over control;
(vii) predicting that the subject will develop metastasis to lung only if expression of one or more of *PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, PSMB7, PSMC1, ILF2, PSMD1, GDI2,* and *SERPINE2* is increased over control, but will not develop metastasis to the lung if one or more of *SPINK4, ANXA9, PLAT, MMP24, CST3, EEF2, F7, F10, RPL9, PRSS23, MMP26,* and *HTRA1* is increased over control.

In one embodiment, the sample to be interrogated for gene expression is a cell or tissue sample from the primary tumor or bodily fluid which may contain tumor cells. Another aspect of the present disclosure is directed to a method of determining the risk that a subject with breast cancer will develop metastasis of said cancer to the brain, said method comprising:
(a) determining in a sample from the subject a level of expression of genes SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2 and CTSB; and (b) predicting that the subject will develop metastasis to the brain if expression of one or more of said genes is increased over control.

Another aspect of the present disclosure is directed to method of determining the risk that a subject with breast cancer will develop metastasis of said cancer to the bone, said method comprising:
(a) determining in a sample from the subject the level of expression of genes MME, PSMB3, SNRNP200, SLPI, PSMD10 and PSMD11; and
(b) predicting that the subject will develop metastasis to the bone if expression of one or more of said genes is increased over control.

Another apect of the present disclosure is directed to a method of determining the risk that a subject with breast cancer will develop metastasis to the lung, said method comprising:
(a) determining in a sample from the subject the level of expression of genes MME, SERPINB3, PSMB3, PI3, PSMA7, TMPRSS5, F12, PSMA6, SPINK2, PSMA3, ADAM9, PLAU, CAPN3, ZNF146, ANXA1, PSMC2, COPS7B, PSMB5, CTSB, PSMD10, PSMC3, ANXA3, PSMA4, ADAMDEC1, USP1, PSMB4, KIFAP3, PSMD4, HSP90AB1, PCSK1N, APP, CANX, CSTB, PSMB7, PSMC1, ILF2, ANXA5, PSMD1, CTSC, GDI2, CTSL1, SERPINE2, SLPI, PSMD2, and S100A10; and
(b) predicting that the subject will develop metastasis to the lung if expression of one or more of said genes is increased over control

Another unclaimed aspect of the present disclosure is directed to a method of predicting metastasis of breast cancer to the brain, bone and/or lung of a patient suffering from breast cancer, said method comprising: isolating a sample from the patient; analyzing the sample for the increased expression of cathepsin S gene; and (i) predicting the breast cancer patient has or is at risk of developing metastasis to the brain if there is increased expression of cathepsin S gene in tumor cells early on in brain metastasis development, relative to control; and/or (ii) wherein, increased expression of cathepsin S gene in tumor cells early on in brain metastasis development, relative to control, does not correlate with metastasis of the patient's breast cancer to the patient's bone or lung.

One unclaimed aspect of the present disclosure is directed to a method of treating, preventing or managing metastasis of cancer cells from a primary tumor in a cancer patient to the patient's brain, said method comprising: administering to said patient an agent which inhibits cathepsin S. In one embodiment, the primary cancer is breast cancer. In another embodiment, the agent is a selective inhibitor of cathepsin S. In a particular embodiment, the agent is a specific inhibitor of cathepsin S. The agent is, in one example, a peptide-based inhibitor of cathepsin S, which is based upon a peptide sequence which comprises 2-20 consecutive residues of a preferred invariant chain cleavage site of cathepsin S. In one embodiment, the agent is administered to the patient suffering from cancer via intravenous injection, intradermal injection, subcutaneous injection, intramuscular injection, intraperitoneal injection, anal supposition, vaginal supposition, oral ingestion or inhalation.

One or more cathepsin S inhibitors are, in one example, administered early on in the metastasis development cascade. In one embodiment, the peptide-based inhibitor of cathepsin S is morpholinurea-leucine-homophenyl alanine-vinylsulfone phenyl (LHVS). In one embodiment, the peptide-based inhibitor is a peptide-based vinylsulfone or a modified peptide-based vinylsulfone. In another embodiment, the peptide-based inhibitor is selected from peptidyl aldehydes, nitriles, α-ketocarbonyls, halomethyl ketones, diazomethyl ketones, (acyloxy)-methyl ketones, vinyl sulfones, ketomethylsulfonium salts, epoxides, and N-peptidyl-O-acyl-hydroxylamines. In another embodiment, the agent is selected from Asn-Leu-vinylsulfone, Arg-Met-vinylsulfone, Leu-Arg-Met-vinylsulfone, Glu-Asn-Leu-vinylsulfone, and Leu-Leu-Leu-vinylsulfone. In one embodiment, the agent is selected from N-(carboxybenzyl)-Asn-Leu-vinylsulfone, N-(carboxybenzyl)-Arg-Met-vinylsulfone, N-(carboxybenzyl)-Leu-Arg-Met-vinylsulfone, N-(carboxybenzyl)-Glu-Asn-Leu-vinylsulfone, and N-(carboxybenzyl)-Leu-Leu-Leu-vinylsulfone.

Another unclaimed aspect of the present disclosure is directed to a method of treating, preventing or managing cancer cell metastasis in a cancer patient, comprising: extracting a sample from the primary tumor, metastatic tumor, or blood of the cancer patient; assaying the sample to determine the expression of cathepsin S and/ or *PSMB6* genes in said sample; and administering a cathepsin S inhibitor if the expression of cathepsin S and/ or *PSMB6* genes is increased over control.

One unclaimed aspect of the present disclosure is directed to a method for preparing a personalized genomics profile for a patient with breast cancer, comprising: extracting mononuclear cells or cancer cells from the primary tumor and subjecting them to gene expression analysis; assaying the sample to determine the expression of cathepsin S and *PSMB6* in said sample; and generating a report of the data obtained by the expression analysis, wherein the report comprises a prediction of the likelihood of the patient being substantially free of metastasis to the brain if, in addition to decreased expression of cathepsin S in the sample over control, expression of *PSMB6* gene is also decreased over control. In one embodiment, the method further comprises predicting that the patient with cancer will develop metastasis to the bone if in the sample over control, expression of *PSMD11* or *SLPI* gene is increased over control.

In one unclaimed aspect, the present disclosure is a kit for determining treatment of a patient with brain metastasis, the kit comprising means for detecting expression and/or activity of cathepsin S and/or *PSMB6* genes at an early stage of brain metastasis; and instructions for recommended treatment based on the presence of increased expression or activity in cathepsin S and/or *PSMB6* genes.

One unclaimed aspect of the present disclosure is a method of analyzing a cell expression profile for determining whether the cell is metastatic to the brain or bone, said method comprising the steps of: (a) extracting the cell; (b) measuring an amount of cathepsin S, *PSMB6, PSMD11* or *SLPI* nucleic acid expression or polypeptide in the cell; and (c) comparing the amount of cathepsin S, *PSMB6, PSMD11* or *SLPI* nucleic acid expression or protein present in the cell to the amount of cathepsin S, *PSMB6, PSMD11* or *SLPI* nucleic acid expression or polypeptide in a sample isolated from normal, non-cancerous cells, wherein: (i) an amplified amount of cathepsin S and *PSMB6* nucleic acid expression or polypeptide in the cell relative to the amount of cathepsin S and *PSMB6* nucleic acid expression or polypeptide in the sample isolated from normal, non-cancerous cells indicates that cancer is likely to metastasize to the brain, and/or (ii) an amplified amount of *PSMD11* and *SLPI* nucleic acid expression or polypeptide in the cell relative to the amount of PSMD11 and *SLPI* nucleic acid expression or polypeptide in the sample isolated from normal, non-cancerous cells indicates that cancer is likely to metastasize to the bone.

In one embodiment, the cell is isolated from the patient's blood, or primary tumor. In another embodiment, the cell is isolated from a primary breast tumor.

In one aspect, the present invention is directed to a kit for determining in a sample from a breast cancer subject expression levels of genes indicative of metastasis of cancer in the subject to brain, bone or lung, the kit comprising one or more components for determining the expression levels of said genes, wherein said one or more components are selected from the group consisting of: a DNA array chip, an oligonucleotide array chip, a protein array chip, an antibody, a plurality of probes; and a set of primers consisting of primers for genes, *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPIN3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1 PCSK1N, CSTB, POM87, PSMC1, PSMD1, CTSC, GDI2, SERPINE2, PSMD2* and *PSME1*

In one aspect, the invention relates to use of a kit of the invention for determining the risk of metastasis of breast cancer to the brain in a breast cancer patient.

In another aspect, a kit of the invention further comprises one or more reagents for RNA extraction; one or more enzymes for syntheses of cDNA and cRNA; one or more reagents for hybridization for DNA chip, oligonucleotide chip, protein chip, western blot, probes, or primers; one or more reagents for binding of said antibodies to proteins indicative of recurrence of cancer; or DNA fragments of control genes.

In another aspect, a kit of the invention further includes instructions for determining the likelihood of metastasis-free survival for a patient based on the expression levels of the genes indicative of cancer metastasis.

In another aspect, the invention relates to a set of primers consisting of at least one primer pair for each of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, CSTB, PSMB7, PSMC1, PSMD1, GDI2, SERPINE2, PSMD2* and *PSME1.*

In another aspect, the invention relates to an array consisting of a substrate or solid support and at least one probe for each of genes *SERPINB3, PI3, SPOCK2, PSMB6, PRSS22, CTSS, KLK10, GZMK, ADAMDEC1, ELANE, ILF2, PSMD11, PSMB4, S100A10*, *APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, CSTB, PSMB7, PSMC1, PSMD1, GDI2, SERPINE2, PSMD2* and *PSME1.*
Another aspect of the invention is directed to method of predicting the likelihood of metastasis-free survival (MFS) of a subject with breast cancer, the method comprising:
(a) detecting the level of expression of genes SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, TPSG1, PSMD2 and PSME1 in a sample from the subject; and
(b) predicting decreased likelihood of metastasis-free survival if any of said genes is increased over control.
In some embodiments, the method further comprising normalizing said expression levels to obtain a normalized expression level of said genes, wherein an increased normalized expression level of at least one of said genes indicates a decreased likelihood of metastasis-free survival without metastasis to the brain, bone or lung.
In some embodiments said sample cancer cells or tissue from the subject with breast cancer

### Brief Description of the Drawings

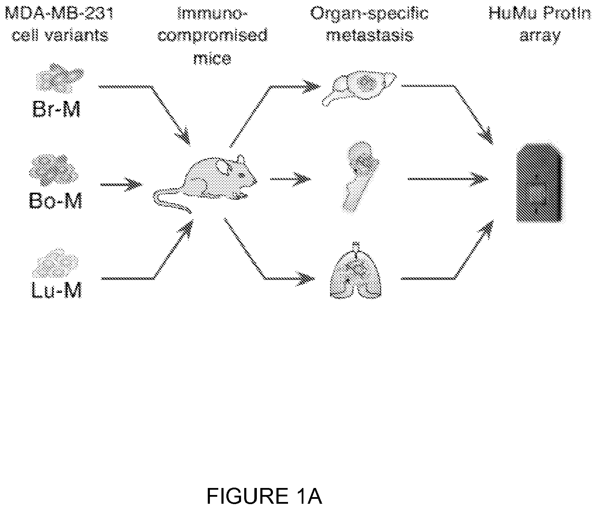

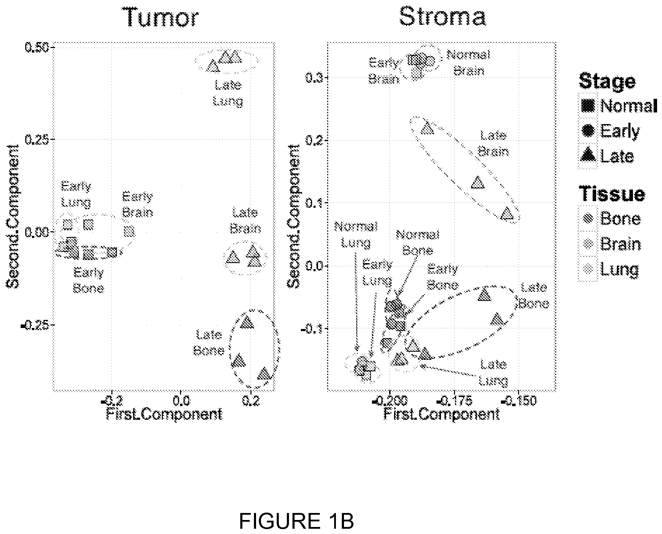

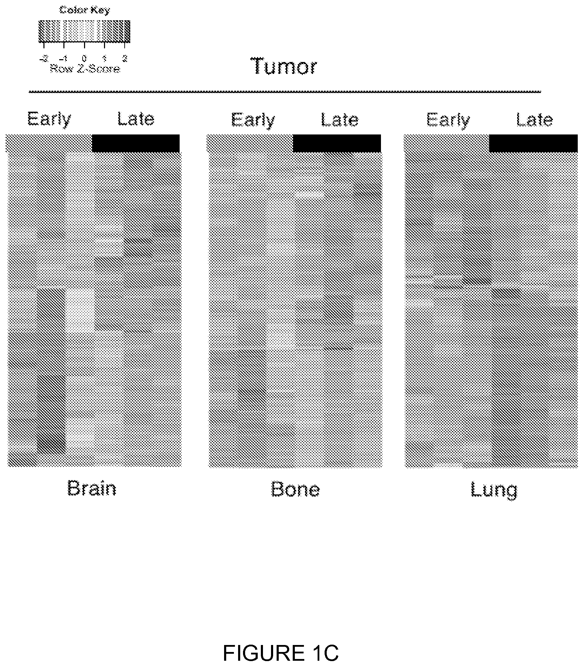

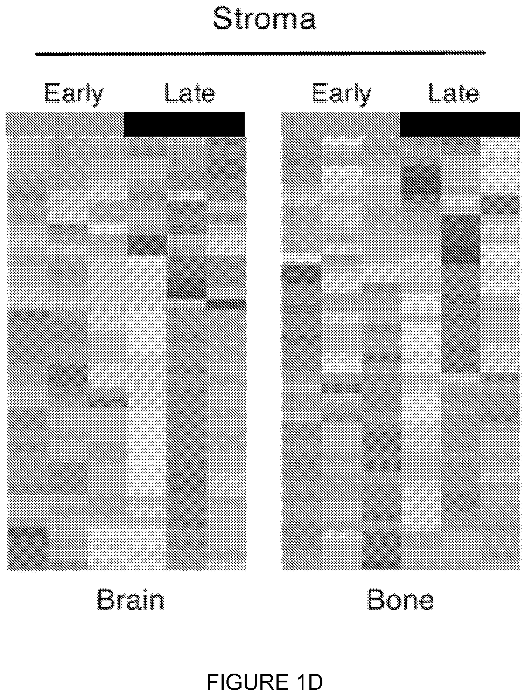

**Figure 1** shows that HuMuProtln array enables simultaneous acquisition of gene expression changes in tumor and stromal cells. **(A)** is a schematic of the experimental design employed to analyze tumor stroma interactions in different metastatic microenvironments. Br-M = brain metastatic, Bo-M = bone metastatic, and Lu-M = lung metastatic variants of the human breast cancer line MDA-MB-231 cell line were injected intracardially or intravenously into immunocompromised mice. RNA was isolated from whole brain-, bone-, and lung metastases that contain human tumor cells and mouse stromal cells. Gene expression changes in xenografted animals were analyzed with the dual-species specific array HuMu Protln (Human/ Murine Proteases and Inhibitors). **(B)** Principal component analysis of the HuMu array data: the 1 st and 2nd components are plotted on the x and y axes respectively. These components together represent the largest sources of variation in the dataset. The first and second components represent 89.98% and 8.44% respectively of the variance in the tumor gene space, and 90.83% and 4.06% in the stromal gene space. This analysis revealed variation in tumor gene expression that was associated with differences between early- and late-stage metastasis. Meanwhile, variation in the stroma was associated with both stage and tissue. Dotted ellipses were drawn manually to indicate related data points within stage or organ. **(C, D)** Heatmaps of **(C)** tumor- and **(D)** stroma-derived genes that were differentially expressed between early and late metastases across different organ sites. The lung stroma did not show extensive differences between early and late stages (Table 1g).

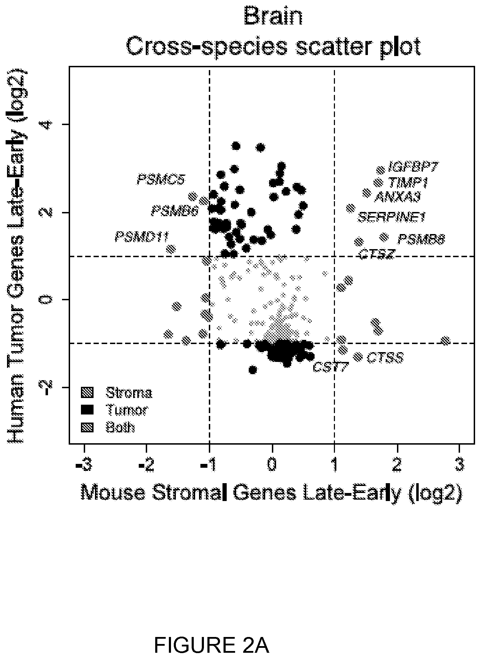

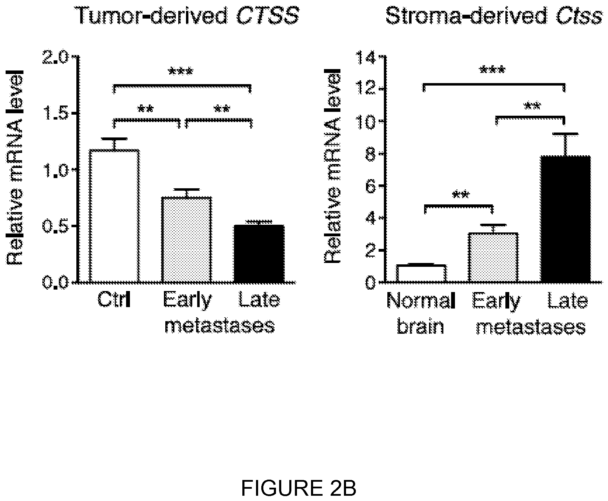

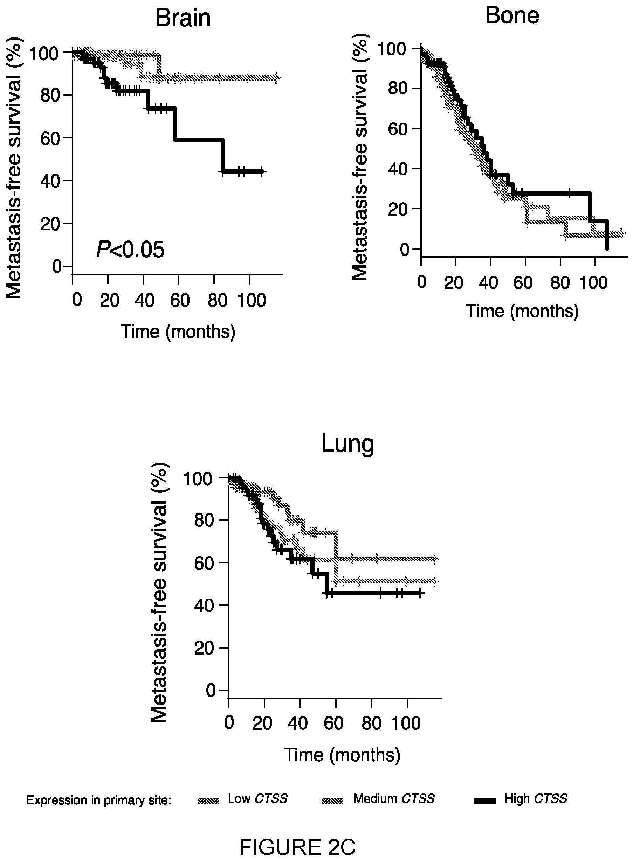

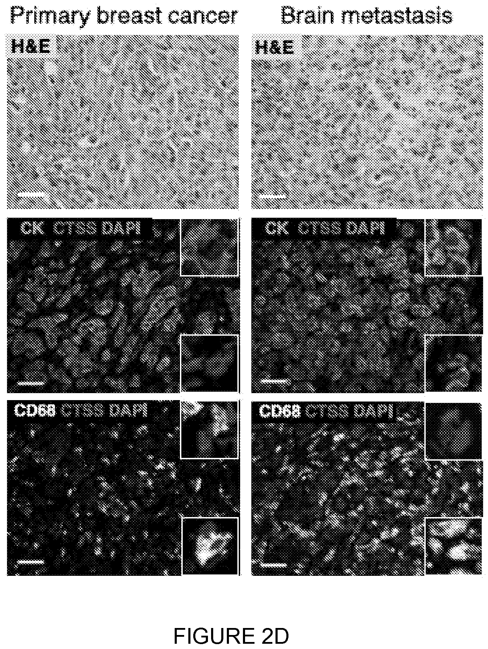

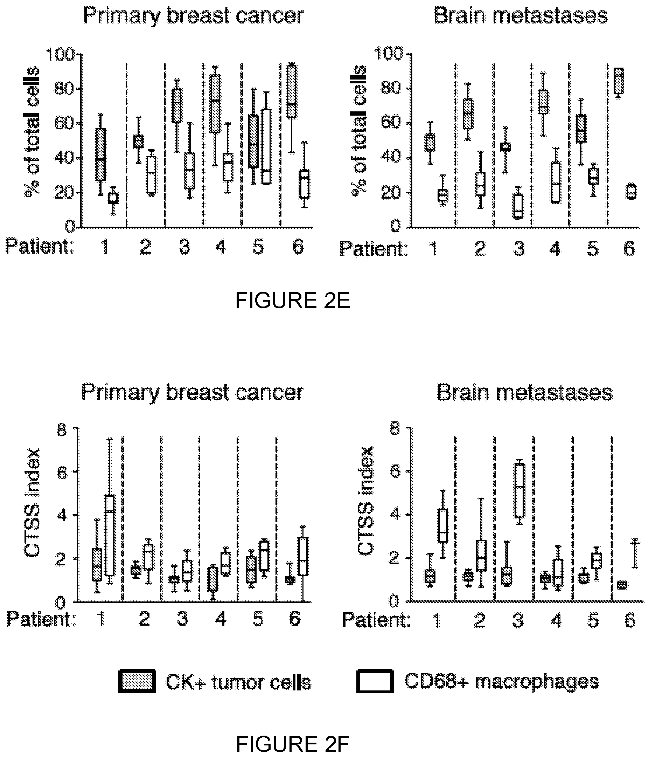

**Figure 2** shows that cathepsin S shows highly regulated stage- and cell type-specific expression changes in experimental brain metastases, and cathepsin S expression in primary breast tumors is inversely correlated with brain metastasis-free survival in patients. **(a)** Cross-species scatter plot shows log-fold expression changes in the tumor and stromal gene space during the transition from early- to late-stage metastases in the brain. Genes that are differentially expressed only in the stroma (mouse) or in the tumor (human) gene space are shown in pink or black respectively. Genes that are differentially expressed in both the stromal and tumor gene space are shown in purple. Grey dots represent homologs with either insufficient fold change or P values. Horizontal and vertical lines denote fold change cut-off for significance. Genes in the lower right quadrant (*CTSS* and *CST7*) represent genes that are downregulated in tumor cells in late metastases, and concomitantly upregulated in stromal cells. **(b)** Expression of tumor-derived (human) and stromal-derived (mouse) cathepsin S (*CTSS* or *Ctss* respectively) in Br-M control (Ctrl; *n*=11) cell line, normal brain (*n*=*12*), and early-*l* late-stage brain metastases (classified by BLI intensity; n=16 for early-stage and n=17 for late-stage metastases) from Athy/nu mice. mRNA expression is depicted relative to the Br-M Ctrl cell line for human *CTSS* and relative to normal brain for mouse *Ctss.* All assays were performed in triplicate and gene expression was normalized to *Ubc* for all stromal genes (mouse origin), *B2M* for tumor cell-derived genes (human origin). **(c)** Metastasis-free survival (MFS) for breast cancer patients (GSE12276 data set) based on low, medium and high *CTSS* expression at the primary site. The Kaplan-Meier plot demonstrates that high *CTSS* expression is inversely correlated with brain MFS, while there is no such association for bone or lung. **(d)** Representative images of matched primary breast cancer and brain metastasis patient samples stained for CTSS (red) and the macrophage marker CD68 (white), or pan-cytokeratin (CK; green) to visualize tumor cells. DAPI staining was used to visualize cell nuclei (blue). Scale bar indicates 50 µm. **(e)** Quantification of proportions of tumor cells and macrophages, presented as the percentage of total DAPI⁺ cells, in matched primary breast cancer and brain metastases samples. **(f)** Quantification of the CTSS index as a measure of relative CTSS levels in tumor cells and macrophages. Data are presented as bars + s.e.m. or as box plots with whiskers to illustrate minimum and maximum values. The horizontal line depicts the mean. *P* values were obtained using two-tailed unpaired *t*-test for (b) and a log-rank test for (c). ***P*<0.01, and ****P*<0.001.

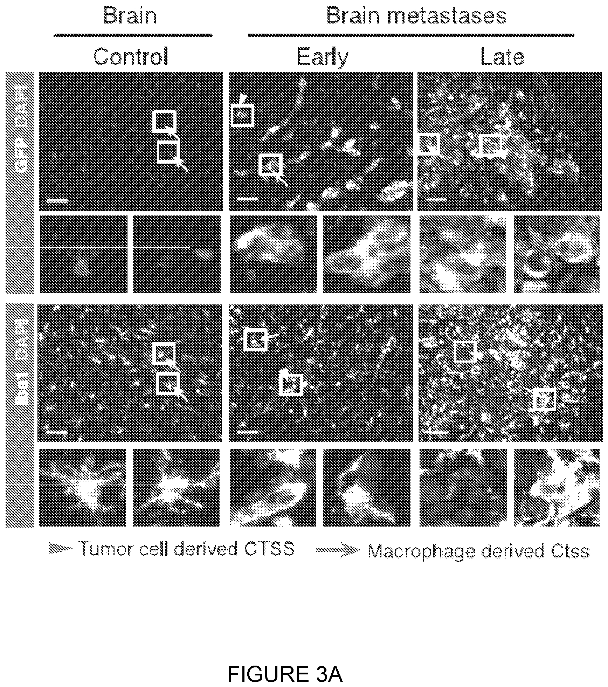

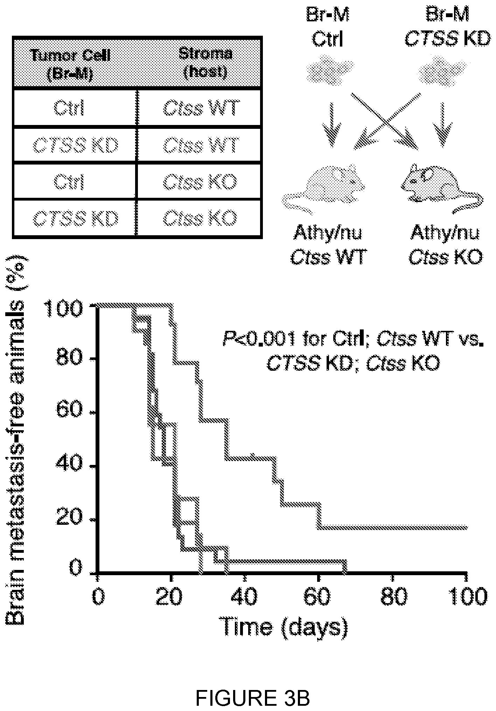

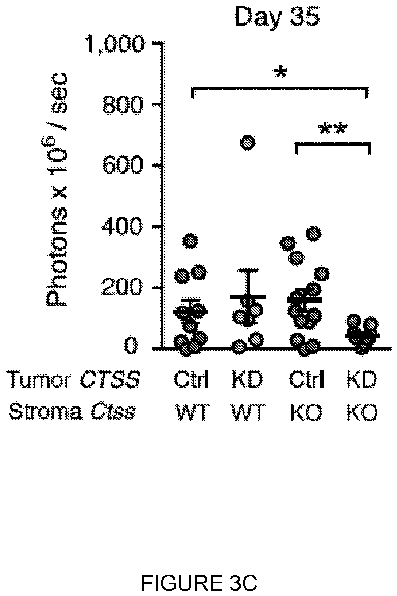

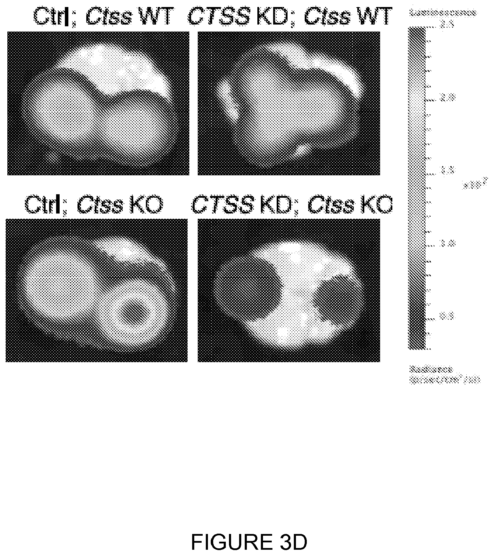

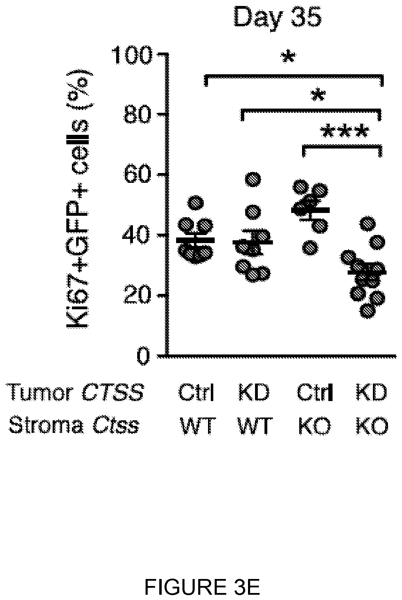

**Figure 3** illustrates that macrophages are the predominant source of stromal-derived cathepsin S and only combined depletion of tumor- and stromal-derived cathepsin S reduces experimental brain metastasis. **(a)** Representative images of normal brain, early- and late-stage brain metastasis (classified by BLI intensity) co-stained for Ctss/CTSS (red) and GFP (tumor cells; green) or Iba1 (macrophages/microglia; white). Tumor cell-derived CTSS is indicated by the arrowhead and macrophage-derived Ctss is indicated by the arrow. **(b)** Kaplan-Meier curve shows the percentage of brain metastasis-free animals in the 4 experimental groups indicated in the table. Ctrl; *Ctss* WT (*n*=21 mice), *CTSS* KD; *Ctss* WT (*n*=16), Ctrl; *Ctss* KO (*n*=*22*), and *CTSS* KD; *Ctss* KO (*n*=12). **(c)** Quantification of the *ex vivo* BLI intensity on day 35 after Br-M tumor cell inoculation. Ctrl; *Ctss* WT (*n*=10), *CTSS* KD; *Ctss* WT (*n*=7), Ctrl; *Ctss* KO (*n*=13), and *CTSS* KD; *Ctss* KO (*n*=11). **(d)** Representative *ex vivo* BLI images of the 4 experimental groups as shown in (c). **(e)** Quantification of tumor cell proliferation (percentage of Ki67⁺GFP⁺ cells) on day 35 after tumor cell inoculation. Ctrl; *Ctss* WT (*n*=8), *CTSS* KD; *Ctss* WT (*n*=8), Ctrl; *Ctss* KO (*n*=6), and *CTSS* KD; *Ctss* KO (*n*=10). Scale bar indicates 50 □m. Circles represent individual mice and horizontal lines represent the mean ± s.e.m. *P* values were obtained with Mantel-Cox log-rank test for MFS and with two-tailed unpaired *t*-test for numerical data. **P*<0.05, ***P*<0.01 and ****P*<0.001.

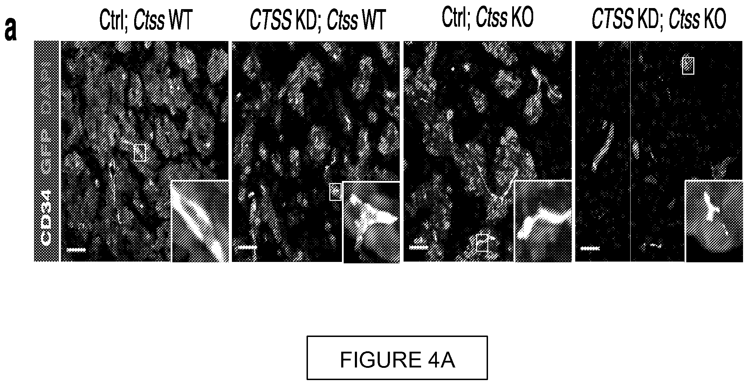

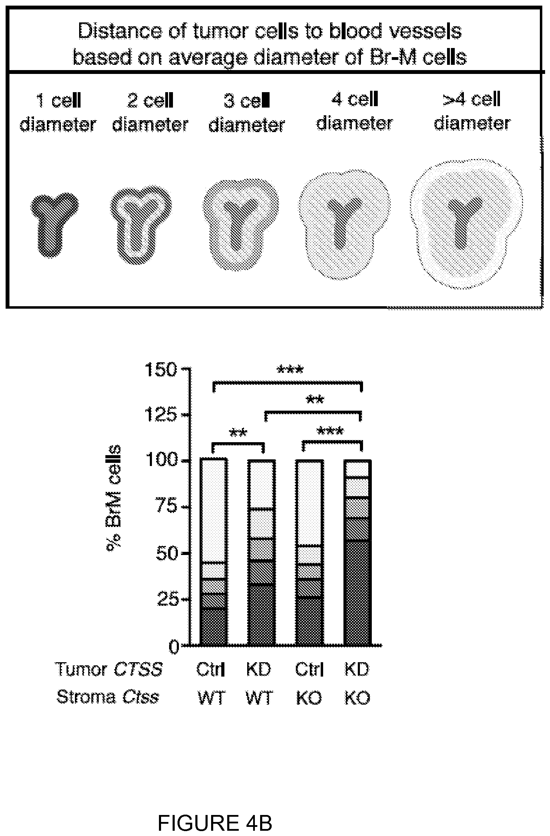

**Figure 4** shows that cathepsin S deficiency in tumor cells and macrophages impairs metastatic seeding and outgrowth. **(a)** Representative images of brain metastases (day 35) stained for GFP (tumor cells; green), the endothelial cell marker CD34 (white), and DAPI to visualize nuclei (blue). Scale bar indicates 50 µm. **(b)** GFP⁺ tumor cells were categorized based on their localization relative to blood vessels, defined as the distance of tumor cells from blood vessels (1 to >4 average tumor cell diameter), and the percentage of tumor cells in each defined area was quantified using Metamorph image analysis software. Ctrl; *Ctss* WT (*n*=*4*), *CTSS* KD; *Ctss* WT (*n*=6), Ctrl; *Ctss* KO (*n*=6), and *CTSS* KD; *Ctss* KO (*n*=6). Categorical data are plotted as stacked bars. P values were obtained with an ordinal Chi-square test for categorical data. ***P*<0.01 and ****P*<0.001.

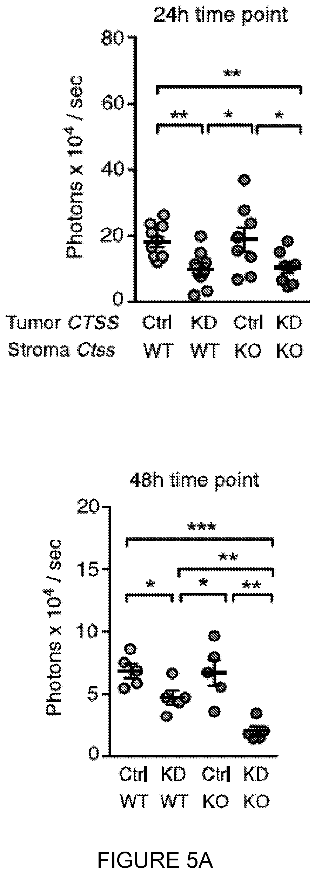

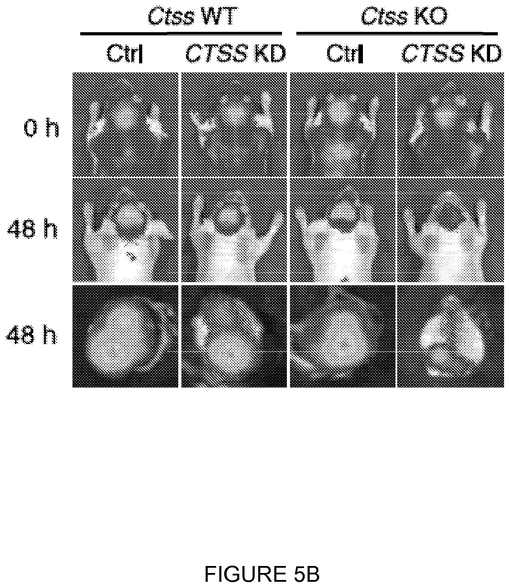

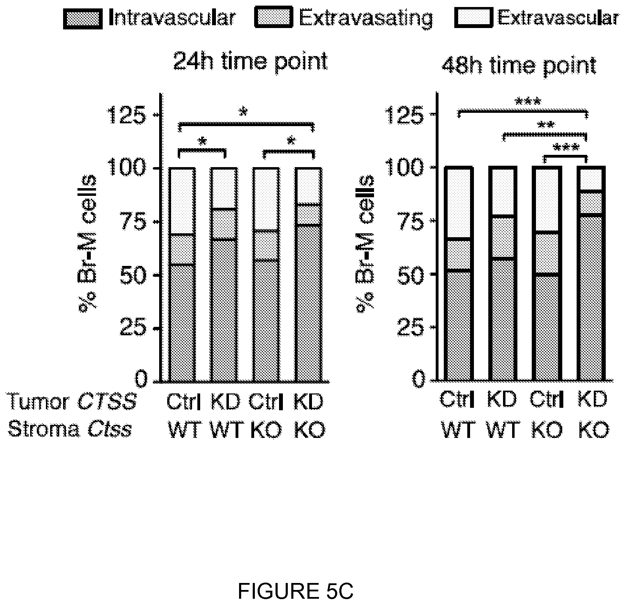

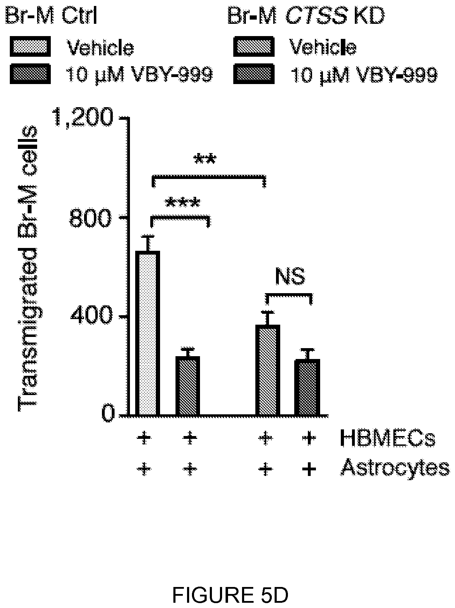

**Figure 5** Cathepsin S mediates blood-brain barrier transmigration of brain metastatic cells. (a) Quantification of BLI intensity at the indicated time points relative to BLI signal immediately after tumor cell inoculation. Ctrl; Ctss WT (n=10), CTSS KD; Ctss WT (n=9), Ctrl; Ctss KO (n=8), and CTSS KD; Ctss KO (n=8) for the 24h time point, and n=5 for each group for the 48h time point. (b) Representative BLI images in the 4 experimental groups immediately (0h) and 48h after tumor cell injection in vivo (top panels) and ex vivo (lower panel). (c) Tumor cells were categorized based on their localization relative to the vasculature defined as intravascular, extravasating and extravascular, and the percentage of viable tumor cells in each category was quantified at the indicated time points. Ctrl; Ctss WT (n=4), CTSS KD; Ctss WT (n=3), Ctrl; Ctss KO (n=3), and CTSS KD; Ctss KO (n=4) for the 24h time point, and n=4 for each group for the 48h time point. (d) Quantification of the number of transmigrated Br-M Ctrl and CTSS KD cells in the presence or absence of the cathepsin S-specific inhibitor VBY-999 through an in vitro BBB formed with human brain microvascular endothelial cells (HBMEC) in co-culture with astrocytes. Circles represent individual mice and horizontal lines represent the mean ± s.e.m. for numerical data shown in (a). Graphs represent mean + s.e.m in (d). Categorical data are plotted as stacked bars. P values were obtained with two-tailed unpaired t-test for numerical data and with an ordinal Chi-square test for categorical data. NS = not significant, *P<0.05, **P<0.01, and ***P<0.001.

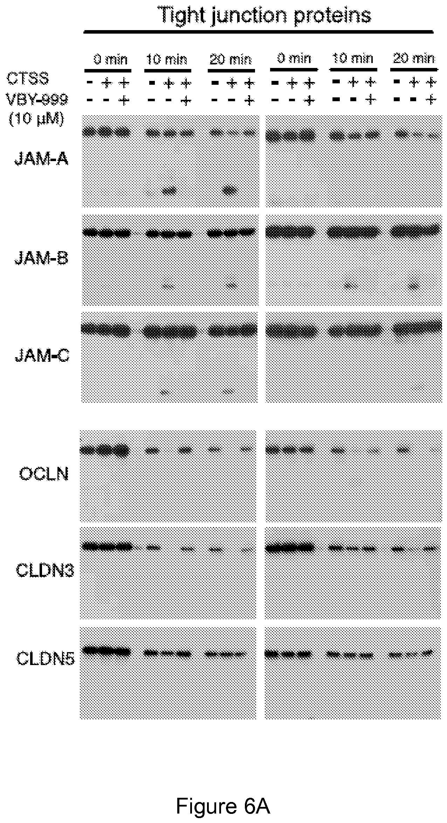

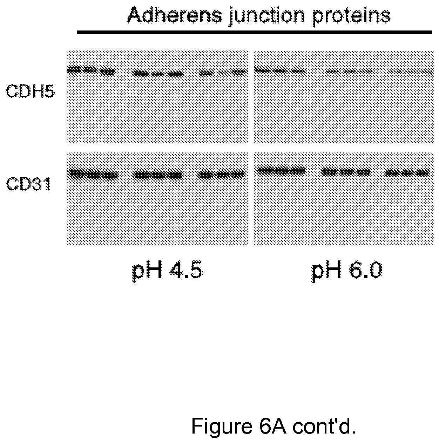

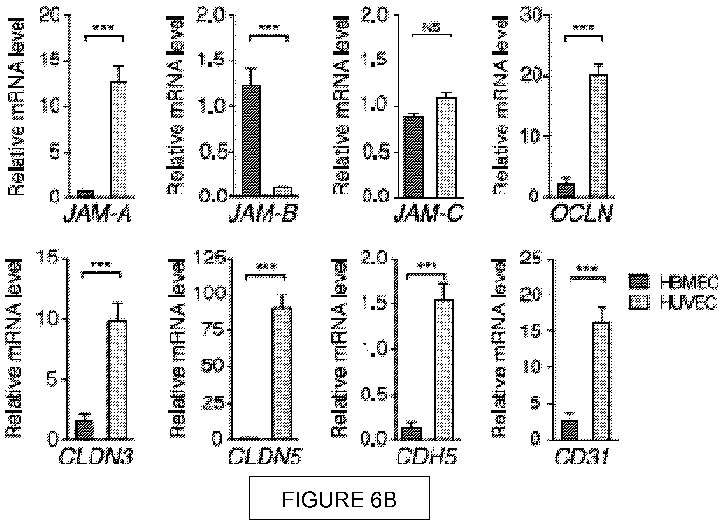

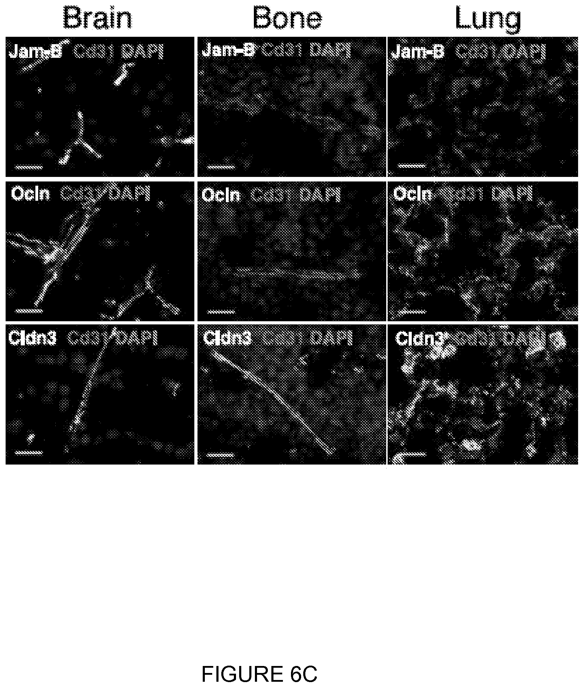

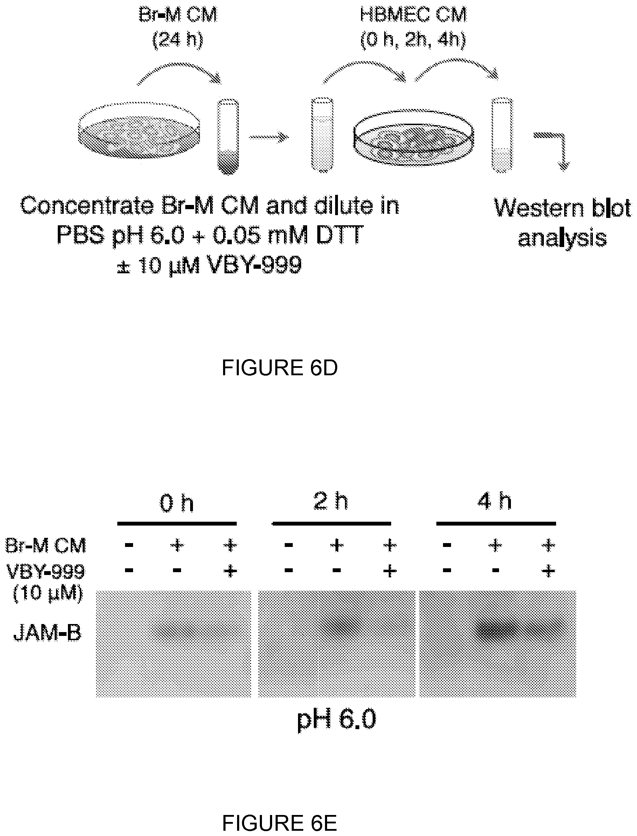

**Figure 6** shows that cathepsin S cleaves tight junction proteins that regulate blood-brain barrier integrity. (a) Western blot analysis of CTSS-mediated cleavage of recombinant tight junction proteins (junctional adhesion molecules (JAM)-A, -B and -C, occludin (OCLN), claudins (CLDN)-3 and-5), and adherens junction proteins cadherin 5 (CDH5) and CD31 for the indicated time points at pH 4.5 and pH 6.0 in the presence or absence of the cathepsin S-specific inhibitor VBY-999. VBY-999 was used at 10 □M, a concentration that efficiently inhibits cathepsin S. (b) mRNA expression of the tight junction and adherens junction molecules in HUVECs and HBMECs (n=9 for each cell line). All assays were run in triplicate and gene expression was normalized to B2M. Expression is depicted relative to expression in HBMECs. (c) Representative images of control brain, bone, and lung sections stained for the tight junction proteins Jam-B, Ocln or Cldn 3 (white), with CD31 (red) to visualize blood vessels. DAPI was used as a nuclear counterstain. (d) Schematic of the cell-based cleavage assay. (e) Western blot analysis showing increased JAM-B in HBMEC-conditioned media (CM) after incubation with Br-M cell CM for the indicated time points. Addition of the cathepsin S specific inhibitor VBY-999 (10 µM) resulted in reduced accumulation of JAM-B in HBMEC CM at the indicated time points. Incubation with PBS pH 6.0, 0.05 mM DTT served as a control for baseline JAM-B shedding of HBMEC. Each western blot shows the representative result of three independent experiments. Scale bar indicates 20 µm. Graphs represent mean + s.e.m. P values were obtained using two-tailed unpaired t-test. NS = not significant, ****P*<0.001.

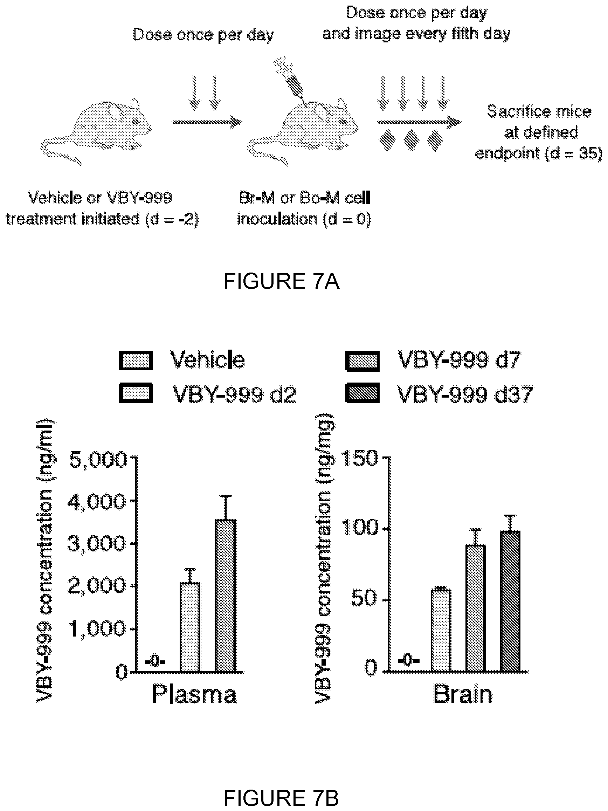

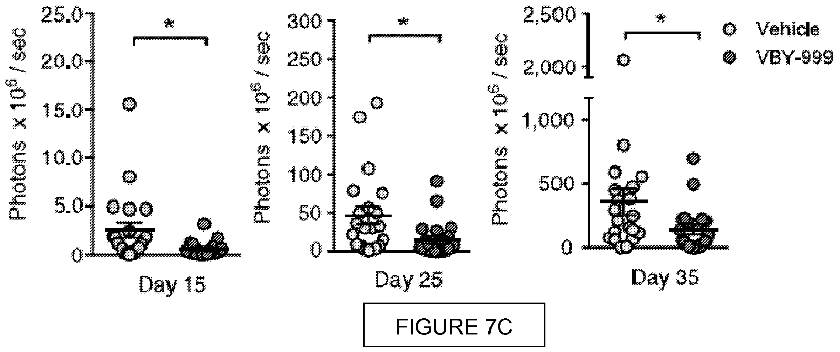

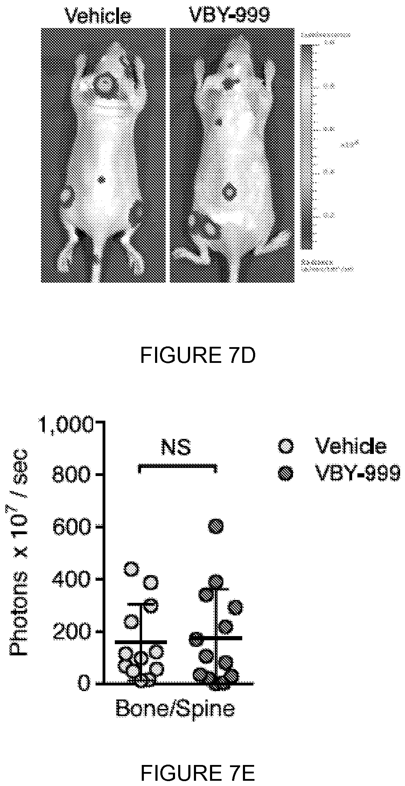

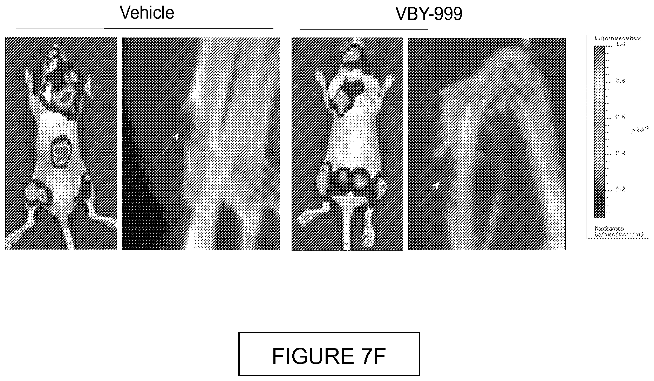

**Figure 7** shows that pharmacological inhibition of cathepsin S reduces brain metastasis formation in a preclinical trial. **(A)** Schematic of the prevention trial experimental design. **(B)** Quantification of VBY-999 concentrations in plasma and brain tissue at the indicated time points after treatment started (n=3 for each group). **(C)** Quantification of BLI intensity in the head region at the indicated time points after Br-M cell inoculation. n=20 for vehicle group (5% dextrose in water (D5W)) and n=21 for VBY-999 treatment group (100 mg/kg/day). The BLI signal in the VBY-999 versus control group is 77, 70 and 65% reduction at each of the three successive time points indicated. **(D)** Representative BLI images at the trial endpoint, d35 after Br-M cell inoculation. **(E)** Quantification of BLI intensity at d35 after Bo-M tumor cell inoculation in the bone and spine region. Vehicle (n=12 mice) and VBY-999 (n=13). **(F)** Representative BLI and X-ray images at day 35 after Bo-M cell inoculation. Arrows indicate osteolytic lesions. Bars represent mean + s.e.m. for (b), circles represent individual mice and horizontal lines represent the mean ± s.e.m for **(C, E).** P values were obtained using two-tailed unpaired t-test. NS = not significant, *P<0.05.

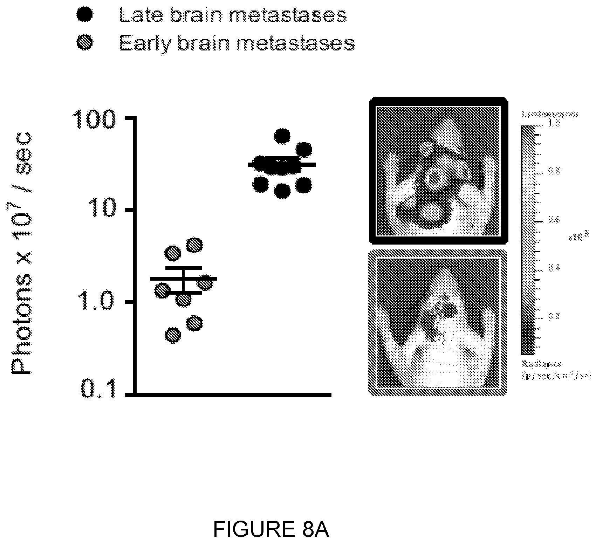

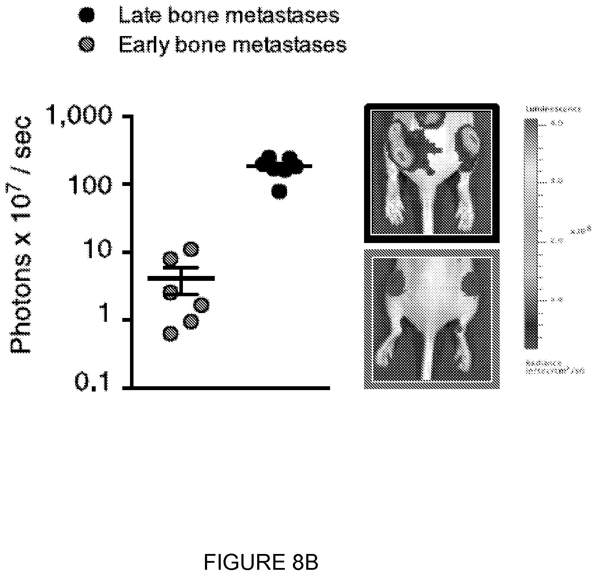

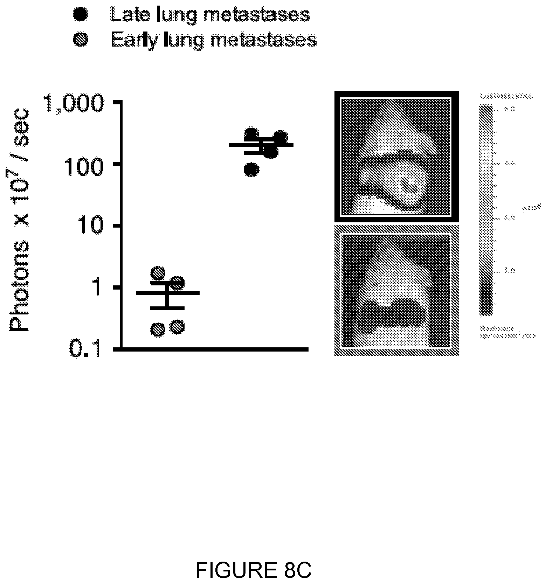

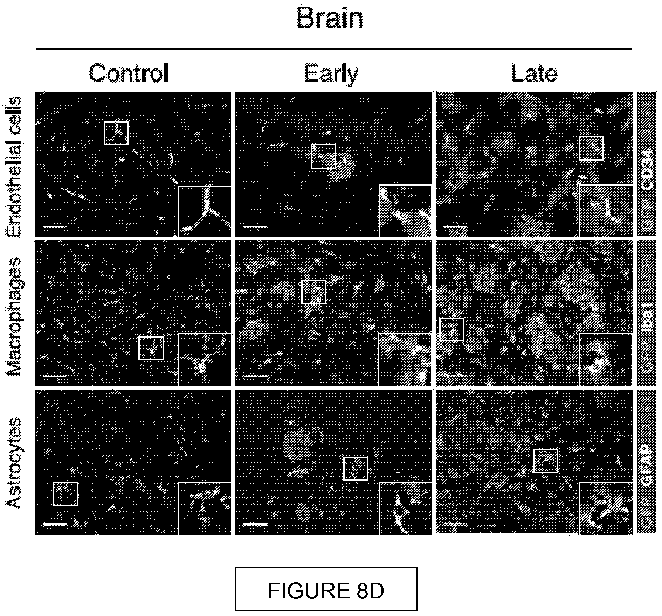

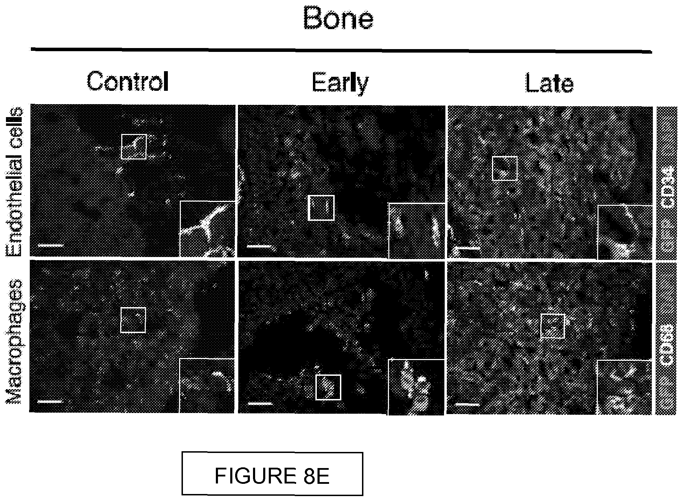

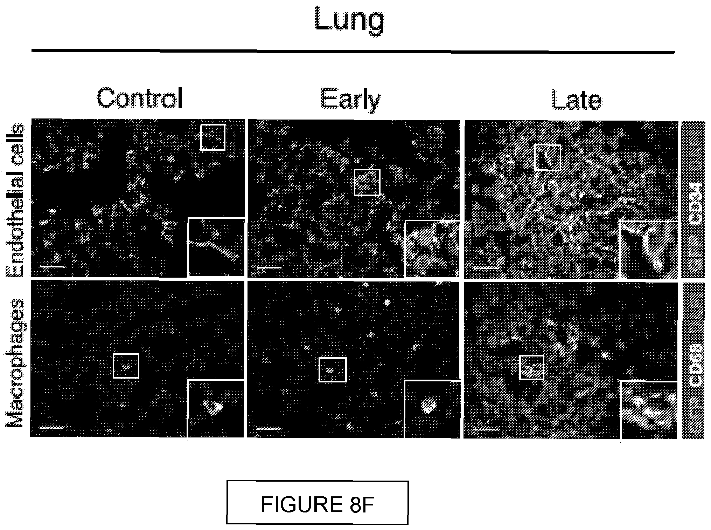

**Figure 8** illustrates characterization of the stromal cell types in early- and late-stage brain, bone and lung metastasis. **(A-C)** Quantification and representative images of the in vivo BLI intensity are shown for (a) brain metastases, **(B)** bone metastases, and **(C)** lung metastases for early and late stages. Circles represent individual mice and horizontal lines represent the mean ± s.e.m. **(D-F)** Representative images of control tissue and early- and late-stage brain, bone, and lung metastases stained for GFP (tumor cells) and the endothelial marker CD34 or the macrophage markers CD68 (lung and bone) or Iba1 (brain). DAPI staining was used to visualize cell nuclei. Brain metastasis sections were also stained with the astrocyte-specific marker GFAP. Scale bar indicates 50 µm.

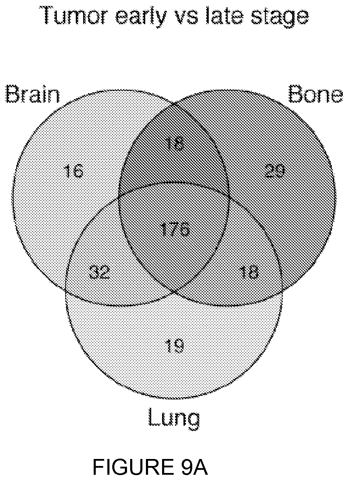

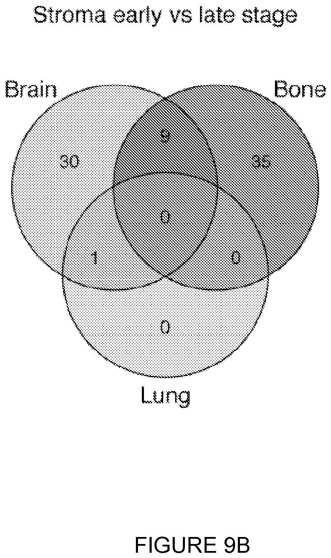

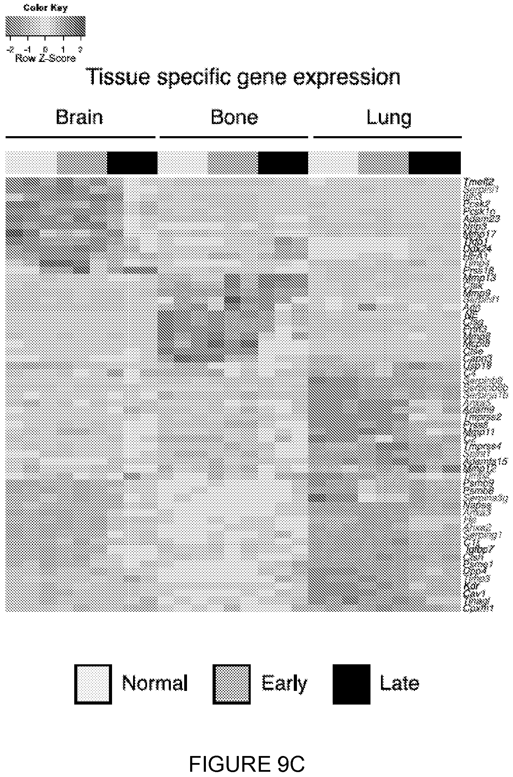

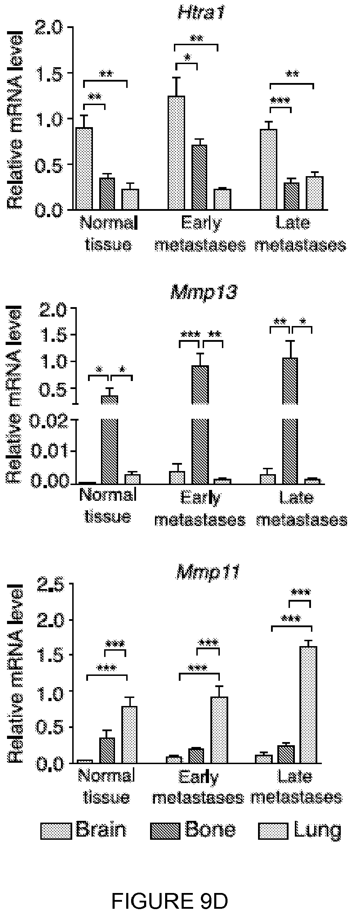

**Figure 9** demonstrates tissue- and stage-specific gene expression changes in tumor and stroma. **(A)** Venn diagram of the tumor-derived genes that are significantly different between early and late metastases across different metastatic sites (Fig. 1c). Of the 308 genes significantly different between early and late stage in either brain, bone or lung metastases, 176 genes change by stage in all three sites. **(B)** Venn diagram depicting the overlap of the 75 stroma-derived genes that are significantly different between early- and late-stage metastases across brain, bone and lung (Fig. 1**D**). Unlike the tumor-derived genes depicted in **(A),** there were no stromal-derived genes that were significantly different between early- and late-stage metastases in all three tissues investigated. **(C)** Heatmap depicting tissue-specific gene expression in the brain, bone and lung stroma. No tissue-specific genes were found for tumor-derived genes. Proteases are denoted in purple, endogenous inhibitors in red, and their interacting partners in black. **(D)** qPCR confirmed the tissue-enriched expression pattern of Htra1 for brain, Mmp13 for bone, and Mmp11 for lung in control tissue, early- and late-stage metastases. Graphs represent mean + s.e.m. P values were obtained using two-tailed unpaired t-test. **P<0.05, **P<0.01, and ***P<0.001.

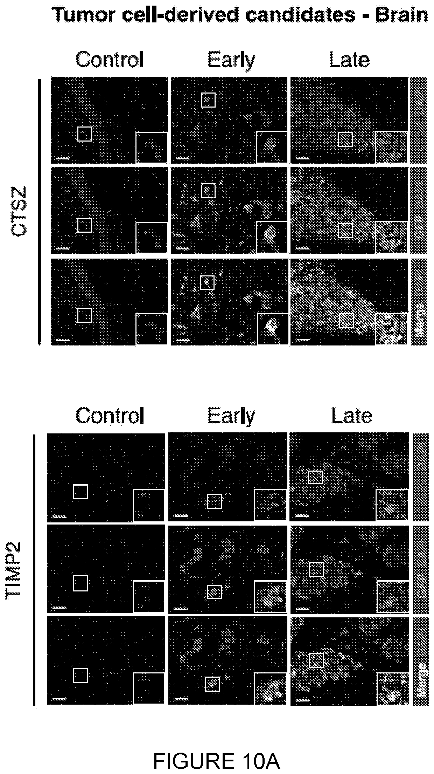

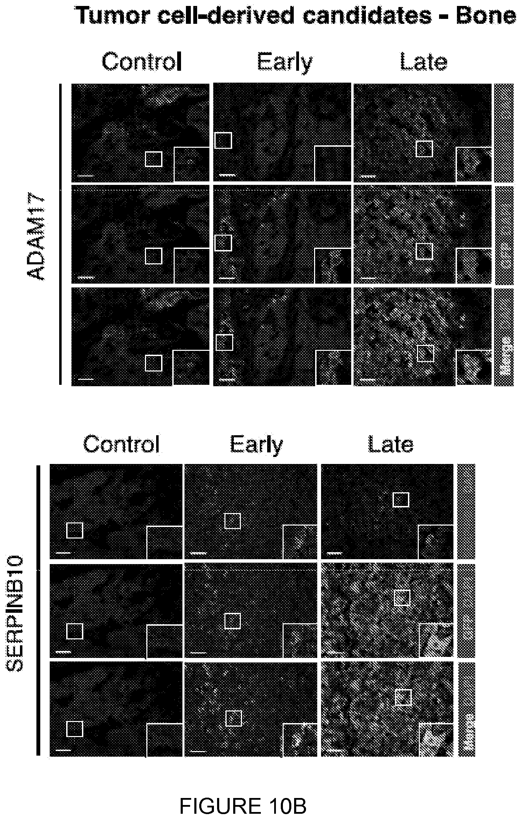

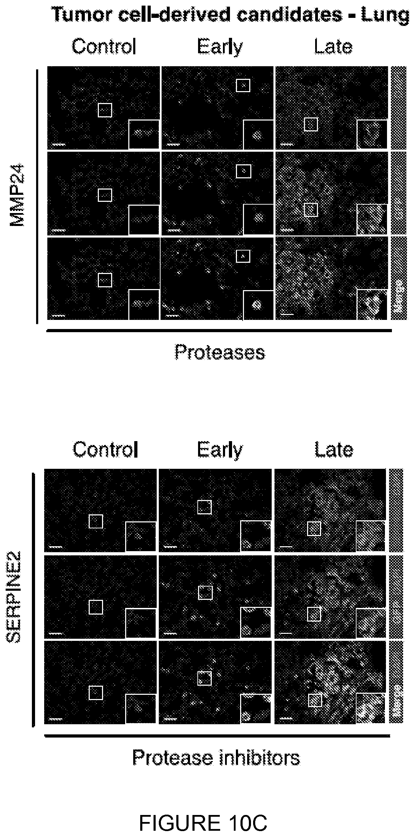

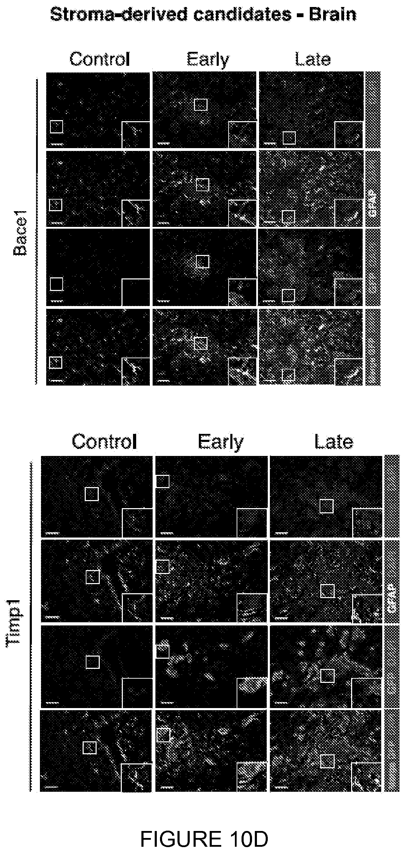

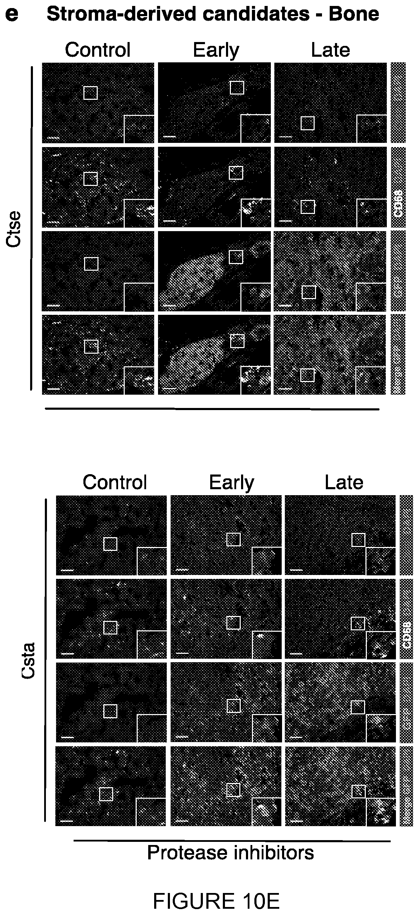

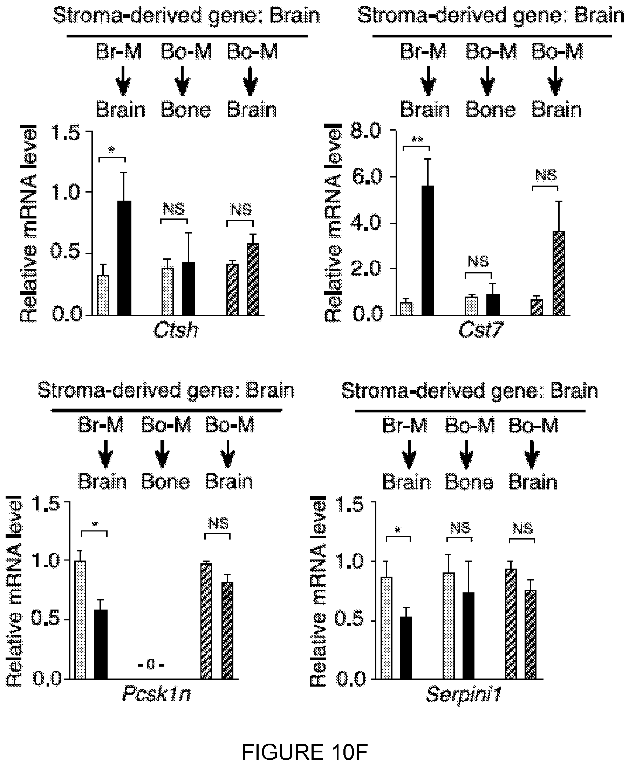

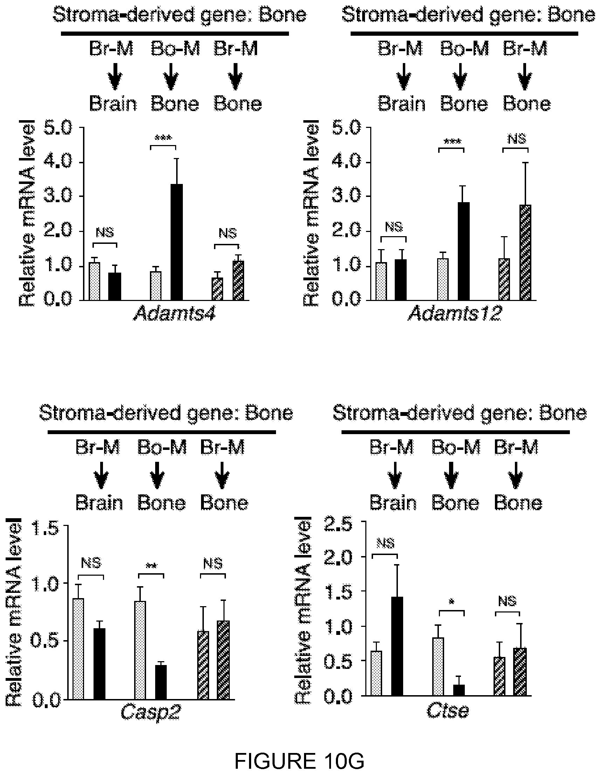

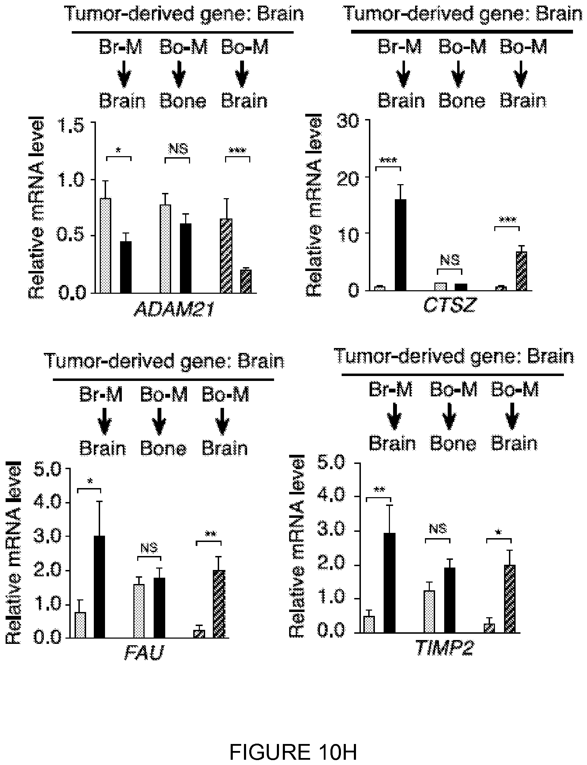

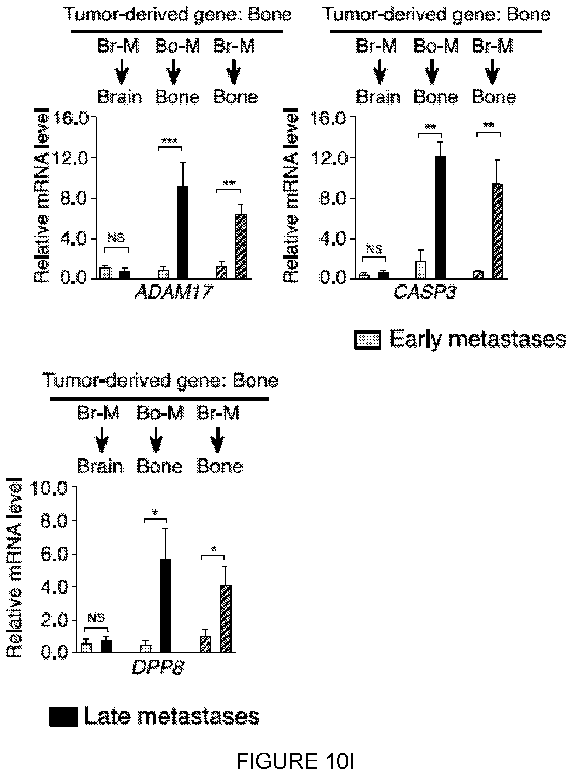

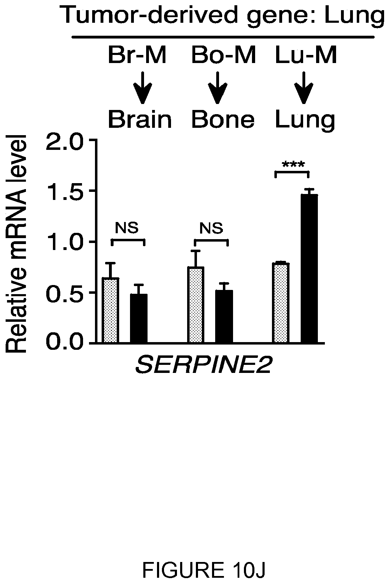

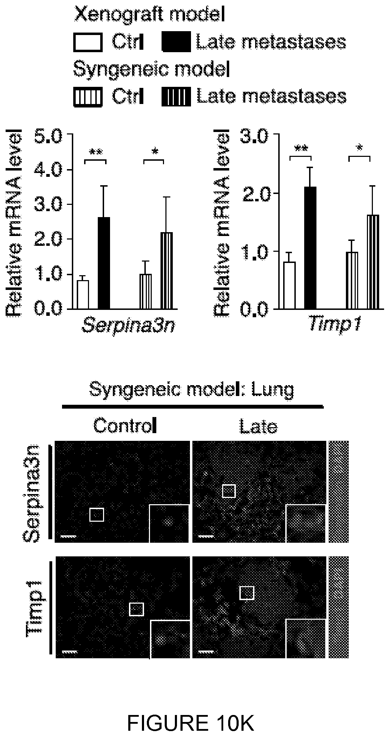

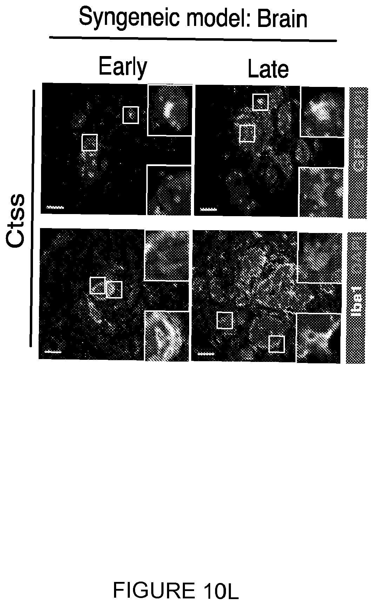

**Figure 10** shows the independent validation of differentially expressed genes in experimental brain, bone and lung metastases. (a-e) Representative images of control (non tumor-burdened) tissue, early- and late-stage site-specific metastases (classified by BLI intensity as in Fig. 8) showing immunofluorescence staining of tumor- and stromal-derived proteases and protease inhibitors exhibiting stage-dependent expression changes in the HuMu Protln array. (a) Brain sections were stained with antibodies against the protease CTSZ and the protease inhibitor TIMP2 as representative candidates that were differentially expressed in tumor cells. (b) Bone sections were stained with antibodies against the protease ADAM17 and the protease inhibitor SERPINB10 to represent differentially expressed candidates in tumor cells in bone metastases. (c) Lung sections were stained with antibodies against the protease MMP24 and the protease inhibitor SERPINE2 to confirm stage-differential expression in tumor cells in lung metastases. (d) Staining for the stromal-derived protease Bace1 and the protease inhibitor Timp1 confirmed stage-specific expression changes in GFAP+ astrocytes. (e) Staining for the protease Ctse and the protease inhibitor Csta confirmed stage-specific stromal changes in bone metastasis. CD68+ macrophages were identified as the predominant source for Csta in bone metastases. qPCR was used to confirm stage-differential expression changes, and to determine if the expression changes in stromal cells in brain or bone metastasis are a general response to tumor cell colonization of the respective organ, or if the expression changes depend on the tumor cell variant. Hatched bars represent 'mismatched' samples (BrM in bone and Bo-M in brain). Filled bars indicate the 'matched' samples. (f-g) qPCR for Ctsh, Cst7, Pcsk1n, Serpini1 (brain) in (f) and Adamts4, Adamts12, Casp2, Ctse (bone) in (g) confirmed stage-differential expression changes in a tissue-dependent manner. 'Mismatched' samples did not show significant changes. (h,i) qPCR for ADAM21, CTSZ, FAU, TIMP2 (brain) in (h) and ADAM17, CASP3, DPP8 (bone) in (i) confirmed stage-differential expression changes in a tissue-dependent manner. 'Mismatched' samples (Br-M to bone and Bo-M to brain) revealed that tumor cells underwent the same significant expression changes between early and late stages as identified for matched samples in the respective tissue. (j) qPCR for SERPINE2 confirmed stage-differential expression changes in Lu-M tumor cells in lung metastasis, while those changes were not present in Br-M and Bo-M cells in brain or bone metastasis, respectively. (k) qPCR Serpina3n and Timp1 confirmed stage-differential expression changes (control vs. late) in the stroma of lungs from xenografted animals as well as lungs from the immunocompetent, syngeneic MMTV-PyMT breast cancer model. (I) Immunofluorescence staining for Serpina3n and Timp1 in lungs of MMTV-PyMT breast cancer model (upper panels) and Ctss (red) in co-staining with GFP (tumor cells; green) or Iba1 (macrophages; white) in the syngeneic PyMT-BrM model (lower panels). Images are representative of three independent samples per stage. Scale bar indicates 50 µm. For qPCR validation: n=11, 15, 15, 13, 7, 15 samples in (f), n=11, 15, 15, 13, 7, 5 samples in (b), n=8, 15, 7, 9, 5, 15 samples in (g), n=8, 15, 7, 9, 5, 5 samples in (h), n=8, 6, 15, 12, 5, 3 samples in (i). n=5 and 9 samples for control lung and late-stage metastases (xenograft model), and n=6 and 9 for control lung and late-stage metastases (syngeneic model). mRNA expression is depicted relative to early-stage metastases in (f-i) and relative to control tissue in (j). All assays were performed in triplicate and gene expression was normalized to Ubc for all stromal genes, B2M for tumor cell-derived genes.. P values were obtained using two-tailed unpaired t-test: NS = not significant, *P<0.05, **P<0.01, and ***P<0.001.

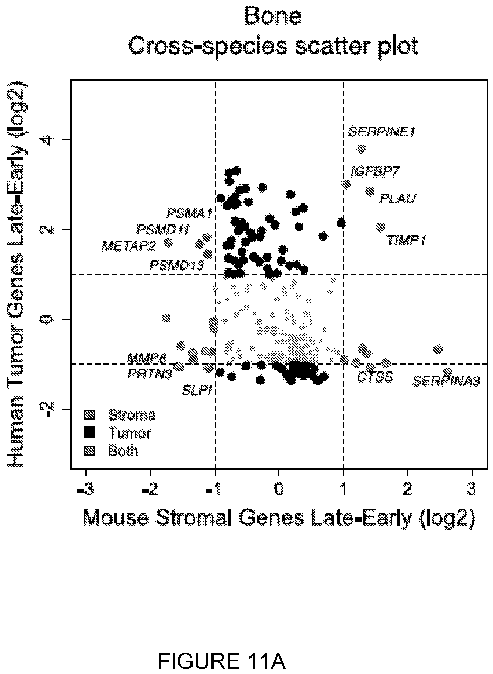

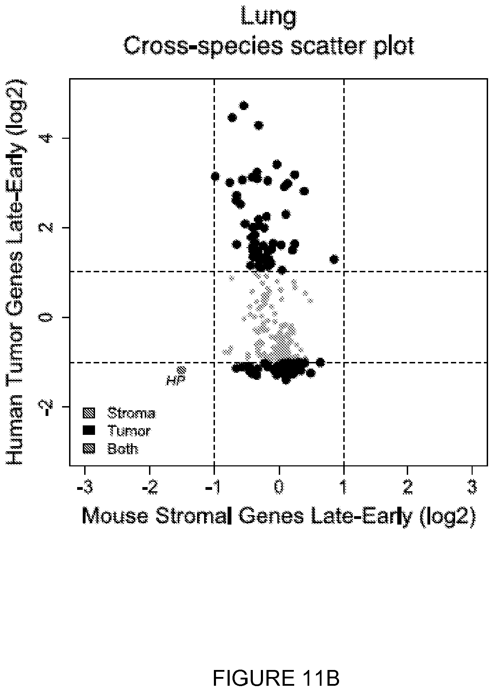

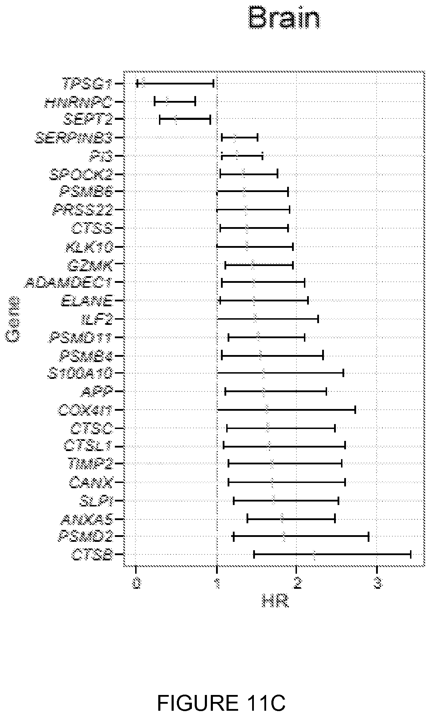

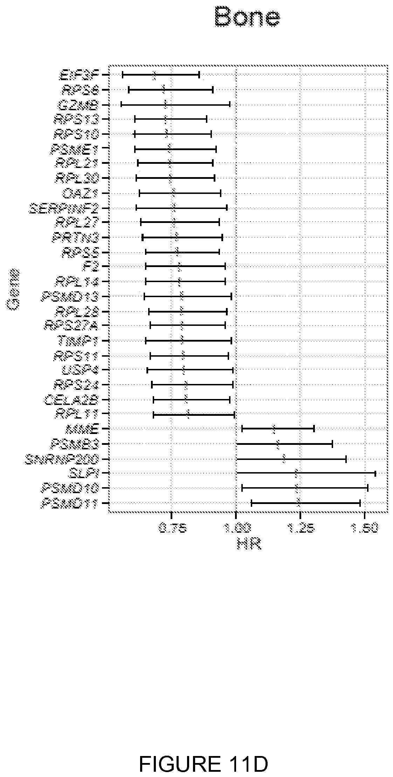

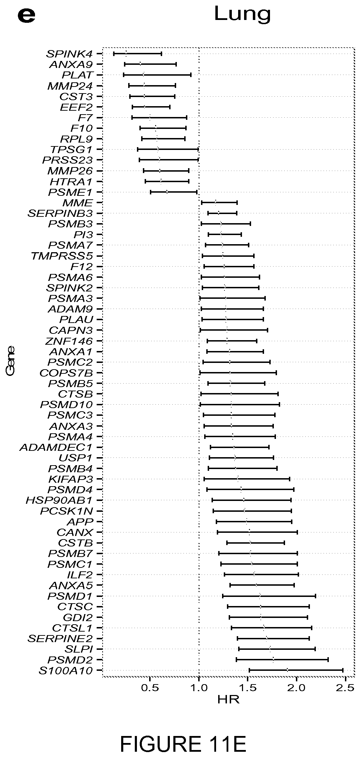

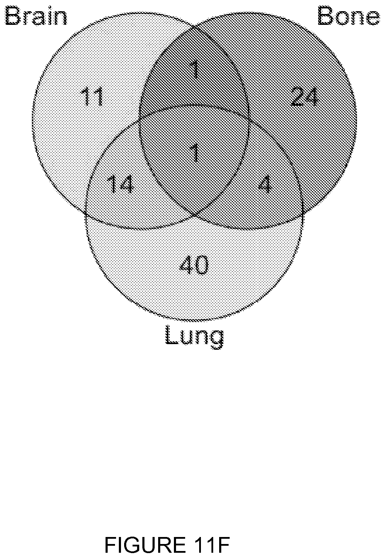

**Figure 11** shows the identification of genes associated with metastasis-free survival (MFS) and differentially expressed between the tumor and stroma. **(A-B)** As shown in Figure 2a for brain metastasis, cross-species scatter plots depict expression changes in the tumor and stromal gene space during the transition from early- to late-stage metastasis for (a) bone and (b) lung metastasis. Differentially expressed genes in the stroma (mouse) or in the tumor (human) gene space are shown in pink or black respectively. Genes that are differentially expressed in both the stroma and the tumor gene space are shown in purple. **(C-E)** Differentially expressed genes shown in Figure 1c were analyzed for association with MFS for either **(C)** brain, **(D)** bone, or **(E)** lung metastasis, depending on the tissue in which the gene was differentially expressed. Scaled gene expression values were used in a Cox proportional hazards model as described in the methods. Hazard ratios (HR) and 95% confidence intervals are shown for each organ site. HR <1.0 is associated with better patient prognosis, whereas HR >1.0 is associated with poor patient prognosis. **(F)** Genes depicted in **(C-E)** are shown in the Venn diagram, where few genes were found to be significantly associated with MFS in multiple tissues. A single gene, *SLPI,* was significantly associated with MFS in all three tissues: high expression of levels of *SLPI* correlated with poor patient prognosis. Hazard ratio significance was determined using Wald's test with a nominal P value cutoff of < 0.05.

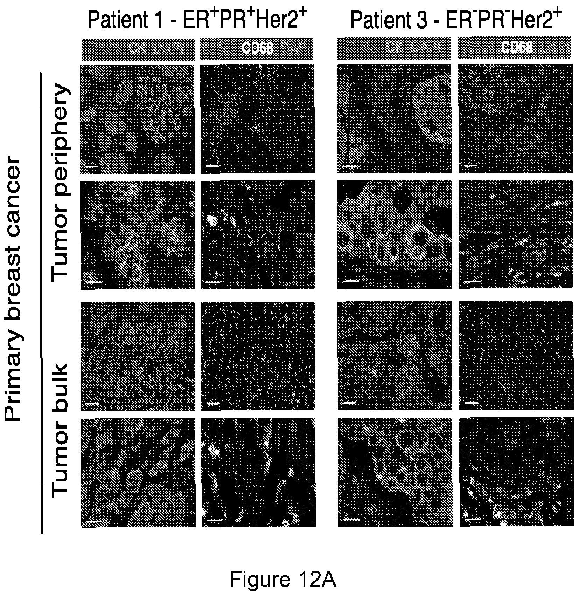

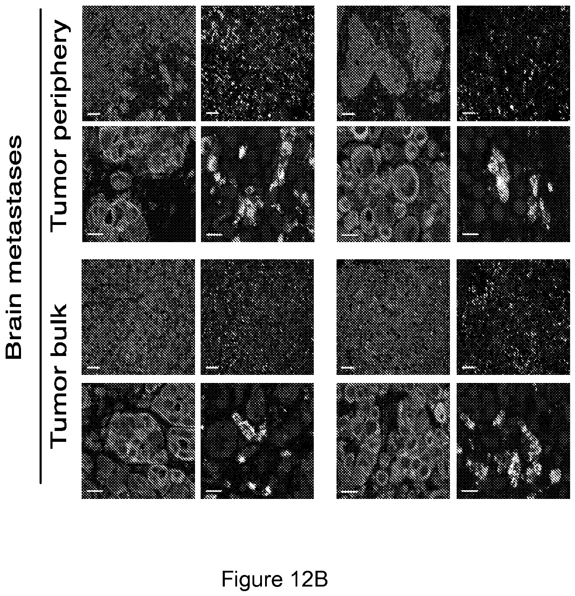

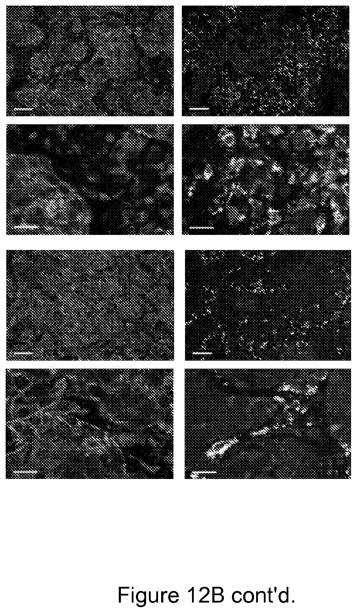

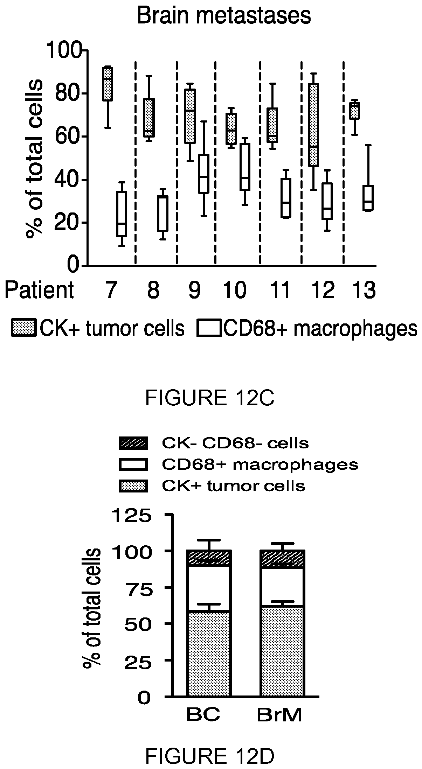

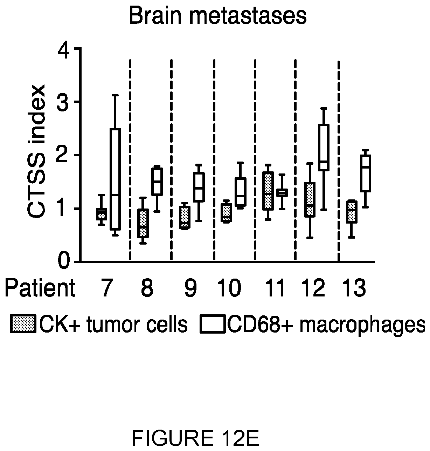

**Figure 12** Tumor cells and macrophages are the major constituent cell types of patient brain metastases and express high levels of CTSS. (a-b) Representative images of (a) primary breast cancer and (b) brain metastases patient samples stained for CTSS (red) in combination with the macrophage marker CD68 (white) or a pan-cytokeratin (CK) antibody to visualize tumor cells (green). DAPI staining was used to visualize cell nuclei (blue). The patient samples shown here represent different subtypes of breast cancer based on ER/PR/Her2 status. Scale bar indicates 100 µm in the upper panel rows and 20 µm in the two lower panel rows. (c) Quantification of proportions of CK+ tumor cells and CD68+ macrophages in brain metastases samples (these are from patients for which there was no matched primary breast tumor tissue available). (d) Combined quantification of proportions of CK+ tumor cells, CD68+ macrophages, and the remaining CK-CD68- cell population in primary breast cancer (n=6) and brain metastasis samples (n=13; either matched to the primary, or unmatched samples). (e) Quantification of the CTSS index as a measure of relative CTSS protein levels in tumor cells and macrophages. Data in (c) and (e) are presented as box plots with whiskers to illustrate minimum and maximum values. The horizontal line depicts the mean. Data in (d) are presented as stacked bars + s.e.m.

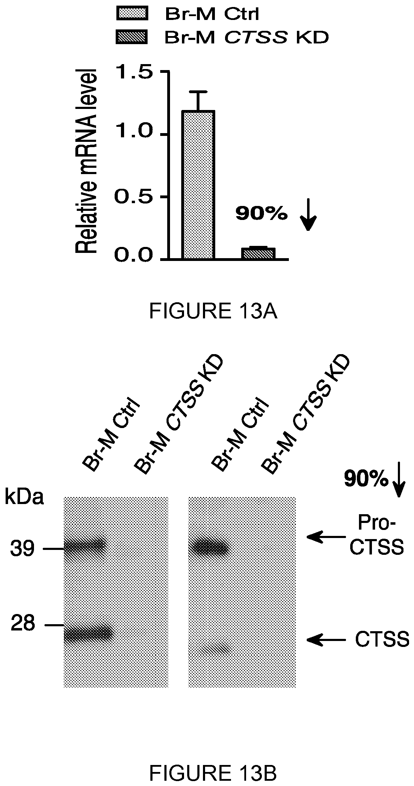

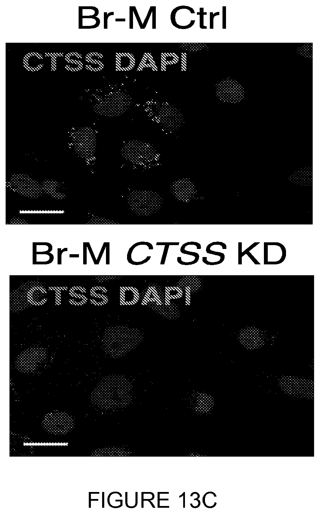

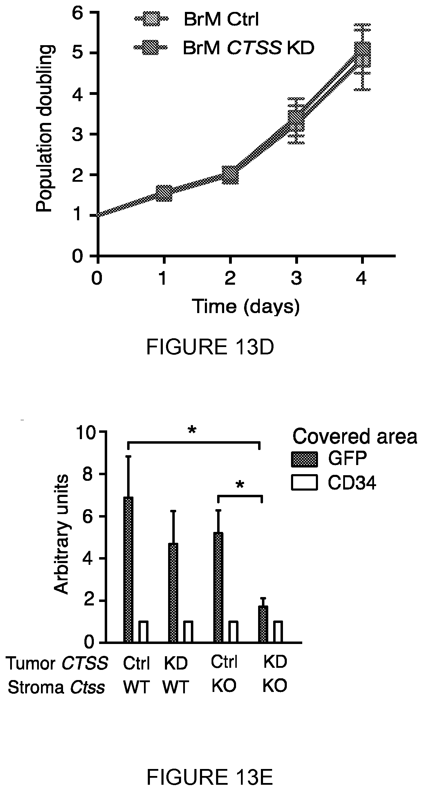

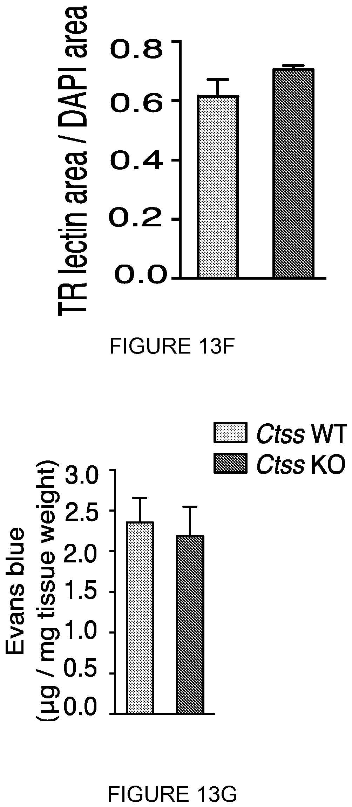

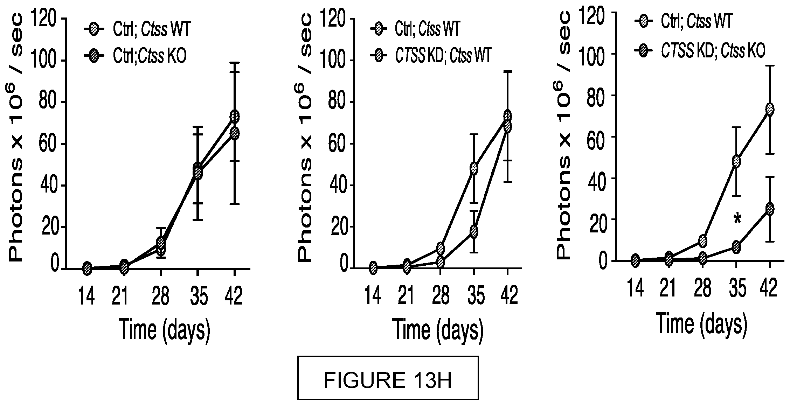

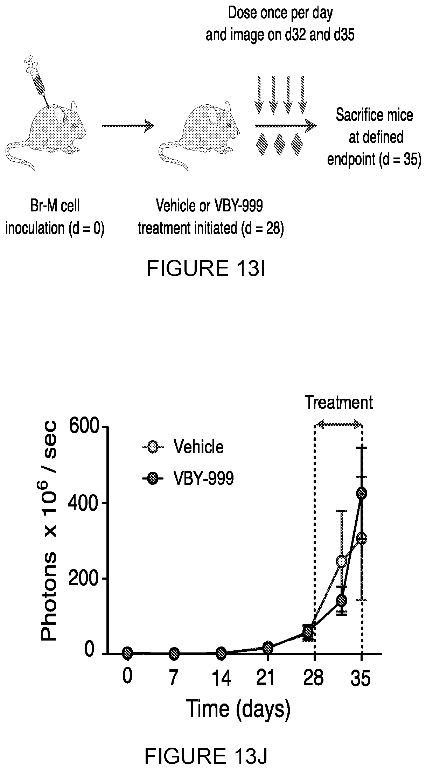

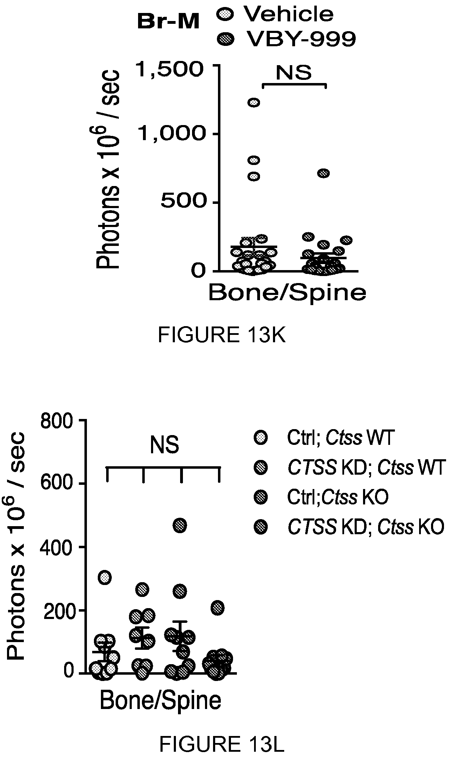

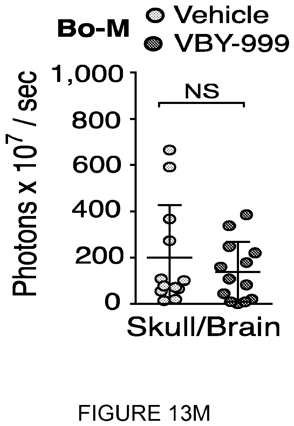

**Figure 13** shows that cathepsin S deficiency differentially alters brain metastasis growth kinetics, but does not affect viability of Br-M cells or vessel formation in mice. (a) Quantification of CTSS mRNA expression in Br-M Ctrl and Br-M CTSS KD tumor cell lines (n=7 replicates). Expression is depicted relative to Br-M Ctrl cells. All assays were run in triplicate and gene expression was normalized to B2M. (b) Western blot analysis of CTSS expression levels in cell lysates and conditioned media (CM) from Br-M Ctrl and Br-M CTSS KD cells. Western blot shows representative result from 3 independent experiments. qPCR and western blotting revealed a 90% knockdown efficiency for CTSS at both the mRNA and protein level. (c) Representative images of CTSS immunofluorescence staining of Br-M Ctrl and Br-M CTSS KD cell lines. DAPI staining was used to visualize cell nuclei (blue). Scale bar indicates 20 µm. (d) CTSS KD does not affect cell viability in culture as determined by MTT assays (n=4 replicates). (e) Brain metastases size and vessel density were defined as the area covered by GFP (tumor cells) or CD34 (endothelial cells) respectively, and the GFP-covered area was quantified relative to CD34-covered area using Metamorph image analysis. (f) Quantification of vessel density in the brain, defined as the ratio of Texas Red Lectin+ area to total DAPI area (n=4 for WT, n=6 for Ctss KO), and (g) assessment of vessel permeability by intraveneous injection of Evan's blue dye (n=8 for each group). (h) Quantification of BLI intensity in vivo at the indicated time points after tumor cell inoculation. Ctrl; Ctss WT (n=16), CTSS KD; Ctss WT (n=15), Ctrl; Ctss KO (n=11), and CTSS KD; Ctss KO (n=10). (i) Schematic of the regression trial experimental design. (j) Quantification of the BLI intensity from d0-d35 after Br-M tumor cell inoculation. At d27, mice were stratified into vehicle and VBY-999 treatment groups (n=7 per group) to achieve equal average BLI intensity at the time of treatment start at d28. Mice were dosed daily with either vehicle or VBY-999 (100 mg/kg) for 7 days and metastasis growth was monitored by BLI imaging and revealed no significant changes between vehicle and VBY-999 treated animals in an intervention trial. (k) Quantification of the BLI intensity in the bone and spine region of vehicle-treated and VBY-999-treated animals from the prevention trial, at d35 following Br-M tumor cell inoculation (n=23 for vehicle and n=21 for VBY-999). (I) Quantification of the BLI intensity in the bone and spine region at d35 following Br-M tumor cell inoculation, in the four experimental groups. Ctrl; Ctss WT (n=10), CTSS KD; Ctss WT (n=8), Ctrl; Ctss KO (n=10), and CTSS KD; Ctss KO (n=11). (m) Quantification of the BLI intensity in the head region (which may arise from skull and/or brain lesions) of vehicle-treated and VBY-999-treated animals from the prevention trial, at d35 following Bo-M tumor cell inoculation (n=12 for vehicle and n=13 for VBY-999). Graphs represent mean ± s.e.m or circles represent individual mice and horizontal lines represent the mean ± s.e.m. P values were obtained using two-tailed unpaired t-test. NS = not significant, *P<0.05.

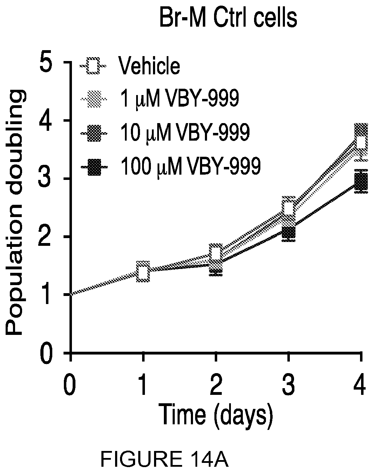

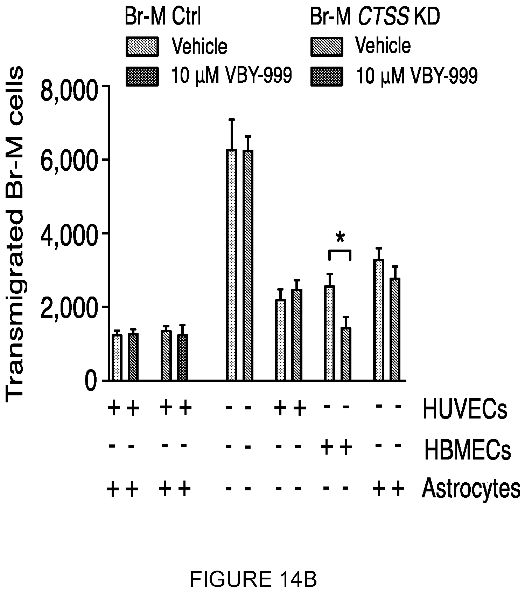

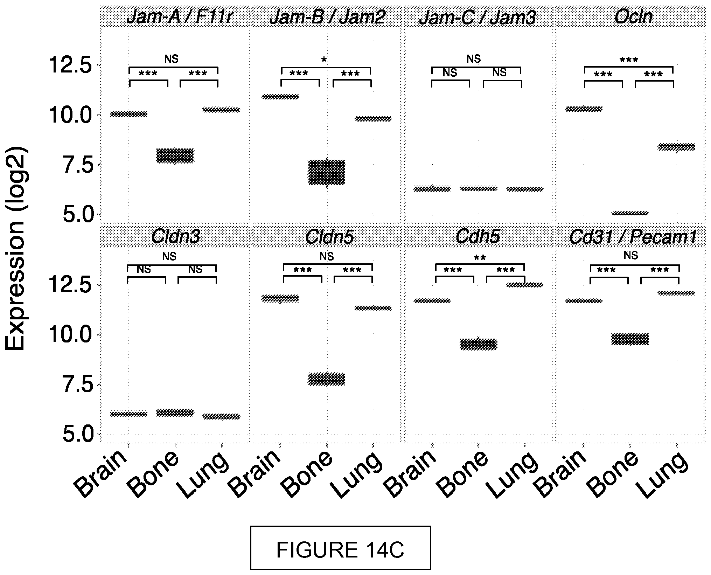

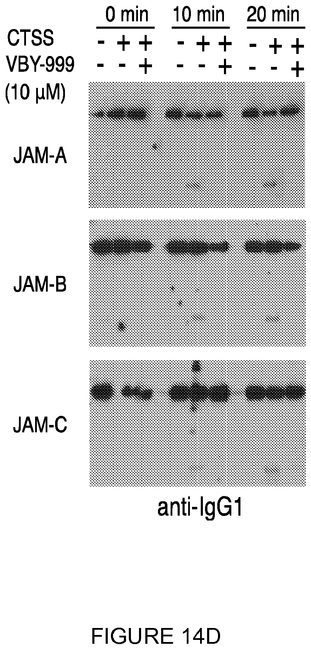

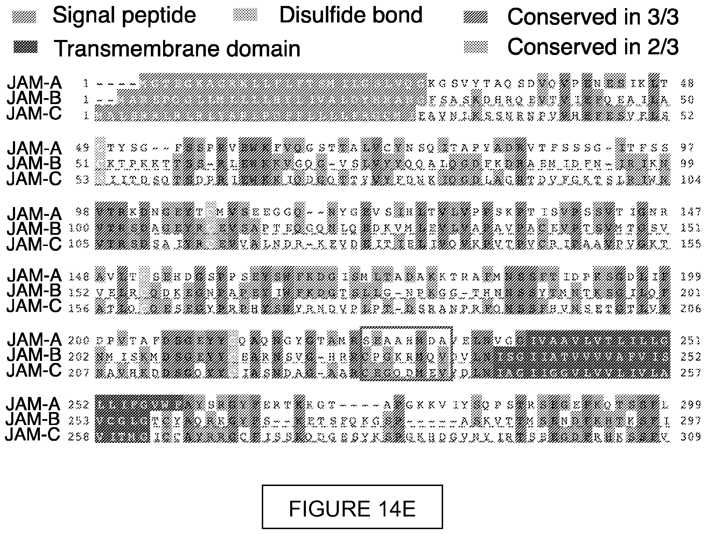

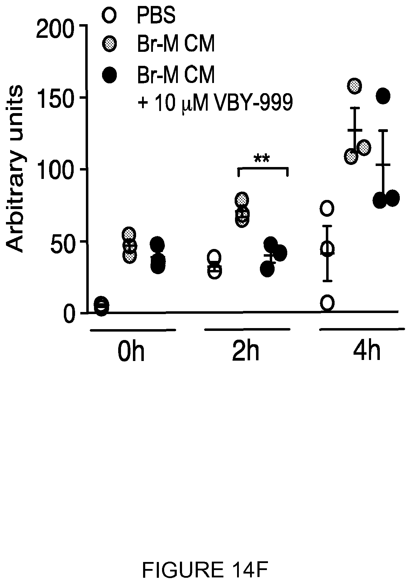

**Figure 14** shows that cathepsin S deficiency impairs transmigration in an *in vitro* BBB assay, and sequence analysis identifies a putative cleavage site for cathepsin S. (a) Pharmacological inhibition of cathepsin S with increasing concentrations of the cathepsin S-specific inhibitor VBY-999 (0 µM (Vehicle) to 100 µM) did not affect Br-M cell viability as determined by MTT assays (n=3 replicates). (b) Quantification of the number of transmigrated Br-M Ctrl and CTSS KD cells in the presence or absence of the cathepsin S-specific inhibitor VBY-999 through an in vitro BBB formed with human umbilical vein endothelial cells (HUVECs) or human brain microvascular endothelial cells (HBMECs) in co-culture with astrocytes and quantification of the number of transmigrated Br-M cells through Transwell chambers on which HUVECs, HBMECs or astrocytes were either seeded as monolayers, or controls in which none of these cells were seeded. (c) Expression of tight junction and adherens junction proteins was confirmed in an independent data set (GSE47067) of FACS sorted endothelial cells. Only Jam-B and Ocln are significantly enriched in brain endothelial cells compared to either lung or bone endothelial cells. (d) Western blot analysis of recombinant JAM-A, -B and -C cleavage by CTSS, in the presence or absence of the specific inhibitor VBY-999 (10 µM), using an antibody that detects the IgG1 domain in the recombinant JAM fusion proteins. (e) Alignment of the amino acid sequence of JAM-A, -B, and -C. Motifs that are conserved in all 3 family members are highlighted in dark purple, and motifs that are conserved in 2 of the 3 JAM family members are depicted in light purple. The putative cleavage location for cathepsin S is indicated by the red box. (f) Quantification of the three independent JAM-B cell based cleavage experiments. Graphs represent mean ± s.e.m. in (a) and (b), as box plots with whiskers to illustrate minimum and maximum values in (c). The horizontal line depicts the mean. Circles represent individual samples and horizontal lines represent the mean ± s.e.m. in (f). P values were obtained using two-tailed unpaired t-test. NS = not significant, *P<0.05, **P<0.01, and ***P<0.001.

### Detailed Description of the Invention

To facilitate understanding of the invention, the following definitions are provided. It is to be understood that, in general, terms are to be given their ordinary meaning or meanings as generally accepted in the art unless otherwise indicated. The terminology used herein is for the purpose of describing particular embodiments only and is not intended to limit the scope of the present invention which will be limited only by the appended claims.

In practicing the present invention, many conventional techniques in molecular biology are used. These techniques are described in greater detail in, for example, Molecular Cloning: a Laboratory Manual 3rd edition, J.F. Sambrook and D.W. Russell, ed. Cold Spring Harbor Laboratory Press 2001 and DNA Microarrays: A Molecular Cloning Manual. D. Bowtell and J. Sambrook, eds. Cold Spring Harbor Laboratory Press 2002.

In the description that follows, a number of terms are used extensively. The following definitions are provided to facilitate understanding of the invention. Unless otherwise specified, "a," "an," "the," and "at least one" are used interchangeably and mean one or more than one.

The terms "cancer", "cancerous", or "malignant" refer to or describe the physiological condition in mammals that is typically characterized by unregulated growth of tumor cells. Examples of a blood cancer include but are not limited to acute myeloid leukemia.

The term "diagnose" as used herein refers to the act or process of identifying or determining a disease or condition in a mammal or the cause of a disease or condition by the evaluation of the signs and symptoms of the disease or disorder. Usually, a diagnosis of a disease or disorder is based on the evaluation of one or more factors and/or symptoms that are indicative of the disease. That is, a diagnosis can be made based on the presence, absence or amount of a factor which is indicative of presence or absence of the disease or condition. Each factor or symptom that is considered to be indicative for the diagnosis of a particular disease does not need be exclusively related to said particular disease; i.e. there may be differential diagnoses that can be inferred from a diagnostic factor or symptom. Likewise, there may be instances where a factor or symptom that is indicative of a particular disease is present in an individual that does not have the particular disease.

"Expression profile" as used herein may mean a genomic expression profile. Profiles may be generated by any convenient means for determining a level of a nucleic acid sequence e.g. quantitative hybridization of microRNA, labeled microRNA, amplified microRNA, cRNA, etc., quantitative PCR, ELISA for quantitation, and the like, and allow the analysis of differential gene expression between two samples. A subject or patient tumor sample, e.g., cells or collections thereof, e.g., tissues, is assayed. Samples are collected by any method known in the art.

The term "expression product" or "gene expression product" as referred to herein may be a protein or a transcript (i.e., an RNA molecule transcribed from the gene).

"Gene" as used herein may be a natural (e.g., genomic) gene comprising transcriptional and/or translational regulatory sequences and/or a coding region and/or non- translated sequences (e.g., introns, 5'- and 3 '-untranslated sequences). The coding region of a gene may be a nucleotide sequence coding for an amino acid sequence or a functional RNA, such as tRNA, rRNA, catalytic RNA, siRNA, miRNA or antisense RNA. The term "gene" has its meaning as understood in the art. However, it will be appreciated by those of ordinary skill in the art that the term "gene" has a variety of meanings in the art, some of which include gene regulatory sequences (e.g., promoters, enhancers, etc.) and/or intron sequences, and others of which are limited to coding sequences. It will further be appreciated that definitions of "gene" include references to nucleic acids that do not encode proteins but rather encode functional RNA molecules such as tRNAs. For the purpose of clarity we note that, as used in the present application, the term "gene" generally refers to a portion of a nucleic acid that encodes a protein; the term may optionally encompass regulatory sequences. This definition is not intended to exclude application of the term "gene" to non-protein coding expression units but rather to clarify that, in most cases, the term as used in this document refers to a protein coding nucleic acid.

"Mammal" for purposes of treatment or therapy refers to any animal classified as a mammal, including humans, domestic and farm animals, and zoo, sports, or pet animals, such as dogs, horses, cats, cows, *etc.* Preferably, the mammal is human.

"Microarray" refers to an ordered arrangement of hybridizable array elements, preferably polynucleotide probes, on a substrate or solid support. Hybridization of sample RNA or DNA via complementary sequences (probes) allows the determination of the level of gene expression in the sample tested.

Therapeutic agents for practicing a method of the present invention include, but are not limited to, inhibitors of the expression or activity of genes identified and disclosed herein, or protein translation thereof. An "inhibitor" is any substance which retards or prevents a chemical or physiological reaction or response. Common inhibitors include but are not limited to antisense molecules, antibodies, and antagonists.

The term "poor" as used herein may be used interchangeably with "unfavorable." The term "good" as used herein may be referred to as "favorable." The term "poor responder" as used herein refers to an individual whose cancer grows during or shortly thereafter standard therapy, for example radiation-chemotherapy, or who experiences a clinically evident decline attributable to the cancer. The term "respond to therapy" as used herein refers to an individual whose tumor or cancer either remains stable or becomes smaller / reduced during or shortly thereafter standard therapy, for example radiation-chemotherapy.

"Probes" may be derived from naturally occurring or recombinant single-or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or *in situ* hybridizations to determine whether DNA or RNA encoding a certain protein is present in a cell type, tissue, or organ.

The term "prognosis" as used herein refers to a prediction of the probable course and outcome of a clinical condition or disease. A prognosis of a patient is usually made by evaluating factors or symptoms of a disease that are indicative of a favorable or unfavorable course or outcome of the disease. The phrase "determining the prognosis" as used herein refers to the process by which the skilled artisan can predict the course or outcome of a condition in a patient. The term "prognosis" does not refer to the ability to predict the course or outcome of a condition with 100% accuracy. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition. A prognosis may be expressed as the amount of time a patient can be expected to survive. Alternatively, a prognosis may refer to the likelihood that the disease goes into remission or to the amount of time the disease can be expected to remain in remission. Prognosis can be expressed in various ways; for example prognosis can be expressed as a percent chance that a patient will survive after one year, five years, ten years or the like. Alternatively prognosis may be expressed as the number of months, on average, that a patient can expect to survive as a result of a condition or disease. The prognosis of a patient may be considered as an expression of relativism, with many factors effecting the ultimate outcome. For example, for patients with certain conditions, prognosis can be appropriately expressed as the likelihood that a condition may be treatable or curable, or the likelihood that a disease will go into remission, whereas for patients with more severe conditions prognosis may be more appropriately expressed as likelihood of survival for a specified period of time.

The term "relapse" or "recurrence" as used in the context of cancer in the present application refers to the return of signs and symptoms of cancer after a period of remission or improvement.

As used herein a "response" to treatment may refer to any beneficial alteration in a subject's condition that occurs as a result of treatment. Such alteration may include stabilization of the condition (e.g., prevention of deterioration that would have taken place in the absence of the treatment), amelioration of symptoms of the condition, improvement in the prospects for cure of the condition. One may refer to a subject's response or to a tumor's response. In general these concepts are used interchangeably herein.

"Treatment" or "therapy" refer to both therapeutic treatment and prophylactic or preventative measures. The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/ or relieve to some extent one or more of the symptoms associated with the disorder.

For the recitation of numeric ranges herein, each intervening number there between with the same degree of precision is explicitly contemplated. For example, for the range of 2-5, the numbers 3 and 4 are contemplated in addition to 2 and 5, and for the range 2.0-3.0, the number 2.0, 2.1, 2.2, 2.3, 2.4, 2.5, 2.6, 2.7, 2.8, 2.9 and 3.0 are explicitly contemplated. As used herein, the term "about" X or "approximately" X refers to +/-10% of the stated value of X.

Inherent difficulties in the diagnosis and treatment of cancer include among other things, the existence of many different subgroups of cancer and the concomitant variation in appropriate treatment strategies to maximize the likelihood of positive patient outcome. Current methods of cancer treatment are relatively non-selective. Typically, surgery is used to remove diseased tissue; radiotherapy is used to shrink solid tumors; and chemotherapy is used to kill rapidly dividing cells.

Often, diagnostic assays are directed by a medical practitioner treating a patient, the diagnostic assays are performed by a technician who reports the results of the assay to the medical practitioner, and the medical practitioner uses the values from the assays as criteria for diagnosing the patient. Accordingly, the component steps of the method of the present invention may be performed by more than one person.

Prognosis may be a prediction of the likelihood that a patient will survive for a particular period of time, or said prognosis is a prediction of how long a patient may live, or the prognosis is the likelihood that a patent will recover from a disease or disorder. There are many ways that prognosis can be expressed. For example prognosis can be expressed in terms of complete remission rates (CR), overall survival (OS) which is the amount of time from entry to death, disease-free survival (DFS) which is the amount of time from CR to relapse or death. In one embodiment, favorable likelihood of survival, or overall survival, of the patient includes survival of the patient for about eighteen months or more.

A prognosis is often determined by examining one or more prognostic factors or indicators. These are markers, the presence or amount of which in a patient (or a sample obtained from the patient) signal a probability that a given course or outcome will occur. The skilled artisan will understand that associating a prognostic indicator with a predisposition to an adverse outcome may involve statistical analysis. Additionally, a change in factor concentration from a baseline level may be reflective of a patient prognosis, and the degree of change in marker level may be related to the severity of adverse events. Statistical significance is often determined by comparing two or more populations, and determining a confidence interval and/or a p value. See, e.g., Dowdy and Wearden, Statistics for Research, John Wiley & Sons, New York, 1983. In one embodiment, confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. Exemplary statistical tests for associating a prognostic indicator with a predisposition to an adverse outcome are described.

One approach to the study of cancer is genetic profiling, an effort aimed at identifying perturbations in gene expression and/or mutation that lead to the malignant phenotype. These gene expression profiles and mutational status provide valuable information about biological processes in normal and disease cells. However, cancers differ widely in their genetic signature, leading to difficulty in diagnosis and treatment, as well as in the development of effective therapeutics. Increasingly, gene mutations are being identified and exploited as tools for disease detection as well as for prognosis and prospective assessment of therapeutic success.

The inventors of the instant application hypothesized that gene expression profiling of brain metastasis would provide a more effective approach to cancer management and/or treatment. The inventors have herein identified that altered expression of a panel of genes is predictive of metastasis and likelihood of metastasis free survival (MFS).

In particular, the present disclosure is directed, *inter alia,* to a method of predicting the likelihood that a patient with cancer will develop metastasis to the brain, bone and/or lung. The method includes isolating a sample from the patient's blood, primary tumor or metastatic tumor, then assaying the sample to determine the expression of cathepsin S (CTSS) gene plus expression in at least one of genes *PSMB6, PSMD11, SLPI, PSMD13, and TIMP1* in said sample. After this assaying step, the method includes (i) predicting that the patient with cancer will develop metastasis to the brain if, in addition to increased expression of cathepsin S in the sample over control, expression of *PSMB6* gene is increased over control, (ii) predicting that the patient with cancer will develop metastasis to the bone if in the sample over control, expression of *PSMD11* and *SLPI* gene is increased over control, and (iii) predicting that the patient with cancer will likely not develop metastasis to the bone if in the sample over control, expression of PSMD13 and TIMP1 is increased. The primary cancer can be breast cancer. cathepsin S and *PSMB6* can be differentially expressed by both the stroma and tumor in early and late stage brain metastasis. PSMD11, SLPI, PSMD13, and TIMP1 can be differentially expressed by both the stroma and tumor in early and late stage bone metastasis.

Methods of monitoring gene expression by monitoring RNA or protein levels are known in the art. RNA levels can be measured by methods known to those of skill in the art including, for example, differential screening, subtractive hybridization, differential display, and microarrays. A variety of protocols for detecting and measuring the expression of proteins, using either polyclonal or monoclonal antibodies specific for the proteins, are known in the art. Examples include Western blotting, enzyme-linked immunosorbent assay (ELISA), radioimmunoassay (RIA), and fluorescence activated cell sorting (FACS).

Some methods require the use of probes and primers specific for an RNA transcript or other expression product of a gene of interest. A probe comprises an isolated nucleic acid attached to a detectable label or other reporter molecule. Typical labels include radioactive isotopes, enzyme substrates, cofactors, ligands, chemiluminescent or fluorescent agents, haptens, and enzymes. Methods for labeling and guidance in the choice of labels appropriate for various purposes are discussed, e.g., in Sambrook et al. (In Molecular Cloning: A Laboratory Manual, CSHL, New York, 1989) and Ausubel et al. (In Current Protocols in Molecular Biology, John Wiley & Sons, New York, 1998).

Primers are short nucleic acid molecules, for instance DNA oligonucleotides 10 nucleotides or more in length. Longer DNA oligonucleotides may be about 15, 20, 25, 30 or 50 nucleotides or more in length. Primers can be annealed to a complementary target DNA strand by nucleic acid hybridization to form a hybrid between the primer and the target DNA strand, and then the primer extended along the target DNA strand by a DNA polymerase enzyme. Primer pairs can be used for amplification of a nucleic acid sequence, e.g., by the polymerase chain reaction (PCR) or other in vitro nucleic-acid amplification methods known in the art. These methods are within the skill of the ordinary artisan.

Diseases associated with bone metastasis include cancers that spread from the primary tumor located in one part of the body to another. For example, an individual with prostate cancer may have a metastasis in their bone. Cells that metastasize are basically of the same kind as those in the original tumor, i.e.; if the cancer arose in the lung and metastasized to the bone, the cancer cells growing in the bone are lung cancer cells. Metastatic-associated diseases which may be treated by methods of the invention include, but are not limited to, skin cancer, brain cancer, ovarian cancer, breast cancer, cervical cancer, colorectal cancer, prostate cancer, liver cancer, lung cancer, stomach cancer, bone cancer, and pancreatic cancer.

The drug combination of the invention may be used for the treatment of humans or animals with cancer, including domestic, sport, laboratory, and farm animals. It is contemplated that the each component of the drug combination may be formulated into a pharmaceutical composition comprising an effective amount of the component and a pharmaceutically acceptable carrier. An effective amount of each component of the drug combination may be administered to the patient in a manner which, when combined with the other components of the drug combination, ultimately decreases the signs or symptoms of a disease associated with a bone metastasis. Examples of signs and/or symptoms that may be monitored to determine the effectiveness of the drug combination include, but are not limited to, PSA level, bone resorption, tumor size, feelings of weakness, and pain perception. Beneficial effects of the instant drug combination may, for instance, include a 50%, 75% or 100% drop in PSA levels or a reduction in tumor size by 50%, 75% or 100%. The amount of each component and the specific pharmaceutically acceptable carrier will vary depending upon, for example, the component being administered, the patient and the condition of this patient, the mode of administration, and the type of cancer being treated.

The present disclosure is also directed to a method of predicting the likelihood that a patient with cancer will develop metastasis to the bone. The method comprises isolating a genetic sample from the patient's blood, primary tumor or metastatic tumor, and subsequently assaying the genetic sample to determine the expression in at least one of genes *PSMD11, TIMP1, PSMD13,* and *SLPI* in said sample. Having assayed for expression of these genes, the method includes (i) predicting that the patient with cancer will develop metastasis to the bone if in the sample, expression of *PSMD11* and *SLPI* is increased over control, and/or (ii) predicting that the patient with cancer will likely not develop metastasis to the bone if in the sample, expression of *PSMD13* and *TIMP1* is increased over control.

As well as brain and bone, metastasis to the lung is also of concern. For example, metastatic breast cancer, either at the time of initial diagnosis or upon recurrence after an initial treatment, commonly occurs in the bone, lung, brain or liver. Between 60% and 70% of women who die from breast cancer have metastatic lung involvement, and in a significant number of cases the lung is the only site of metastasis. The most common signs of lung metastases are: shortness of breath and dry cough. In some cases, women will not experience any symptoms; cancer will only be detected by chest X-ray or CT scan. Thus, the ability to identify early on those cancers that pose the greatest risk of lung metastasis over time would provide an improved prognosis through the use of increased monitoring. The present disclosure also teaches methods that relate using genes that are shown in Fig. 11E to predict the likelihood of patients with primary or metastatic tumors later developing lung metastasis.

Cathepsins are lysosomal cysteine proteases that belong to the papain superfamily. They are widely distributed and differentially expressed among tissues. These enzymes have a role in processes that involve proteolysis and turnover of specific proteins and tissues. Cathepsins also participate to proenzyme activation and to antigen presentation by MHC class 2 proteins in antigen-presenting cells. The various members of this family are differentially expressed, and some forms of cathepsins are closely associated with monocytes, macrophages, and other cells of the immune system. The secreted forms of several members of this family function in tissue remodelling through degradation of collagen, fibronectin, laminin, elastin, and other structural proteins and are implicated in the inflammatory response.

Cathepsin S, also known as CTSS, is a protein that in humans is encoded by the *CTSS* gene. The term "cathepsin S" has its general meaning in the art and refers to a secreted cysteine protease from the family of cathepsins. The term may include naturally occurring "cathepsin S" and variants and modified forms thereof. The term may also refer to fusion proteins in which a domain from cathepsin S that retains the cathepsin S activity is fused, for example, to another polypeptide (e.g., a polypeptide tag such as are conventional in the art). The cathepsin S can be from any source, but typically is a mammalian (e.g., human and non- human primate) cathepsin S, particularly a human cathepsin S. An exemplary native cathepsin S amino acid sequence is provided in GenPept database under accession number AAB22005 and an exemplary native nucleotide sequence encoding for cathepsin S is provided in GenBank database under accession number NM 004079.

The expression "inhibitor of cathepsin S" should be understood broadly; it encompasses inhibitors of cathepsin S activity and inhibitors of cathepsin S expression. An "inhibitor of expression" refers to a natural or synthetic compound that has a biological effect to inhibit or significantly reduce the expression of a gene. Consequently an "inhibitor of cathepsin S expression" refers to a natural or synthetic compound that has a biological effect to inhibit or significantly reduce the expression of the gene encoding for the cathepsin S gene.

Particularly, a "selective inhibitor of cathepsin S expression" refers to such compound which inhibits the cathepsin S expression more strongly than that of cathepsins L or K expression in the sense that the inhibitor is at least 10 times, more preferably at least 100 times and most preferably at least 1000 times stronger inhibitor of the cathepsin S expression.

An "inhibitor of activity" has its general meaning in the art, and refers to a compound (natural or not) which has the capability of reducing or suppressing the activity of a protein. It can be an antibody which binds the activity site of cathepsin S and inhibits its activity. Particularly, a "selective inhibitor of cathepsin S activity" refers to such compound which inhibits the cathepsin S activity more strongly than that of cathepsins L and K activity in the sense that the inhibitor is at least 10 times, more preferably at least 100 times and most preferably at least 1000 times stronger inhibitor of the cathepsin S activity. As used herein, the term "subject" denotes a mammal, such as a rodent, a feline, a canine, and a primate. Preferably, a subject according to the invention is a human.

One aspect of the present disclosure a method of predicting metastasis of breast cancer to the brain, bone and/or lung of a patient suffering from breast cancer. The method comprises obtaining a sample from the patient and analyzing it for increased expression of cathepsin S. The method includes (i) predicting the breast cancer patient has or is at risk of developing metastasis to the brain if there is increased expression of Cathepsin S gene in tumor cells early on in brain metastasis development, relative to control; and (ii) predicting the breast cancer patient is not likely to develop metastasis to the bone and lung if there is increased expression of Cathepsin S gene in tumor cells early on in brain metastasis development. Macrophages and/or primary tumor cells may be isolated as the sample.

In addition to increased expression of cathepsin S, the expression of one or more of the twenty one other genes shown in FIG 10C were increased with brain metastasis. Further, the expression of one or more of the twenty five other genes shown in FIG 10D were increased with bone metastasis. Further, the expression of one or more of the forty two other genes shown in FIG 10E were increased with lung metastasis.

In one embodiment, in its broadest meaning, the term "preventing" or "prevention" refers to preventing the onset of or advancement of brain metastasis formation in a subject or subject at risk of developing, for example, brain, bone or lung metastasis.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type.

One aspect of the present disclosure relates to methods and compositions (such as pharmaceutical compositions) for treating and/or preventing metastatic cancer associated disorders, for example, brain metastasis.

The disclosure relates, *inter alia,* to the use of inhibitors of cathepsin S activity for the treatment of brain metastasis and associated disorders. Particularly, the disclosure relates to the use of selective inhibitors of cathepsin S activity for the treatment and/or impairment of of brain metastasis outgrowth. In another embodiment, the disclosure relates to the use of inhibitors of cathepsin S expression for the treatment of brain metastasis. Particularly, the invention relates to the use of selective inhibitors of cathepsin S expression for the treatment of brain metastasis.

In particular, the present disclosure is, in one example, directed to a method of treating, preventing or managing metastasis of cancer cells from a primary tumor in a cancer patient to the patient's brain, bone and/or lung. The method comprises administering to the patient with cancer, for example breast cancer, an agent which inhibits cathepsin S. The agent can be a selective inhibitor of cathepsin S relative to a cysteine protease selected from cathepsins K, L, H and B. Alternatively, the agent can be a specific inhibitor of cathepsin S. The agent can be a peptide-based inhibitor of cathepsin S, which is based upon a peptide sequence which comprises 2-20 consecutive residues of a preferred invariant chain cleavage site of cathepsin S. The agent may be administered to the patient suffering from cancer via intravenous injection, intradermal injection, subcutaneous injection, intramuscular injection, intraperitoneal injection, anal supposition, vaginal supposition, oral ingestion or inhalation. The cathepsin S inhibitor can be administered early on in the metastasis development cascade.

Cathepsin S inhibitors are known in the art and some are already approved or currently in clinical trials for indications such as systemic lupus erythematosus (SLE), psoriasis and irritable bowel syndrome. One example, VBY-129 (commercially available from Virobay, Inc.), is a potent, competitive and reversible inhibitor of purified cathepsin S that is also highly selective against other human cathepsins (B, F, L, K and V). VBY-129 has potent activity in cellular assays and in animal models of disease.

The peptide-based inhibitor of cathepsin S can be morpholinurealeucine-homophenyl alanine-vinylsulfone phenyl (LHVS). The peptide-based inhibitor can be a peptide-based vinylsulfone or a modified peptide-based vinylsulfone. The peptide-based inhibitor can be selected from peptidyl aldehydes, nitriles, α-ketocarbonyls, halomethyl ketones, diazomethyl ketones, (acyloxy)-methyl ketones, vinyl sulfones, ketomethylsulfonium salts, epoxides, and N-peptidyl-O-acyl-hydroxylamines. The agent can be selected from Asn-Leu-vinylsulfone, Arg-Met-vinylsulfone, Leu-Arg-Met-vinylsulfone, Glu-Asn-Leu-vinylsulfone, and Leu-Leu-Leu-vinylsulfone. The agent can be selected from N-(carboxybenzyl)-Asn-Leu-vinylsulfone, N-(carboxybenzyl)-Arg-Met-vinylsulfone, N-(carboxybenzyl)-Leu-Arg-Met-vinylsulfone, N-(carboxybenzyl)-Glu-Asn-Leu-vinylsulfone, and N-(carboxybenzyl)-Leu-Leu-Leu-vinylsulfone.

One aspect of the present disclosure is a method of treating, preventing or managing cancer cell metastasis in a cancer patient. The method comprises extracting a sample from the primary tumor, metastatic tumor, or blood of the cancer patient, and then assaying the sample to determine the expression of cathepsin S and/ or *PSMB6* genes in said sample, and subsequently administering a cathepsin S inhibitor if the expression of cathepsin S and/ or *PSMB6* genes is increased over control.

In one embodiment, the inhibitor of cathepsin S activity may be an inhibitor of activity of this cathepsin, e.g. a small organic molecule. Several molecules have been described as inhibitors of cathepsin S activity. According to the invention, inhibitors of cathepsin S activity that could be used are described in Gauthier JY et al, 2007. (The identification of potent, selective, and bioavailable cathepsin S inhibitors. Bioorganic & Medicinal Chemistry Letters 17 (2007) 4929-4933).

Other examples of molecules that could be used are: the Paecilopeptin, the dipeptide α-keto-β-aldehyde or the 4- Morpholineurea-Leu-HomoPhe-vinylsulphone (LHVS) or an antibody against cathepsin S described in the patent application WO2007128987. These molecules can also derive from the development of ligand-based and structure-based pharmacophore models for noncovalent and covalent cathepsin S inhibitors (Markt et al.: Discovery of novel cathepsin S inhibitors by pharmacophore-based virtual high-throughput screening. J Chem Inf Model 48:1693-1705, 2008) or pyrrolopyrimidine-based inhibitors (Irie et al.: Discovery of selective and nonpeptidic cathepsin S inhibitors. Bioorg Med Chem Lett 18:3959-3962, 2008).

In another embodiment, the inhibitor of cathepsin S activity is an antibody or antibody fragment that can partially or completely blocks the cathepsin S enzymatic activity (i.e. a partial or complete cathepsin S blocking antibody or antibody fragment). In particular, the inhibitor of cathepsin S activity may consist in an antibody directed against the cathepsin S, in such a way that said antibody blocks the activity of cathepsin S. Antibodies directed against the cathepsin S can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies against cathepsin S can be prepared and isolated using any technique that provides for the production of antibody molecules by continuous cell lines in culture. Techniques for production and isolation include but are not limited to the hybridoma technique originally described by Kohler and Milstein; the human B-cell hybridoma technique and the EBV-hybridoma technique. Alternatively, techniques described for the production of single chain antibodies (e.g., U.S. Pat. No. 4,946,778) can be adapted to produce anti- cathepsin S, single chain antibodies. Cathepsin S inhibitors useful in practicing the present invention also include anti-cathepsin S fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to cathepsin S.

Humanized and human anti-cathepsin S antibodies and antibody fragments thereof can also be prepared according to known techniques. Methods for making antibodies, are well known in the art.

In still another embodiment, the inhibitor of cathepsin S activity is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods.

One aspect is directed to a kit for determining treatment of a patient with brain metastasis. The kit comprises means for detecting expression and/or activity of cathepsin S and/or *PSMB6* genes at an early stage of brain metastasis. The kit also includes instructions for recommended treatment based on the presence of increased expression or activity in cathepsin S and/or *PSMB6* genes.

### Inhibitor of cathepsin S expression

Another aspect of the invention relates to selective inhibitor of cathepsin S expression. Inhibitors of cathepsin S expression for use in the present invention may be based on anti- sense oligonucleotide constructs. Anti-sense oligonucleotides, including anti-sense RNA molecules and anti-sense DNA molecules, would act to directly block the translation of Cathepsin S mRNA by binding thereto and thus preventing protein translation or increasing mRNA degradation, thus decreasing the level of Cathepsin S, and thus activity, in a cell. For example, antisense oligonucleotides of at least about 15 bases and complementary to unique regions of the mRNA transcript sequence encoding Cathepsin S can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732).

Small inhibitory RNAs (siRNAs) can also function as inhibitors of Cathepsin S expression for use in the present invention. Cathepsin S expression can be reduced by contacting a subject or cell with a small double stranded RNA (dsRNA), or a vector or construct causing the production of a small double stranded RNA, such that cathepsin S expression is specifically inhibited (i.e. RNA interference or RNAi). Methods for selecting an appropriate dsRNA or dsRNA-encoding vector are well known in the art for genes whose sequence is known (see U.S. Pat. Nos. 6,573,099 and 6,506,559) and International Patent Publication Nos. WO 01/36646, WO 99/32619, and WO 01/68836). shRNAs (short hairpin RNA) can also function as inhibitors of Cathepsin S expression for use in the present invention.

Antisense sequences to cathepsin S may readily be chosen and produced by one of ordinary skill in the art on the basis of the known nucleic acid sequence of the cathepsin S gene (see; e.g., GenBank Accession Nos. M86553, M90696, S39127; and Wiedersranders et at., J. Biol. Chem. 267; 13708-13713 (1992)). In order to be sufficiently selective and potent for cathepsin S inhibition, such cathepsin S-antisense oligonucleotides should comprise at least 10 bases and, more preferably, at least 15 bases. In one embodiment, the antisense oligonucleotides comprise 18-20 bases.

Ribozymes can also function as inhibitors of cathepsin S expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleo lyric cleavage. Engineered hairpin or hammerhead motif ribozyme molecules that specifically and efficiently catalyze endonucleolytic cleavage of cathepsin S mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable.

Both antisense oligonucleotides and ribozymes useful as inhibitors of cathepsin S expression can be prepared by known methods. These include techniques for chemical synthesis such as, for example, by solid phase phosphorothioate chemical synthesis. Alternatively, anti-sense RNA molecules can be generated by *in vitro* or *in vivo* transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a mean of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides, siRNAs, shRNAs and ribozymes of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and preferably cells expressing Cathepsin S. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

One class of vectors includes plasmid vectors. Plasmid vectors have been extensively described in the art and are well known to those of skill in the art. Recently, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript, pSIREN. Other plasmids are well known to those of ordinary skill in the art. Additionally, plasmids may be custom designed using restriction enzymes and ligation reactions to remove and add specific fragments of DNA. Plasmids may be delivered by a variety of parental, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In one embodiment, the disclosure relates to a pharmaceutical composition for treating and/or preventing brain metastasis and/or associated disorders, said composition comprising an inhibitor of cathepsin S expression and/or activity. In one embodiment, the inhibitor is a selective inhibitor of cathepsin S expression and/or activity.

The inhibitor(s) of cathepsin S may be combined with pharmaceutically acceptable excipients, and optionally sustained-release matrices, such as biodegradable polymers, to form therapeutic compositions.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

The inhibitor of cathepsin S of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants.

The inhibitor of cathepsin S of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

One aspect of the present disclosure is directed to a method of predicting the likelihood that a patient with cancer will develop metastasis to the bone. The first step of the method includes isolating a genetic sample from the patient's blood, primary tumor or metastatic tumor. The sample is then assayed to determine the expression of *ADAMDEC1* gene; and based on the expression profile, once can predict (i) that the patient with cancer will develop metastasis to the brain and/or lung if expression of *ADAMDEC1* is increased over control, and/or (ii) that the patient with cancer will not develop metastasis to the bone if expression of *ADAMDEC1* is increased over control.

Another aspect of the present disclosure is directed to a method of analyzing a cell expression profile for determining whether the cell is metastatic to the brain, bone or lung. The method comprises extracting the cell, measuring an amount of cathepsin S, *PSMB6, PSMD11, and SLPI* nucleic acid expression or polypeptide in the cell, and comparing the amount of cathepsin S, *PSMB6, PSMD11, and SLPI* nucleic acid expression or protein present in the cell to the amount of cathepsin S, *PSMB6, PSMD11, and SLPI* nucleic acid expression or polypeptide in a sample isolated from normal, non-cancerous cells. Having done so, an amplified amount of cathepsin S and *PSMB6* nucleic acid expression or polypeptide in the cell relative to the amount of cathepsin S and *PSMB6* nucleic acid expression or polypeptide in the sample isolated from normal, non-cancerous cells indicates that cancer is likely to metastasize to the brain. On the other hand, an amplified amount of *PSMD11* and *SLPI* nucleic acid expression or polypeptide in the cell relative to the amount of *PSMD11* and *SLPI* nucleic acid expression or polypeptide in the sample isolated from normal, non-cancerous cells indicates that cancer is likely to metastasize to the bone. The cell is, in one example, isolated from the patient's blood, primary tumor or metastatic tumor. In another example, the cell is isolated from a primary breast tumor or a metastatic breast tumor.

The disclosure is also directed to a method for preparing a personalized genomics profile for a patient with breast cancer. The method comprises extracting mononuclear cells or cancer cells from the primary tumor and subjecting them to gene expression analysis, and assaying the sample to determine the expression of cathepsin S gene plus expression of at least one of genes *PSMB6, PSMD11, and SLPI* in said sample. The method also includes generating a report of the data obtained by the expression analysis, wherein the report comprises a prediction of the likelihood of the patient being substantially free of metastasis to the brain if, in addition to decreased expression of cathepsin S in the sample over control, expression of *PSMB6* gene is also decreased over control. The disclosure further comprises, in one example, predicting that the patient with cancer will develop metastasis to the bone if, in the sample over control, expression of *PSMD11* and *SLPI* gene is increased over control.

### EXAMPLES

The invention, having been generally described, may be more readily understood by reference to the following examples, which are included merely for purposes of illustration of certain aspects and embodiments of the present invention, and are not intended to limit the invention in any way.

### Differential expression of proteases and protease inhibitors in different metastatic microenvironments

In order to investigate tumor-stroma interactions in different metastatic environments we used a mouse model for organ-specific experimental metastasis (Fig. 1a). In this model, three different metastatic variants of the human breast cancer cell line MDA-MB-231 (Refs. 23-25) were injected either intracardially or intravenously into immunocompromised mice, resulting in the development of brain, bone, or lung metastases. While previous studies focused on tumor cell-specific expression changes identified by profiling each of these metastatic variant cell lines in culture23-25, we have been able to additionally capture the stromal contribution by removing intact whole tumors at distinct stages of metastatic seeding and outgrowth in different organs, and subjecting them to expression analyses (Fig. 1 a, Fig. 8).

An important technological advance that allowed us to simultaneously query tumor and stromal gene expression on the same platform is the "HuMu Protln" custom array (Hu= Human, Mu= Murine, Prot= Protease and In= Inhibitor), which surveys the mRNA expression of proteases, their endogenous inhibitors and interacting partners26. The uniqueness of this array is based on the species-specificity of the probe sets, with no cross-reactivity between the human and mouse genes26. This platform thus allowed us to distinguish between expression changes in the tumor (human) and stromal (mouse) gene space in response to metastatic seeding and outgrowth (early- and late-stage metastases respectively, Fig. 8), with the goal of identifying tumor-stroma interactions that modulate organ-specific metastasis. Each of the metastatic cell variants was transduced with a triple-fusion TK-GFP-Luc imaging vector, enabling non-invasive bioluminescence imaging (BLI) as a read-out of metastatic burden, as previously described²⁷. Early- and late-stage metastases in each organ site were harvested based on BLI output, as described in further detail in the methods, and correspond to micrometastatic and macrometastatic disease respectively.

Principal component analysis (PCA) was used to evaluate the global trends in proteolytic network gene expression across tissue and stage for both tumor cells and stromal cells (Fig. 1b). Analysis of tumor cell-specific gene expression revealed pronounced changes between early- and late-stage metastases, across all three of the metastatic sites examined. Meanwhile, stromal genes were differentially expressed between early- and late-stage metastases in a tissue-dependent manner. Specifically, the brain and bone stroma showed the most robust changes in gene expression as metastases progressed from early- to late-stage. Across all tissues, there were few changes in gene expression between the normal tissue (i.e. non-tumor burdened), and the stroma of early metastases (brain, bone = 0 genes, lung = 3 genes, Table 8a). This could reflect the relatively low disease burden at the early stages, resulting in a minimal impact on the organ as a whole. Alternatively, this may indicate that expression changes in proteolytic genes in the stroma are not as important in the earliest stages of metastatic extravasation and seeding, possibly due to a predominant role for tumor-supplied proteases or non-protease factors in the stroma at this stage.

Differential gene expression analyses revealed that many genes changed in tumor cells in the brain (242 genes), bone (241 genes) and lung (245 genes) between early- and late-stage metastases (Fig. 1c, Table 1b-d). By comparison, there were fewer stage-specific differentially expressed genes identified in the stroma of the brain (40 genes) and bone (44 genes), and only one differentially expressed gene, haptoglobin, in the lung stroma (Fig. 1d, Table 1eg). In tumor cells, a substantial proportion of differentially expressed genes were shared across all three metastatic sites (176 genes, Fig. 9a), whereas few differentially expressed stromal genes were shared across >1 metastatic site (10 genes, Fig. 9b). Rather, there were multiple tissue-specific proteases and inhibitors in the brain, bone and lung stroma (Fig. 9c). Quantitative real-time PCR (qPCR) confirmed the tissue-specific enrichment for representative candidates for each organ site in normal tissue, and early- and late-stage metastases (Fig. 9d). Representative proteases and protease inhibitors that exhibited stage-specific gene expression changes between early and late metastases were validated using immunostaining (Fig. 10a-e) and qPCR (Fig. 10f-j). In addition, we validated several of the genes identified in lung metastasis xenografts (Table 1a) in the immunocompetent MMTV-PyMT model of breast-to-lung metastasis (Fig. 10k).

We also asked whether the expression changes in stromal cells in organ-specific metastases are a general response to tumor cell colonization of the respective tissue, or if the expression changes are specific to the metastatic cell variant used. In the models used herein, bone metastases occasionally develop in animals inoculated with the brain-metastatic (Br-M) variant, and conversely brain metastases can be observed in mice inoculated with bone-metastatic (Bo-M) cells. This allowed us to compare stromal and tumor gene expression in the 'matched' (Br-M to brain, Bo-M to bone) and 'mismatched' (Br-M to bone, Bo-M to brain) samples. Interestingly, for the genes tested, we found that stromal gene expression changes depend on tumor-stroma interactions that are specific to the metastatic tumor cell variant (Fig. 10f, g). By contrast, tumor gene expression in different metastatic variants responds to the same microenvironment in a similar manner, suggesting an important effect of the stroma on the tumor gene expression program (Fig. 10h, i).

### Cathepsin S is negatively associated with metastasis-free survival in patients with brain metastasis

While previous whole tumor analyses have been useful for identifying genes associated with site-specific metastases, few studies have been able to identify genes that are concordantly or discordantly expressed in tumor cells and stroma. To address this, we took advantage of the species specificity of the HuMu arrays to separately profile stroma- and tumor-derived genes in a cross-species analysis. We identified genes for which both human and mouse homologs were significantly altered in each metastatic site (Fig. 2a, Fig. 11a, b). Cathepsin S showed a particularly intriguing expression pattern: while tumor-derived cathepsin S was high in early brain metastases and decreased in late-stage metastases, stromal cathepsin S displayed the inverse pattern, with higher expression in late-stage brain metastases compared to early-stage. qPCR using species-specific probes for cathepsin S confirmed these data in an independent sample set (Fig. 2b). To distinguish between the cellular sources of cathepsin S, we will refer to tumor/ human CTSS in capitals, and stromal/ mouse Ctss in lower-case.

Using a publicly available gene expression dataset of locally advanced primary breast cancer with complete clinical annotation (GSE12276)23, we investigated whether there were any associations between CTSS expression at the breast primary site and organ-specific metastasis-free survival (MFS). Patients were separated into three equal tertiles of low, medium and high CTSS expression as described in the methods. Kaplan-Meier analysis was used to assess MFS for brain, bone and lung metastasis. Interestingly, the high CTSS expression group was associated with decreased MFS only for the brain (Fig. 2c). CTSS expression levels did not significantly associate with either bone or lung MFS. This was further evident in a complementary Cox proportional hazards model analysis with a hazard ratio (HR) of 1.4 for brain MFS alone (95% confidence interval (C.I.) 1.05-1.89; P = 0.0209) (Fig. 11c).

We used similar analyses to determine if other genes that were differentially expressed between early- and late-stage metastases in the experimental model (Fig. 1c) were also associated with differences in patient survival (Table 2). We found that in addition to CTSS, 26 other genes were significantly associated with brain MFS. Of these, 23 genes were negatively associated with brain MFS. Only TPSG1, HNRNPC or SEPT2 expression was associated with improved brain MFS (Fig. 11c, 1 Table 2a). 30 genes were associated with bone MFS, of which 6 genes (MME, SNRNP200, PSMB3, SLPI, PSMD10, and PSMD11) were negatively associated with bone MFS (Fig. 11d, Table 2b). 59 genes were associated with lung MFS, of which 45 genes were negatively associated with lung MFS (Fig. 11e, Table 2c). Only one gene, SLPI, was found to be associated with MFS in brain, bone and lung, where high expression correlated with poor patient prognosis (Fig. 11c-e). Although tumor cells underwent largely congruent changes in gene expression from early- to late-stage metastases across the three metastatic sites (Fig. 9a), only 20 of these genes were significantly associated with MFS at multiple sites, whereas the majority of genes were associated with tissue-specific MFS (brain = 11 genes, bone = 24 genes, lung = 40 genes) (Fig. 11f, Table 2a-c).

CST7 in brain metastasis, together with CTSS and SERPINA3 in bone metastasis, were the only genes that showed the same stage-dependent and cell type-specific expression changes as CTSS in brain metastasis (Fig. 2a, Fig. 11a). Given that we did not observe an association of CTSS expression with patient bone MFS, and neither CST7 nor SERPINA3 expression associated with brain and bone MFS respectively (data not shown), we chose to further investigate the potential role of cathepsin S specifically in brain metastasis, a function not previously ascribed to this protease, or any cathepsin family member.

The patient expression data above was derived from whole tumor samples, thus precluding cell type-specific expression analyses. We therefore utilized an independent set of patient tissue samples of brain metastases, with matched primary breast tumors in approximately half of the cases (Table 3). Across all samples (breast cancer and brain metastases), we found that the major cell types contributing to the tumor mass were cytokeratin (CK)+ tumor cells (55-85%) and CD68+ macrophages (10-35%), with a minor fraction representing CK-CD68- cells (Fig. 2d, e, Fig. 12a-d). We examined the cellular source of CTSS and found that the highest level of CTSS staining (CTSS index) was in CD68+ macrophages. CTSS was also expressed in CK+ tumor cells, albeit at lower levels than in macrophages, in both primary tumors and matched brain metastases (Fig. 2d, f, Fig. 12a, b, e). Notably, CTSS expression in tumor cells was found in all molecular subtypes of breast cancer analyzed here (Fig. 2d, f, Fig. 12a, b, e, Table 3).

### Combined depletion of cathepsin S in tumor and stromal cells reduces experimental brain metastasis

We next investigated the stromal cell source of Ctss in the experimental brain metastasis model. Seeding and outgrowth of brain metastasis induced a pronounced stromal response that was characterized by an accumulation of astrocytes and macrophages/microglia in metastatic lesions (Fig. 8d). Detection of cathepsin S using an antibody that recognizes both mouse and human homologs, in combination with a range of cell-type specific markers, identified macrophages as the predominant stromal cell type expressing Ctss in brain metastases and normal brain (Fig. 3a). We observed a gradual increase of Ctss expression in Iba1+ macrophages from normal brain to early- and late-stage metastases. Interestingly, Ctss expression was highly induced in Iba1+ macrophages that were localized in close proximity to metastases. CTSS expression was also detectable in tumor cells, though at lower levels than in macrophages, mirroring the patient analyses. At late stages, CTSS expression was undetectable in the majority of the tumor cells. We found a similar expression pattern in an immunocompetent brain metastasis model (Fig. 10I). These data confirm the stage- and cell type-dependent expression changes at the protein level as predicted by the HuMu array.

Given the reciprocal, cell type-specific expression pattern of cathepsin S, we next sought to investigate if the tumor and stromal sources play important, perhaps complementary roles in the seeding and outgrowth of experimental brain metastases. To address this, we performed short hairpin (sh)-RNA-mediated CTSS knockdown (KD) in the brain metastatic (Br-M) cells, achieving a 90% reduction of CTSS expression at both the mRNA and protein level, and a corresponding reduction in secreted CTSS protein (Fig. 13a-c). There was no effect of CTSS knockdown on tumor cell proliferation in culture (Fig. 13d). After backcrossing Ctss knockout (KO) mice28 into the Athy/nu background, we generated four experimental groups (shown in Fig. 3b) to analyze the effects of targeting tumor or stromal cathepsin S alone, or in combination, compared to the control group. Interestingly, only the combined removal of tumor and stromal cathepsin S significantly reduced brain metastasis incidence, as monitored by BLI output (Fig. 3b, Control (Ctrl); Ctss wild-type (WT) vs. CTSS KD; Ctss KO, P<0.001), whereas targeting either source separately had no effect. A separate cohort of mice for all four experimental groups was aged until day 35 after tumor cell injection, which was selected as the time point by which all mice in the control group had developed brain metastases (Fig. 3b). Ex vivo BLI analysis of the brain at this endpoint revealed a significant 64% decrease in signal output in the CTSS KD; Ctss KO group alone (Fig. 3c, d). Together, these results indicate that while there is a stage-dependency to cell type-specific cathepsin S expression, contributions from both cellular sources are required to regulate brain metastasis.

### Cathepsin S promotes transmigration of the blood-brain barrier by metastatic cells

To gain insights into the mechanisms underlying impaired metastatic seeding and/ or outgrowth specifically in the CTSS KD; Ctss KO group, we next analyzed multiple tumorigenic processes in brain metastases at day 35. We found that both the size and proliferation rate of tumors in CTSS KD; Ctss KO mice were significantly lower than any of the other groups (Fig. 3c-e), while we did not observe significant differences in the apoptosis rate at d35 between the experimental groups (data not shown). In investigating these phenotypes, it was evident that the small lesions which did develop in the CTSS KD; Ctss KO mice were closely apposed to the vasculature, with the majority of tumor cells being only 1 cell diameter from the vessel, and there was a pronounced reduction in growth at a distance from blood vessels (Fig. 4a, b). Similarly, analysis of the area covered by GFP+ tumor cells relative to the area covered by CD34+ blood vessels confirmed this significant reduction (Fig. 13e). This was not a consequence of changes in blood vessel density, as there were no differences across the experimental metastasis groups (data not shown). Moreover, Ctss deletion did not alter blood vessel density or permeability in the normal brain of non-tumor bearing animals (Fig. 13f, g). These results are suggestive of either a potential defect in seeding of single brain metastatic cells in the earliest stages, or a subsequent impairment in colonization, or perhaps insufficiencies in both processes.

To further investigate these possibilities, we assessed metastatic seeding in the experimental metastasis model across the four experimental groups. We examined the earliest steps of brain metastatic cell homing and survival29, specifically the first 48h. We found that 24h after CTSS KD tumor cell injection, there was a reduction in BLI signal in both WT and Ctss KO mice (Fig. 5a, b). The experimental group in which CTSS KD cells were injected into WT mice showed a BLI signal close to the control group after 48h, while there was a progressive decrease in BLI signal in the CTSS KD; Ctss KO group (Fig. 5a, b). Similarly, analysis of the proportion of viable tumor cells still within the blood vessel lumen (intravascular), in the process of extravasating, or fully extravascular, revealed significant differences in the CTSS KD; WT group at 24h, and in the CTSS KD; Ctss KO group at both 24h and 48h (Fig. 5c).

Given that there was an initial reduction in tumor cell extravasation in the CTSS KD; Ctss WT group (Fig. 5a, c), although the incidence of detectable brain metastasis was ultimately not affected (Fig. 3b), we assessed subsequent metastatic colony outgrowth. While there was an initial trend towards delayed growth in the CTSS KD; Ctss WT cohort, brain tumors ultimately grew to the same extent as the controls (Fig. 13h). In contrast, the kinetics of tumor growth in the CTSS KD; Ctss KO group did not recover over the same time course (Fig. 13h). These results suggest that tumor- and stromal-derived cathepsin S show some functional redundancy during seeding and outgrowth and that the impact of each cellular source is most likely regulated by differential expression levels at distinct stages. Additionally, tumor cell derived-CTSS may be important for the initial steps of blood-brain barrier (BBB) transmigration and extravasation into the brain, whereas stromal-supplied Ctss is subsequently involved in supporting tumor cell survival to successfully form brain micrometastases, and only their combined depletion impairs the entire cascade of metastatic seeding and outgrowth. Interestingly, a similar finding was recently reported in a colorectal carcinoma model, where depletion of both tumor and stromal sources of cathepsin S was also required to slow tumor growth³⁰.

### Cathepsin S promotes BBB transmigration via junctional protein cleavage

The BBB is a selective barrier between the systemic circulation and the brain, which is formed by specialized endothelial cells, pericytes and astrocytes31. While the BBB restricts the entry of most macromolecules, it is not an impenetrable barrier in transmigration of metastasizing cancer cells into the brain. We therefore examined the potential role of tumor cell-supplied CTSS in breaching the BBB, by using an in vitro BBB assay32. We performed either genetic or pharmacological depletion of CTSS in Br-M cells via shRNA-mediated knockdown, or a cathepsin S-specific inhibitor VBY-999 respectively, which does not affect viability of Br-M cells (Fig. 14a). Inhibition or knockdown of CTSS did not affect the ability of Br-M cells to cross a BBB formed by human umbilical endothelial vein cells (HUVECs) and astrocytes (Fig. 14b). By contrast, when human brain microvascular endothelial cells (HBMECs) were used instead of HUVECs, there was a significant reduction in Br-M cells crossing the BBB, and this was further impaired by 55-65% via genetic or pharmacological depletion of CTSS respectively (Fig. 5d). Cell layers that were formed by HBMECs without the addition of astrocytes also significantly decreased the transmigration capability of CTSS KD Br-M cells (Fig. 14b), whereas transmigration of Br-M cells across HUVECs or astrocytes alone was not altered by CTSS depletion (Fig. 14b).

Tight junctions and adherens junctions between adjacent cells are critical for maintaining BBB integrity, and are composed of different proteins including junctional adhesion molecules (JAMs), occludin, claudins and cadherins31, 33, 34. Therefore, we investigated whether any of these proteins represented potential CTSS substrates. We first performed biochemical cleavage assays using recombinant CTSS and recombinant proteins for each of the potential substrates, under similar conditions to those we previously reported for the identification of E-cadherin cleavage by CTSS6. CTSS efficiently cleaved the three JAM family members JAM-A, -B and -C at pH 4.5, the acidic pH of the lysosome, and maintained robust cleavage of JAM-B specifically at pH 6.0, the acidified pericellular pH measured in solid tumors35. Importantly, cathepsin S retains activity even at neutral pH36. JAM cleavage was inhibited by the cathepsin S-specific inhibitor VBY-999 in all cases (Fig. 6a). Occludin and Claudin (CLDN)-3 were also cleaved by CTSS, whereas CLDN5 and the adherens junction proteins CD31 and CDH5 (VE-cadherin) were unaffected by incubation with CTSS (Fig. 6a). We next examined the mRNA expression levels of each of these junctional components in both endothelial cell types (HBMECs and HUVECS) that were used for the in vitro BBB assay. Interestingly, we found that JAM-B levels were significantly higher in HBMECs compared to HUVECs. JAM-B was the only candidate substrate that displayed this differential expression; the other junctional proteins were generally expressed at higher levels in HUVECs (Fig. 6b). Staining of several junctional proteins revealed tissue-specific expression for Jam-B, which was detectable only in brain, and not bone or lung (Fig. 6c). Occludin and Cldn3 showed a somewhat broader expression pattern (Fig. 6c). The tissue-specific enrichment of genes encoding junctional proteins was confirmed by querying publicly available datasets37 (Fig. 14c).

As the effects of CTSS depletion or inhibition on Br-M transmigration were only observed when HBMECs were used in the BBB assay, and given the organ-specificity of Jam-B expression (Fig. 6c), we reasoned that JAM-B might be the most relevant substrate in this assay. We next aimed to identify the putative cleavage location for CTSS in JAM-B. We compared the fragment sizes of each cleavage product that was detectable with JAM-A, -B, or -C specific antibodies to fragments that contain the immunoglobulin (Ig)G1 domain, which is linked to recombinant JAM proteins (Fig. 14d). The molecular weight of the JAM cleavage products and pH dependence of JAM processing by CTSS suggests that all 3 family members share a similar but not fully conserved CTSS cleavage site that is localized close to the transmembrane domain. Alignment of the amino acid sequence of the JAM family members identified the sequence indicated in Figure 14e as the putative cleavage site for CTSS, which contains a sequence consistent with specificity preferences for CTSS that were previously identified in biochemical studies³⁸⁻⁴⁰. Cleavage in this region of the JAM proteins likely leads to shedding of the JAM extracellular domain, thereby disrupting cell-cell adhesion. We performed cell-based cleavage assays as illustrated in Figure 6d to test if tumor cell-secreted CTSS mediates shedding of JAM-B from the HBMEC cell surface. Indeed, incubation of HBMECs with tumor cell-conditioned media (CM) led to a CTSS-mediated accumulation of JAM-B in HBMEC CM after 2-4h. The effect was decreased by the addition of the cathepsin S-specific inhibitor VBY-999 (Fig. 6e, Fig. 14f). These results are consistent with the impairment of BBB transmigration in vitro and in vivo when CTSS is targeted, as CTSS-mediated shedding of the JAM-B extracellular domain would be expected to disrupt the integrity of tight junctions thereby allowing tumor cells to breach the BBB.

### Cathepsin S inhibition reduces experimental brain metastasis formation

Given our identification of cathepsin S as an important regulator of brain metastasis in experimental models and the negative association with patient survival, we examined whether its pharmacological inhibition is sufficient to reduce metastatic seeding and colonization in a preclinical prevention trial (Fig. 7a). Mice were treated with VBY-999 for 2 days to inhibit cathepsin S activity prior to tumor cell inoculation, and were then continuously treated with VBY-999 until the trial endpoint of 35 days post-tumor cell inoculation. Pharmacokinetic analysis showed that VBY-999 levels in the plasma were significantly above the required concentration for target inhibition at the time of tumor cell inoculation, and confirmed that VBY-999 efficiently crosses the BBB with stable concentrations in the brain throughout the duration of the trial (Fig. 7b). Interestingly, we found a significant 65-77% reduction in BLI signal at different time points during the trial, and at the trial endpoint of 35 days (Fig. 7c, d, P<0.05).

Initiation of VBY-999 treatment in fully established, end-stage brain metastases did not result in a significant difference in tumor burden (Fig. 13i, j), indicating that targeting this enzyme is most critical in seeding and early outgrowth. We also investigated the organ specificity of cathepsin S inhibition by assessing bone metastasis in a prevention trial setting, using two different approaches. First, as bone and spine metastases can develop in the brain metastasis model, we assessed whether there was an effect on these lesions following VBY-999 treatment. There was no significant difference between the treatment groups, which was further supported by the finding that genetic depletion of cathepsin S also had no effect on bone metastasis formation (Fig. 13k, l). Similarly, VBY-999 treatment of the bone metastasis model specifically in a prevention trial showed no change in BLI output or development of osteolytic metastases (Fig. 7e, f, Fig. 13m). These data are consistent with our finding that CTSS expression levels in patients only correlated with brain MFS, and not bone MFS. In sum, cathepsin S inhibition is efficient in substantially and specifically reducing brain metastasis if cathepsin S activity is blocked throughout the course of experimental brain metastasis.

### Mice

All animal studies were approved by the Institutional Animal Care and Use Committee of Memorial Sloan-Kettering Cancer Center. Athymic/nude mice were purchased from NCI Frederick or bred within the MSKCC animal facility. The cathepsin S knockout mouse line (*Ctss* KO) was generated as described previously²⁰ and backcrossed for 6 generations to the Athymic/nude background. NOD/SCID mice were purchased from Charles River Laboratories. MMTV-PyMT⁵³ immunocompetent transgenic mice (FVB/n) were bred within the MSKCC animal facility.

### Cell lines

Brain- (Br-M), bone- (Bo-M) and lung- (Lu-M) metastatic variants of the human breast cancer cell line MDA-MB-231 (denoted parental) were generated as previously described¹⁶⁻¹⁸ and labeled with the triple imaging vector (TK-GFP-Luc; TGL)²⁷ to allow for non-invasive *in vivo* imaging of tumor growth over time. The MDA-MB-231 variants were cultured in DMEM+10% FBS. Mouse Br-M variants were derived from the TS1 cell line⁵⁴ that was previously isolated from MMTV-PyMT mammary tumors. These are denoted PyMT-BrM cells.

Human Umbilical Vein Endothelial Cells (HUVEC) were purchased from the ATCC. Human Brain Microvascular Endothelial Cells (HBMEC) and Human Astrocytes (HA) were purchased from Sciencell. HUVEC and HBMEC were cultured on gelatin coated cell culture dishes, and HA on poly-L-lysine coated cell culture dishes in endothelial cell media (ECM, Sciencell) + 10% FBS supplemented with endothelial cell growth factors (ECGF).

### Generation of brain, bone and lung metastases

For brain and bone metastases in xenografted mice, 1x10⁴ brain-metastatic cells (Br-M) or 1x10⁵ bone-metastatic cells (Bo-M) were inoculated into the left cardiac ventricle of 6-8 week old female Athymic/nude mice. For lung metastasis generation, 1x10⁵ lung-metastatic cells (Lu-M) were injected into the lateral tail vein of 6-8 week old female NOD/SCID mice.

For brain metastasis generation in immunocompetent mice, 1 x 10⁵ PyMT-BrM cells were inoculated into the left cardiac ventricle of 6-8 week old female FVB/n mice. Early and late metastases were defined by their bioluminescence intensity (BLI) at the time of tissue harvest for samples used in the microarray analysis and the independent sample set used for validation. Brain metastases that had a BLI output between 4.3x106 to 4.2x107 photons / sec were classified as early-stage metastases and were collected between 3-4 weeks after tumor cell inoculation. Late-stage brain metastases had a BLI output between 1.6x108 to 6.4x108 photons / sec and were collected between 5-8 weeks after tumor cell inoculation. Histological and morphometric analyses of these different stages showed that early-stage brain metastases are comprised of clusters of ∼50-200 cells, and can be considered similar to 'micrometastases', and late-stage metastases consist of clusters of ∼5,000-15,000 cells, corresponding to 'macrometastases'. Representative images of the different stages are shown in Figure 8d. Early-stage bone metastases were defined by a BLI intensity that ranged between 6.3x106 to 1.1x108 photons / sec and were harvested 3 weeks after tumor cell inoculation. Late-stage bone metastases showed a minimal BLI intensity of 8x108 photons / sec and a maximal BLI intensity of 2.5x109 photons / sec and were harvested 5 weeks after tumor cell inoculation. Histological and morphometric analyses of bone metastases showed that early-stage lesions are comprised of clusters of ∼50-200 cells, and late-stage metastatic clusters consist of ∼2,000-10,000 cells. Representative images of the different stages are shown in Figure 8e. The generation of 'mismatched' samples (Br-M in bone or Bo-M in brain) followed the same criteria for early- and late-stage metastasis. Early-stage lung metastases were harvested 48h after tumor cell inoculation. The BLI intensity at this time point ranged between 2.1x10⁶ to 1.7x10⁷ photons/sec. Late-stage lung metastases were harvested 5 weeks after tumor cell inoculation with an average BLI intensity between 8.1x108 to 3x109 photons / sec. Histological and morphometric analyses of lung metastases showed that early-stage lesions are comprised of cells diffusely present throughout the lung (∼2,000-4,000 cells per sectional plane, per entire lung), and late-stage metastatic clusters consist of ∼1,000-5,000 cells. Representative images of the different stages are shown in **Figure 8f****.** Late-stage lung metastases from the spontaneous MMTV-PyMT breast-to-lung metastasis model were harvested from 14 week-old female PyMT mice.

For tissue isolation, mice were lethally anesthetized with 10 mg/ml ketamine/1 mg/ml xylazine and retro-orbitally injected with 15 mg/ml luciferin. Mice were then intracardially perfused with PBS. Tumor-burdened tissue was identified by the presence of BLI signal for brain and bone metastases. For lung metastases, part of the left lung lobe was collected. Snap frozen samples were collected for RNA and protein isolation and tissues were fixed in 4% paraformaldehyde (PFA) for histology.

### Microarray analysis

For microarray analysis, all samples were prepared and processed by the Genomics Core Facility at MSKCC. RNA was isolated using Trizol (Invitrogen) and the quality was assessed by running on an Agilent Bioanalyzer. Total RNA was reverse transcribed and labeled using the Genechip 3' IVT Express Kit (Affymetrix). The resulting cRNA was hybridized to HuMu Prot/In chips (Affymetrix). All bioinformatics analyses were completed in R using the Bioconductor suite of packages. The 'affy' package was used for robust multi-array average normalization followed by quantile normalization. Mouse and human samples and probes were normalized separately. With the exception of the cross-species scatterplots, all subsequent bioinformatics analyses regarded the tumor and stroma separately.

The 'limma' package was used to identify differentially expressed genes across tissue and metastatic stage for both tumor and stroma. Differential expression was considered significant at a fold change of □ 2 with a false discovery rate of 10%. Tissue-specific genes were identified by the intersection of pairwise comparisons: e.g. lung stroma-specific genes were identified by the intersection of genes significantly enriched in lung vs. bone and genes significantly enriched in lung vs. brain. Stage-specific genes were identified in a tissue-specific manner comparing early- and late-stage metastases. The microarray data is deposited at NCBI GEO under the accession number GSE47930.

Principal component analysis (PCA) was completed using the covariance matrix in the 'princomp' package in R. The first two components are plotted in Figure 1 b. Homologs for mouse and human genes were identified using the HomoloGene Database through the NCBI (url www.ncbi.nlm.nih.gov/homologene). Homolog pairs were plotted with mouse/stroma tissue-specific, early vs. late, fold change on the x axis, and human/tumor tissue-specific, early vs. late, fold change on the y axis.

### External Datasets and Survival Analysis

For gene expression analysis of mouse endothelial cells, raw data from GSE47067 37 was imported into R and normalized as above. For patient analysis, normalized gene expression data was downloaded from the GEO (GSE12276). Each gene was mean centered and scaled by standard deviation. Patients were split into tertiles (lower 33%, middle 33%, upper 33%) of CTSS gene expression for Kaplan-Meier survival analysis. The scaled, continuous CTSS gene expression was used for Hazard Ratio (HR) calculation. Similar analyses were completed for genes in Figure 11c-e. Survival analysis was completed using the 'survival' package in R. Hazard ratios were determined utilizing the 'coxph' function from the 'survival' package. Nominal P values are reported for HR significance in Table 2 with a significance cutoff of 0.05 used to identify genes significantly associated with metastasis-free survival. P values were generated using the log-rank statistic for Kaplan-Meier analysis and Wald's test for the Hazard Ratio analysis.

### Clinical samples

The specimen of primary breast tumors and brain metastases used in this study were obtained at MSKCC, Massachusetts General Hospital (MGH), Brigham and Women's Hospital (BWH) and Dana Farber Cancer Institute according to protocols approved by the human subjects institutional review boards of MSKCC, DFCI, MGH, and BWH. Information about the clinical samples can be found in Table 3.

### Generation of CTSS knock-down lines

Five shRNA sequences targeting *CTSS* were obtained from the RNAi Codex and RNAi Consortium. shRNA sequences were inserted into the targeting hairpin sequence for the pRetroSuper vector. Correct insertion into the vector was verified by digestion and sequencing of the vector. Plasmids with the correct shRNA targeting sequence were transfected into H29 viral packaging cells. Viral particles were concentrated from the H29 cell supernatant, added to the target cells in the presence of polybrene and cells were selected with puromycin. One of the four shRNAs (*CTSS* shRNA: GATAAAGTTTGCTAAGTAA - TTACTTAGCAAACTTTATC) was used for subsequent experiments to target *CTSS* with 90% KD efficiency. A non-targeting shRNA [CGCCATAAATATAACTTTA - TAAAGTTATATTTATGGCG] was used as control.

### Targeting tumor- and stroma-derived CTSS in vivo

1x10⁴ Br-M cells (Br-M *CTSS* KD or Br-M Ctrl) were inoculated into the left ventricle of 6-8 week old female Athymic/nude or *Ctss* KO Athymic/nude mice. Metastases formation was monitored once per week by bioluminescence imaging using a Xenogen IVIS-200 Optical In Vivo Imaging System to determine metastasis incidence in the four experimental groups shown in the table in **Figure 3b****.** In addition, numberical values of the increase in BLI intensity present the kinetics of tumor progression **(****Figure 13h****).** An independent cohort of mice was injected with Br-M cells as described above and sacrificed at day 35 after tumor cell inoculation for subsequent analysis of proliferation, apoptosis, angiogenesis, and metastatic outgrowth.

For *in vivo* extravasation experiments, Athymic/nude or *Ctss* KO Athymic/nude mice were inoculated with 5x10⁵ Br-M Ctrl or Br-M *CTSS* KD cells. BLI intensity was monitored 0h, 24h and 48h after tumor cell inoculation and the BLI intensity was plotted relative to the BLI intensity immediately after tumor cell inoculation (0h time point).

### Identification of cathepsin S inhibitor VBY-999

VBY-999 was provided by Virobay Inc., Menlo Park, CA and is part of an extensive structure-based drug discovery program. VBY-999 is a covalent reversible inhibitor with an electrophilic nitrile warhead. The detailed chemical synthesis and structure of compounds in the structural series including VBY-999 can be found in issued US Patent 7,547,701. Recombinant purified human and mouse cathepsin S were used to assess potency of VBY-999 and determine inhibition constants. Activity on the peptide substrate Z-Leu-Arg-AMC was determined in vitro by measuring hydrolysis of the substrate with spectrofluorimetric quantitation of AMC. The VBY-999 inhibitor was preincubated with cathepsin S for 15 min at room temperature (25 °C) after which the substrate was added to initiate the 30 min reaction. Assay incubation buffer included 25 mM CH3COONa, pH 4.5, 2.5 mM DTT, and 0.05 M NaCl. Appropriate reaction conditions and peptide substrates for other cysteine and serine proteases were utilized to screen for selectivity of VBY-999 for cathepsin S. VBY-999 has an inhibition constant Ki(app) = 290 pM on the purified human cathepsin S enzyme, and > 3000-fold selectivity versus the related cathepsins K, L, B, and F. Potency on the closely related cathepsins K, L, and F was Ki(app) > 3 µM, with potency on cathepsin B Ki(app) = 700 nM. Potency on mouse cathepsin S enzyme was verified on mouse cathepsin S purified enzyme. VBY-999 has an inhibition constant Ki(app) = 690 pM on mouse cathepsin S. No measurable inhibition was detected for any other cysteine, serine or aspartyl proteases tested.

### VBY-999 Inhibitor Preclinical Trial

For administration to mice, the VBY-999 inhibitor was formulated in a nanoparticle-based suspension formulation and further diluted in 5% dextrose in water (D5W) at a concentration of 10 mg/ml. Subcutaneous dosing of VBY-999 provided a dosing formulation and route that allows high and sustained plasma concentrations of the drug to be achieved, which was confirmed using a bioanalytical LC-MS/MS method after 2 and 7 days of treatment (Fig. 7b). This results in full inhibition of the enzyme target for the duration of the trial, following once-daily dosing. In order to determine if VBY-999 had sufficient penetration of the CNS to be available for cathepsin S inhibition in the brain and at the blood-brain barrier site, and to confirm that concentrations in the brain remain stable throughout the duration of the trial, VBY-999 concentration was determined at day 2, day 7, and day 37 after treatment start by LC-MS/MS (Fig. 7b). These data indicate that VBY-999 levels in the plasma were significantly above the required concentration for target inhibition at the time of tumor cell inoculation and that VBY-999 levels in the brain remain stable throughout the 37-day treatment schedule at a level sufficiently greater than the enzyme inhibition constant, and are thus expected to effectively inhibit cathepsin S activity. For the prevention trials, mice were randomly assigned into vehicle and VBY-999 treatment groups and treatment was commenced two days before tumor cell inoculation (d= -2). Mice were dosed with 100 mg/kg VBY-999 or vehicle (D5W) by subcutaneous injection once daily. At day 0, Athymic/nude mice were inoculated with 1x104 Br-M Ctrl cells or 1x105 Bo-M Ctrl cells. Metastases formation was monitored every fifth day by bioluminescence imaging using a Xenogen IVIS-200 Optical In Vivo Imaging System during the trial period from day 0 to day 35 after tumor cell inoculation. For the Bo-M trial, mice were subjected to X-ray analysis at d35 after tumor cell inoculation using a SPECT-CT scanner (X-SPECT). For the regression trial, mice were stratified into vehicle and VBY-999 treatment groups at d27 after tumor cell inoculation to achieve equal average BLI intensity at the time of treatment start at d28. Mice were dosed daily with either vehicle or VBY-999 (100 mg/kg) for 7 days and metastasis growth was monitored by BLI imaging at d32 and d35.

### RNA isolation, cDNA synthesis and quantitative real-time PCR

RNA was isolated with Trizol, DNase treated, and 0.2 µg of RNA was used for cDNA synthesis. Details about the Taqman assays can be found in Table 4. All species-specific Taqman assays were chosen based on their location in the mRNA sequence that allows for maximal discrimination between mouse and human transcripts. For each Taqman assay, species specificity was tested by qPCR using mouse or human samples as controls.

### Collection of conditioned media, protein isolation and western blotting.

Conditioned media (CM) from Br-M cell lines was generated by incubating confluent cell layers in serum-free DMEM media for 24 hours. Collected (CM) was passed through 0.22 µm filters to remove cellular debris. For western blotting, CM was concentrated by centrifugation in Centrifugal Filter Units (Millipore). For protein isolation from cells in monolayer, cells were harvested by scraping and lysed in RIPA lysis buffer (Pierce) with 1x complete Mini protease inhibitor cocktail (Roche). For protein isolation from tissue, snap frozen tissue was homogenized in RIPA lysis buffer (Pierce) with 1x complete Mini protease inhibitor cocktail (Roche) followed by Dounce homogenization. Protein was quantified using the BCA assay (Pierce). Protein lysates were loaded onto SDS-PAGE gels and transferred to PVDF membranes for immunoblotting. Membranes were probed with antibodies as indicated in Table 5 and detected using the appropriate HRP-conjugated secondary antibodies using chemoluminescence detection (Millipore). Bands from western blots were quantified in the dynamic range using the Gel analysis module in ImageJ software.

### Generation of Serpina3n Antibody

Peptides targeting murine Serpina3n were determined via alignment of the protein sequence for serpina3n against mouse Serpin a3 family members, Serpina3b, c, f, g, k, and m, as well as human SERPINA3. From this alignment, divergent regions were located and peptides were chosen that corresponded to regions 373-396 (a3n-no1), 225-248 (a3n-no2), and 398-418 (a3n-no3) of Serpina3n. 10-14mg of each peptide was synthesized by the Pocono Rabbit Farm and Laboratory, with 2mg of each peptide conjugated to KLH and 2mg of each peptide conjugated to BSA. The KLH-conjugated peptides were used to generate an immune response in Armenian hamsters and BALB/c mice by the Monoclonal Antibody Core Facility at MSKCC. Serum from hamsters and mice was tested via ELISA using the BSA-conjugated peptides in Nunc Maxisorp ELISA plates (protocol provided by the MAb core). The best responding hamster to all three peptides was used for fusion, and positive colonies were screened by ELISA and for response to each peptide. Ten positive colonies were saved for each peptide. Clones were also screened by immunohistochemistry and immunofluorescence for ability to recognize murine Serpina3n in mouse tissue. One colony (13H5, which responded to peptide a3n-no1) was selected for subcloning.

### Immunocytochemistry

For immunocytochemistry, cells were cultured on glass coverslips and fixed in 4% paraformaldehyde in 0.1M phosphate buffer for 20 min at room temperature. Cells were permeabilized in PBS with 0.25% Triton X-100 for 10 min. Cells were blocked in 0.5% PNB (phosphate-NaCl) in PBS for at least 1 hour at room temperature, followed by incubation in goat anti-human CTSS primary antibody diluted 1:100 in 0.25% PNB overnight at 4°C. Cells were then washed in PBS and incubated with the donkey anti-goat Alexa568 secondary antibody (Molecular Probes) at a dilution 1:500 in 0.25% PNB for 1 hour at room temperature. After washing in PBS, cells were counterstained with DAPI (5 mg/ml stock diluted 1:5,000 in PBS) for 5 minutes prior to mounting with ProLong Gold Antifade mounting media (Invitrogen).

Paraffin-embedded sections were processed using a Ventana automated staining device. The automated deparaffinization/ rehydration, citrate buffer-based antigen retrieval, and blocking of unspecific protein binding and endogenous peroxidase was followed by incubation with mouse anti-human CD68 (Dako) primary antibody and goat anti-human CTSS (R&D Systems) or mouse anti-human CK (Dako) and goat anti-human CTSS (R&D Systems) overnight at 4°C. Sections were then washed in PBS and incubated with donkey-anti mouse HRP labeled secondary antibody (Jackson Immunoresearch, 1:200) in 0.25% PNB buffer in PBS for 1.5h followed by incubation with Alexa488 labeled tyramide (Invitrogen) at a 1:200 dilution in amplification buffer for 8 min. Sections were then washed in PBS and incubated with donkey anti-goat Alexa568 (Molecular Probes) at a dilution of 1:500 in 0.25% PNB for 1h at room temperature. Frozen sections that were used for Jam-B, Cldn3 and Ocln staining were processed using a Ventana automated staining device. The automated rehydration, citrate buffer-based antigen retrieval, and blocking of unspecific protein binding and endogenous peroxidase was followed by incubation with rat anti-mouse Jam-B (Pierce) primary antibody and goat anti-mouse Cd31 (R&D Systems), rabbit anti-mouse Cldn3 (Invitrogen) and goat anti-mouse Cd31 (R&D Systems), or rabbit anti-mouse Ocln (Invitrogen) and goat anti-mouse Cd31 (R&D Systems) overnight at 4°C. Sections were then washed in PBS and incubated with donkey anti-rat or donkey anti-rabbit biotin labeled secondary antibody (Vector, 1:200) in PBS + 0.03% Tween for 1.5h followed by incubation with Streptavidin-Cy5 (Invitrogen, 1:200) PBS + 0.03% Tween for 20 min. Sections were then washed in PBS and incubated with donkey anti-goat Alexa568 (Molecular Probes) at a dilution of 1:500 in 0.25% PNB for 1h at room temperature. After washing in PBS, tissue sections were counterstained with DAPI (5 mg/ml stock diluted 1:5,000 in PBS) for 5 min prior to mounting with ProLong Gold Antifade mounting media (Invitrogen). Apoptotic cells were stained via terminal dUTP nick end labeling (TUNEL) following the manufacturer's instructions (Trevigen), with the modification of using Streptavidin-Cy5 (Invitrogen; 1:200) instead of Streptavidin-FITC.

Tissue sections and cells on coverslips were visualized under a Carl Zeiss Axioimager Z1 microscope equipped with an ApoTome.2 and a TissueGnostics stage to allow for automated image acquisition. The analysis of proliferation and apoptosis were performed using TissueQuest analysis software (TissueGnostics) as previously described6, 55. All parameters of metastatic outgrowth and angiogenesis were quantitated using MetaMorph software (Molecular Devices). Briefly, vasculature was visualized by Texas Red Lectin (Vector Laboratories) injections or by staining of the endothelial cell marker CD34. Tumor cells were detected by their expression of the GFP reporter. The area covered by CD34 and GFP staining was quantified. To determine the number of tumor cells that are present within an area of 1 - >4 average tumor cell diameter, the blood vessel area was dilated by 1 - 4 average tumor cell diameter with an increment of 1 tumor cell diameter and the number of tumor cells in each area was determined. Tumor cells that were localized outside an area of 4 average tumor cell diameter were defined as >4 tumor cell diameter away from CD34+ blood vessels as illustrated in Figure 4b. Vessel density was quantified as the area covered by Texas Red Lectin relative to the area covered by DAPI.

To histologically quantify the percentage of intravascular, extravasating or extravasated tumor cells (Fig. 5c), brain sections were stained for TUNEL+ cells to exclude non-viable tumor cells from the analysis. Brain sections were automatically acquired using TissueQuest software (TissueGnostics), which used a z-stack (5 images above and below the focal plane, 0.3 µm steps, 20x objective) to generate a maximal intensity projection (MIP) image of each acquired brain area. Tumor cells were detected via cell tracker green (Invitrogen) and vasculature was visualized by Texas Red Lectin (Vector Laboratories) injections. Tumor cells were counted manually and their localization relative to the vasculature was determined.

For analysis of human samples, 5-10 fields of view were acquired using a 20x objective (total magnification 200x) and a Zeiss Apotome to ensure cells were in the same optical section. The number of CK+ tumor cells and CD68+ macrophages, and their relative CTSS intensities (CTSS index) was evaluated using CellProfiler 2.0 software. A CellProfiler module was generated that allowed for the detection of tumor cells and macrophages based on their DAPI and CK signal, or DAPI and CD68 signal, respectively. The CTSS signal intensity was measured in the whole cell population (DAPI+) and associated with a specific cell type (macrophages or tumor cells), and the proportion of CTSS signal associated with CK+ tumor cells or CD68+ macrophages was calculated relative to the overall CTSS signal intensity in all DAPI+ cells.

### Measurement of vessel permeability

6-8 week old Athymic/nude mice were injected with Evan's blue dye (30 mg/kg) into the tail vein. 30 mins after injection, mice were anesthetized and perfused with acidified fixative (1% PFA in 0.05 mM citrate buffer, pH 3.5). 30 mg of brain tissue was incubated in 500 µl formamide (Sigma) to extract Evan's blue at 60□C overnight. Absorbance was measured at 610 nm and 740 nm on a spectraMax 340pc plate reader (Molecular Devices).

### In vitro blood-brain barrier transmigration assays

In vitro blood-brain barrier (BBB) transmigration assays were performed as previously described32. The artificial BBB was formed with either HUVECs or HBMECs (20,000 cells / well) in co-culture with HA cells (100,000 cells / well) for 3 days on Transwell-inserts with 3 µm fluoroblock membranes. Cell-tracker green (CMFDA)-labeled Br-M Ctrl or Br-M CTSS KD cells (20,000 cells / well) were allowed to transmigrate for 18h through the artificial BBB towards a FBS gradient, in the presence or absence of VBY-999 (10 µM). Tumor cell transmigration through empty inserts (coated with gelatin and poly-L-lysine) or inserts coated with HUVECs, HBMECs or HAs alone were used to determine the baseline migratory potential and the contribution of the single cell types to BBB formation. Tumor cell transmigration was stopped through fixation of the cells in 4% PFA. Cells were counterstained with Hoechst dye (5 mg/ml stock diluted 1:5,000 in PBS) for 5 min prior to mounting with ProLong Gold Antifade mounting media (Invitrogen). The number of transmigrated tumor cells was quantified by analyzing 200 fields of views (FOVs) that were acquired with a 20x objective (200x total magnification) using TissueQuest analysis software (TissueGnostics).

### In vitro and cell-based cleavage assays

Recombinant inactive CTSS was obtained from R&D Systems. CTSS was activated at 50 ng/µl in 50 mM sodium acetate, 5 mM DTT, 0.25 M NaCl (pH 4.5) for 1.5h at 37°C. For the in vitro cleavage reaction, activated CTSS was incubated with recombinant proteins in the presence or absence of the cathepsin S inhibitor VBY-999 (10 µM) for 0, 10 or 20 min in 50 mM sodium acetate, 5 mM DTT, 0.25 M NaCl at pH 4.5 and pH 6.0. Details about the recombinant proteins used in the in vitro cleavage assay can be found in Table 6. The in vitro cleavage reaction was stopped by adding SDS sample buffer and reducing agent (Invitrogen) to each reaction and the samples were boiled at 95°C for 5 min. Aliquots were subjected to western blot analysis as described above. Information about the antibodies can be found in Table 5. All experiments were repeated independently at least three times.

For cell-based cleavage assays, HBMECs were grown to 100% confluence in a 10 cm plate. Conditioned media from Br-M cells was collected as described above. 200 µl of concentrated Br-M CM (collected from two 10 cm plates of confluent Br-M cells) was diluted in 6.5 ml PBS pH 6.0 + 0.05 mM DTT for each 10 cm plate of HBMECs. The cleavage reaction was performed in the presence or absence of the cathepsin S inhibitor VBY-999 (10 µM) for 0h, 2h, and 4h. PBS pH 6.0 + 0.05 mM DTT was used as a control. The supernatant from the HBMEC cell layers was collected after the indicated time points, concentrated and subjected to western blot analysis as described above.

### Proliferation Assays

Cell growth rate was determined using an MTT cell proliferation kit (Roche). Briefly, cells were plated in triplicate in 96-well plates (2.5 x 103 for Br-M Ctrl and Br-M CTSS KD cells) in the presence or absence of 0.1 - 100µm VBY-999. Reduction of the MTT substrate was detected by colorimetric analysis using a plate reader as per the manufacturer's recommended protocol. 10 µl of MTT labeling reagent was added to each well and then incubated for 4h at 37°C, followed by the addition of 100 µl MTT solubilization reagent overnight. The mixture was gently resuspended and absorbance was measured at 595 nm and 750 nm on a spectraMax 340pc plate reader (Molecular Devices).

### Data presentation and statistical analysis

Data are presented as means with standard error (s.e.m.) or as statistical scatter plots using GraphPad Prism Pro5. Numeric data were analyzed using unpaired two-tailed Student's t-test unless otherwise noted. Kaplan-Meier survival curves, heatmaps and scatterplots were generated in R v 2.15.2 using the base R graphics, 'gplots' or 'ggplot2' packages. P values were generated using the Log-Rank statistic for Kaplan-Meier Analysis and Wald's test for the Hazard Ratio. P < 0.05 was considered as statistically significant. All code used to analyze the data and generate the plots is available at the following url: bitbucket.org/bowmanr/joycelab-humu-brain-met-ctss.

While several aspects of the present invention have been described and depicted herein, alternative aspects may be effected by those skilled in the art to accomplish the same objectives. Those skilled in the art will recognize, or be able to ascertain using no more than routine experimentation, many equivalents to the specific embodiments of the invention described herein.

In Table 1 below, gene symbol, gene name, P value and fold change of expression differences in the experimental metastasis models are indicated in each column. Fold change is depicted such that a negative (-) value is associated with downregulation in late-stage metastases, while a positive value is associated with upregulation in late-stage metastases. For Table 1a, positive values are associated with upregulation in early-stage metastases compared to normal lung, while negative values are associated with downregulation in early-stage lung metastases compared to normal lung. To identify subtle changes in gene expression between normal lung and early stage lung metastases, a fold change cutoff of + 1.5 was used in conjunction with a nominal P-value cut off of 0.05. For every other analysis, a 2.0 fold change cutoff was used. P values were calculated as described in the methods using a two-tailed Students t-test.

In Table 2 below, (a-c) Hazard ratio, 95% confidence interval (CI), and P values for genes identified from analysis of patient dataset GSE12276, which are also listed in Fig. 11. The "metastatic site association" column denotes whether a gene shows association with patient MFS in multiple tissues, as summarized in Figure 11f. Tumor-derived differentially expressed genes (DEG) whose expression changes by stage in the experimental model (Fig. 1c, Table 1) were assessed for MFS using a cox proportional hazards model, as described in the Online Methods. Hazard ratios with 95% Cl's that do not cross 1.0 are considered significant. Nominal P values were determined using Wald's test. (d) Summary of the number of DEGs associated with MFS at each organ site. These tests aimed to address whether selecting genes for differential expression at the metastatic site enriched for genes associated with site-specific MFS. These tests demonstrate that the set of genes differentially expressed in the bone is enriched for genes associated with bone MFS, while the brain and lung DEGs are not significantly enriched for genes associated with brain or lung MFS. (e) Summary of differentially expressed genes in the bone, and their association with brain and lung MFS. We sought to determine if the significant enrichment of genes associated with bone MFS in the bone DEG set was specific to the bone, or whether there was also an association with brain and lung MFS. These hypergeometric tests demonstrate that the set of genes differentially expressed in the bone is enriched for genes associated with bone MFS only, and not genes associated with brain or lung MFS. Differences in the numbers between Table 1 and here are due to incomplete overlap between coverage of genes on the HuMu Protln array, and genes in the GSE12276 patient dataset as indicated. P values were generated using a hypergeometric test.

In Table 3 below, for patients 1-6 matched pairs of primary breast cancer and brain metastasis samples were available. For patients 7-13 only brain metastasis samples were available. MFS: metastasis-free survival. ER: estrogen receptor, PR: progesterone receptor, HER2: human epidermal growth factor receptor 2, Pos: positive, Neg: negative, N/A: not assessed, MFS: metastasis-free survival.

**Table 1: Tumor- and stroma-derived changes in gene expression between early and late stages of brain, bone and lung metastases**

| **a. Stroma-derived genes that show metastasis-associated expression in the lung** | | | |
|---|---|---|---|
| **symbol** | Name | *P* Value | Fold Change Early vs Normal |
| ***Serpina3g*** | serpin peptidase inhibitor, clade A (alpha-1 antiproteinase, antitrypsin), member 3g | 1.63E-05 | -2.53 |
| ***Serpina3n*** | serpin peptidase inhibitor, clade A (alpha-1 antiproteinase, antitrypsin), member 3n | 7.86E-04 | 2.36 |
| ***Timp1*** | tissue inhibitor of metalloproteinase 1 | 2.12E-02 | 1.66 |

| **b. Tumor-derived genes that show stage-specific expression in brain metastasis** | | | |
|---|---|---|---|
| **Symbol** | Name | *P* Value | Fold Cange Late vs Early |
| ***PSMB1*** | proteasome (prosome, macropain) subunit, beta type, 1 | 3.40E-14 | 7.22 |
| ***SEC11A*** | SEC11 homolog A (S. cerevisiae) | 6.01E-14 | 4.31 |
| ***TIMP1*** | TIMP metallopeptidase inhibitor 1 | 3.04E-13 | 6.37 |
| ***PSMC2*** | proteasome (prosome, macropain) 26S subunit, ATPase, 2 | 3.77E-13 | 5.56 |
| ***PSMA3*** | proteasome (prosome, macropain) subunit, alpha type, 3 | 4.43E-13 | 4.75 |
| ***CTSL1*** | cathepsin L1 | 7.24E-13 | 7.39 |
| ***TMED2*** | transmembrane emp24 domain trafficking protein 2 | 9.75E-13 | 5.85 |
| ***ANXA9*** | annexin A9 | 1.17E-12 | -2.59 |
| ***EIF3F*** | eukaryotic translation initiation factor 3, subunit F | 1.21E-12 | 4.22 |
| ***PSMD6*** | proteasome (prosome, macropain) 26S subunit, non-ATPase, 6 | 1.74E-12 | 3.48 |
| ***RPL4*** | ribosomal protein L4 | 1.81E-12 | 6.63 |
| ***MME*** | membrane metallo-endopeptidase | 2.39E-12 | -2.49 |
| ***PSMA6*** | proteasome (prosome, macropain) subunit, alpha type, 6 | 2.75E-12 | 3.36 |
| ***DAD1*** | defender against cell death 1 | 3.10E-12 | 3.67 |
| ***GDl2*** | GDP dissociation inhibitor 2 | 3.90E-12 | 3.93 |
| ***PSMA1*** | proteasome (prosome, macropain) subunit, alpha type, 1 | 3.95E-12 | 4.16 |
| ***SRSF9*** | serine/arginine-rich splicing factor 9 | 4.36E-12 | 2.92 |
| ***PSMB6*** | proteasome (prosome, macropain) subunit, beta type, 6 | 8.37E-12 | 4.77 |
| ***MMP24*** | matrix metallopeptidase 24 (membrane-inserted) | 9.43E-12 | -2.42 |
| ***RPL14*** | ribosomal protein L14 | 9.47E-12 | 8.58 |
| ***KLK2*** | kallikrein-related peptidase 2 | 1.01E-11 | -2.37 |
| ***APP*** | amyloid beta (A4) precursor protein | 1.07E-11 | 3.43 |
| ***CELA3B*** | chymotrypsin-like elastase family, member 3B | 1.08E-11 | -2.52 |
| ***ADAM11*** | ADAM metallopeptidase domain 11 | 1.15E-11 | -3.05 |
| ***PSMC1*** | proteasome (prosome, macropain) 26S subunit, ATPase, 1 | 1.17E-11 | 3.39 |
| ***PSMB5*** | proteasome (prosome, macropain) subunit, beta type, 5 | 1.28E-11 | 4.15 |
| ***RPL 18*** | ribosomal protein L18 | 1.43E-11 | 5.79 |
| ***FETUB*** | fetuin B | 1.62E-11 | -2.54 |
| ***LY6H*** | lymphocyte antigen 6 complex, locus H | 1.87E-11 | -2.54 |
| ***KLK6*** | kallikrein-related peptidase 6 | 2.07E-11 | -2.54 |
| ***CTSC*** | cathepsin C | 2.07E-11 | 3.86 |
| ***ANXA5*** | annexin A5 | 2.28E-11 | 6.33 |
| ***CAV1*** | caveolin 1, caveolae protein, 22kDa | 2.60E-11 | 5.94 |
| ***P13*** | peptidase inhibitor 3, skin-derived | 2.70E-11 | -2.25 |
| ***NARS*** | asparaginyl-tRNA synthetase | 2.70E-11 | 4.49 |
| ***AZIN1*** | antizyme inhibitor 1 | 2.79E-11 | 2.55 |
| ***PSMB4*** | proteasome (prosome, macropain) subunit, beta type, 4 | 2.97E-11 | 6.03 |
| ***EEF2*** | eukaryotic translation elongation factor 2 | 3.28E-11 | 3.32 |
| ***SPINT1*** | serine peptidase inhibitor, Kunitz type 1 | 4.32E-11 | -2.58 |
| ***HPX*** | hemopexin | 4.50E-11 | -2.76 |
| ***PSMB2*** | proteasome (prosome, macropain) subunit, beta type, 2 | 4.98E-11 | 3.99 |
| ***USP22*** | ubiquitin specific peptidase 22 | 5.05E-11 | 5.02 |
| ***PSMC5*** | proteasome (prosome, macropain) 26S subunit, ATPase, 5 | 5.19E-11 | 5.14 |
| ***PSMA4*** | proteasome (prosome, macropain) subunit, alpha type, 4 | 5.68E-11 | 4.50 |
| ***METAP2*** | methionyl aminopeptidase 2 | 6.79E-11 | 2.70 |
| ***RPL19*** | ribosomal protein L19 | 6.81E-11 | 4.02 |
| ***PSMD13*** | proteasome (prosome, macropain) 26S subunit, non-ATPase, 13 | 7.32E-11 | 3.15 |
| ***MMP25*** | matrix metallopeptidase 25 | 7.63E-11 | -2.49 |
| ***PRSS8*** | protease, serine, 8 | 7.99E-11 | -2.37 |
| ***PARK7*** | parkinson protein 7 | 8.11E-11 | 5.29 |
| ***ADAMTS13*** | ADAM metallopeptidase with thrombospondin type 1 motif, 13 | 8.35E-11 | -2.20 |
| ***JTB*** | jumping translocation breakpoint | 8.76E-11 | 3.57 |
| ***PSMD2*** | proteasome (prosome, macropain) 26S subunit, non-ATPase, 2 | 8.88E-11 | 5.31 |
| ***COPS3*** | COP9 constitutive photomorphogenic homolog subunit 3 (Arabidopsis) | 9.55E-11 | 2.55 |
| ***NAPSA*** | napsin A aspartic peptidase | 9.70E-11 | -2.48 |
| ***SNRNP200*** | small nuclear ribonucleoprotein 200kDa (U5) | 9.79E-11 | 3.79 |
| ***SERPINA4*** | serpin peptidase inhibitor, clade A (alpha-1 antiproteinase, antitrypsin), member 4 | 9.85E-11 | -2.37 |
| ***PRSS22*** | protease, serine, 22 | 1.09E-10 | -2.29 |
| ***ILF2*** | interleukin enhancer binding factor 2, 45kDa | 1.18E-10 | 2.66 |
| ***PSMB7*** | proteasome (prosome, macropain) subunit, beta type, 7 | 1.18E-10 | 3.06 |
| ***RPL10A*** | ribosomal protein L10a | 1.21E-10 | 6.62 |
| ***PSMB3*** | proteasome (prosome, macropain) subunit, beta type, 3 | 1.25E-10 | 3.35 |
| ***C6*** | complement component 6 | 1.28E-10 | -2.43 |
| ***MMP15*** | matrix metallopeptidase 15 (membrane-inserted) | 1.32E-10 | -2.52 |
| ***ADAM9*** | ADAM metallopeptidase domain 9 | 1.32E-10 | 3.04 |
| ***ANXA1*** | annexin A1 | 1.39E-10 | 12.3 |
| ***CTSS*** | cathepsin S | 1.57E-10 | -2.49 |
| ***F10*** | coagulation factor X | 1.60E-10 | -2.08 |
| ***HPN*** | hepsin | 1.76E-10 | -2.23 |
| ***SERPINF2*** | serpin peptidase inhibitor, clade F (alpha-2 antiplasmin, pigment epithelium derived factor), member 2 | 1.98E-10 | -2.27 |
| ***SPINK2*** | serine peptidase inhibitor, Kazal type 2 | 2.07E-10 | -2.14 |
| | (acrosin-trypsin inhibitor) | | |
| ***FSTL1*** | follistatin-like 1 | 2.14E-10 | 3.85 |
| ***ADAM6*** | ADAM metallopeptidase domain 6, pseudogene | 2.16E-10 | -2.56 |
| ***KLK14*** | kallikrein-related peptidase 14 | 2.20E-10 | -2.11 |
| ***PCSK4*** | proprotein convertase subtilisin/kexin type 4 | 2.28E-10 | -2.01 |
| ***GZMM*** | granzyme M (lymphocyte met-ase 1) | 2.42E-10 | -2.42 |
| ***RPL27*** | ribosomal protein L27 | 2.65E-10 | 4.34 |
| ***CST8*** | cystatin 8 (cystatin-related epididymal specific) | 2.75E-10 | -2.32 |
| ***MASP1*** | mannan-binding lectin serine peptidase 1 (C4/C2 activating component of Ra-reactive factor) | 2.90E-10 | -2.36 |
| ***PSME1*** | proteasome (prosome, macropain) activator subunit 1 (PA28 alpha) | 2.96E-10 | 3.05 |
| ***ADAMTS7*** | ADAM metallopeptidase with thrombospondin type 1 motif, 7 | 3.04E-10 | -2.47 |
| ***KAT7*** | K(lysine) acetyltransferase 7 | 3.08E-10 | -2.52 |
| ***KRTAP4-7*** | keratin associated protein 4-7 | 3.11E-10 | -2.56 |
| ***RHOA*** | ras homolog family member A | 3.20E-10 | 5.01 |
| ***ATP6V0E1*** | ATPase, H+ transporting, lysosomal 9kDa, V0 subunit e1 | 3.24E-10 | 3.88 |
| ***TMPRSS5*** | transmembrane protease, serine 5 | 3.30E-10 | -2.25 |
| ***TPSG1*** | tryptase gamma 1 | 3.32E-10 | -2.28 |
| ***FBLN1*** | fibulin 1 | 3.46E-10 | -2.58 |
| ***EIF3M*** | eukaryotic translation initiation factor 3, subunit M | 3.59E-10 | 2.83 |
| ***CST7*** | cystatin F (leukocystatin) | 4.25E-10 | -2.23 |
| ***TMPRSS7*** | transmembrane protease, serine 7 | 4.32E-10 | -2.16 |
| ***ST14*** | suppression of tumorigenicity 14 (colon carcinoma) | 4.53E-10 | -2.06 |
| ***RPS6*** | ribosomal protein S6 | 4.55E-10 | 3.80 |
| ***SERPINE1*** | serpin peptidase inhibitor, clade E (nexin, plasminogen activator inhibitor type 1), member 1 | 4.56E-10 | 4.26 |
| ***CELA2B*** | chymotrypsin-like elastase family, member 2B | 4.61E-10 | -2.11 |
| ***GZMK*** | granzyme K (granzyme 3; tryptase II) | 4.79E-10 | -2.24 |
| ***HNRNPK*** | heterogeneous nuclear ribonucleoprotein K | 4.79E-10 | 4.77 |
| ***PSMB8*** | proteasome (prosome, macropain) subunit, beta type, 8 (large multifunctional peptidase 7) | 4.83E-10 | 2.69 |
| ***MMP11*** | matrix metallopeptidase 11 (stromelysin 3) | 5.16E-10 | -2.22 |
| ***PSMC3*** | proteasome (prosome, macropain) 26S subunit, ATPase, 3 | 5.41E-10 | 3.07 |
| ***PSMA2*** | proteasome (prosome, macropain) subunit, alpha type, 2 | 5.51E-10 | 4.24 |
| ***KLK13*** | kallikrein-related peptidase 13 | 5.72E-10 | -2.21 |
| ***MMP28*** | matrix metallopeptidase 28 | 6.14E-10 | -2.41 |
| ***SPINK4*** | serine peptidase inhibitor, Kazal type 4 | 6.19E-10 | -2.22 |
| ***MMP27*** | matrix metallopeptidase 27 | 6.66E-10 | -2.06 |
| ***USP6*** | ubiquitin specific peptidase 6 (Tre-2 oncogene) | 6.81E-10 | -2.40 |
| ***KLK10*** | kallikrein-related peptidase 10 | 7.18E-10 | -2.49 |
| ***ANXA3*** | annexin A3 | 7.82E-10 | 5.43 |
| ***ADAMTS12*** | ADAM metallopeptidase with thrombospondin type 1 motif, 12 | 8.82E-10 | -2.06 |
| ***CPA3*** | carboxypeptidase A3 (mast cell) | 9.02E-10 | -2.75 |
| ***TMPRSS4*** | transmembrane protease, serine 4 | 9.44E-10 | -2.31 |
| ***LRP1B*** | low density lipoprotein receptor-related protein 1B | 9.96E-10 | -2.25 |
| ***ADAMTS15*** | ADAM metallopeptidase with thrombospondin type 1 motif, 15 | 1.05E-09 | -2.02 |
| ***KRTAP4-5*** | keratin associated protein 4-5 | 1.09E-09 | -2.36 |
| ***CBX3*** | chromobox homolog 3 | 1.17E-09 | 2.60 |
| ***CPB1*** | carboxypeptidase B1 (tissue) | 1.22E-09 | -2.05 |
| ***PSMD1*** | proteasome (prosome, macropain) 26S subunit, non-ATPase, 1 | 1.27E-09 | 3.07 |
| ***S100A10*** | S100 calcium binding protein A10 | 1.29E-09 | 7.89 |
| ***RPS5*** | ribosomal protein S5 | 1.31E-09 | 4.98 |
| ***CTRL*** | chymotrypsin-like | 1.42E-09 | -2.28 |
| ***CELA3A*** | chymotrypsin-like elastase family, member 3A | 1.46E-09 | -2.12 |
| ***SPINT2*** | serine peptidase inhibitor, Kunitz type, 2 | 1.54E-09 | 4.44 |
| ***CANX*** | calnexin | 1.74E-09 | 5.71 |
| ***KLK7*** | kallikrein-related peptidase 7 | 1.74E-09 | -2.51 |
| ***REEP5*** | receptor accessory protein 5 | 1.94E-09 | 2.68 |
| ***CAPNS1*** | calpain, small subunit 1 | 2.01E-09 | 3.96 |
| ***SERPINB10*** | serpin peptidase inhibitor, clade B (ovalbumin), member 10 | 2.04E-09 | -2.46 |
| ***KLK15*** | kallikrein-related peptidase 15 | 2.20E-09 | -2.13 |
| ***F2*** | coagulation factor II (thrombin) | 2.57E-09 | -2.01 |
| ***PSMD3*** | proteasome (prosome, macropain) 26S subunit, non-ATPase, 3 | 2.72E-09 | 2.91 |
| ***RPS25*** | ribosomal protein S25 | 2.85E-09 | 5.21 |
| ***SPARC*** | secreted protein, acidic, cysteine-rich (osteonectin) | 2.90E-09 | 7.90 |
| ***ANXA6*** | annexin A6 | 2.91E-09 | 2.58 |
| ***SLPI*** | secretory leukocyte peptidase inhibitor | 3.13E-09 | -2.18 |
| ***RPL21*** | ribosomal protein L21 | 3.16E-09 | 3.17 |
| ***ADAM29*** | ADAM metallopeptidase domain 29 | 3.17E-09 | -2.23 |
| ***CTSD*** | cathepsin D | 3.26E-09 | 6.48 |
| ***PSMD4*** | proteasome (prosome, macropain) 26S subunit, non-ATPase, 4 | 3.27E-09 | 3.15 |
| ***COPS4*** | COP9 constitutive photomorphogenic homolog subunit 4 (Arabidopsis) | 3.29E-09 | 2.49 |
| ***RNPS1*** | RNA binding protein S1, serine-rich domain | 3.97E-09 | 2.34 |
| ***PSMD8*** | proteasome (prosome, macropain) 26S subunit, non-ATPase, 8 | 4.14E-09 | 2.41 |
| ***PRSS23*** | protease, serine, 23 | 4.30E-09 | 6.06 |
| ***TMPRSS3*** | transmembrane protease, serine 3 | 4.30E-09 | -2.16 |
| ***COX4I1*** | cytochrome c oxidase subunit IV isoform 1 | 4.35E-09 | 2.14 |
| ***HNRNPC*** | heterogeneous nuclear ribonucleoprotein C (C1/C2) | 4.49E-09 | 2.25 |
| ***SNX3*** | sorting nexin 3 | 4.85E-09 | 2.14 |
| ***SERPINF1*** | serpin peptidase inhibitor, clade F (alpha-2 antiplasmin, pigment epithelium derived factor), member 1 | 6.12E-09 | 8.29 |
| ***IGFBP7*** | insulin-like growth factor binding protein 7 | 6.21E-09 | 7.75 |
| ***ITIH1*** | inter-alpha-trypsin inhibitor heavy chain 1 | 6.24E-09 | -2.18 |
| ***RPL30*** | ribosomal protein L30 | 6.71E-09 | 4.26 |
| ***USP1*** | ubiquitin specific peptidase 1 | 6.72E-09 | 5.14 |
| ***REN*** | renin | 6.91E-09 | -2.14 |
| ***KLK9*** | kallikrein-related peptidase 9 | 7.02E-09 | -2.21 |
| ***RPL12P35*** | ribosomal protein L12 pseudogene 35 | 7.24E-09 | 5.56 |
| ***RPL 17*** | ribosomal protein L17 | 8.44E-09 | 6.98 |
| ***CELA1*** | chymotrypsin-like elastase family, member 1 | 8.45E-09 | -2.28 |
| ***SRP14*** | signal recognition particle 14kDa (homologous Alu RNA binding protein) | 8.78E-09 | 5.48 |
| ***MMP8*** | matrix metallopeptidase 8 (neutrophil collagenase) | 8.84E-09 | -2.56 |
| ***ERH*** | enhancer of rudimentary homolog (Drosophila) | 8.85E-09 | 2.90 |
| ***HINT1*** | histidine triad nucleotide binding protein 1 | 9.06E-09 | 3.52 |
| ***CTSB*** | cathepsin B | 9.52E-09 | 3.26 |
| ***KIFAP3*** | kinesin-associated protein 3 | 9.83E-09 | 3.97 |
| ***ARF1*** | ADP-ribosylation factor 1 | 9.90E-09 | 3.78 |
| ***CAPN2*** | calpain 2, (m/II) large subunit | 1.04E-08 | 4.26 |
| ***SERPINA5*** | serpin peptidase inhibitor, clade A (alpha-1 antiproteinase, antitrypsin), member 5 | 1.13E-08 | -2.11 |
| ***EIF3D*** | eukaryotic translation initiation factor 3, subunit D | 1.18E-08 | 2.33 |
| ***SEPT2*** | septin 2 | 1.18E-08 | 3.30 |
| ***DDX39B*** | DEAD (Asp-Glu-Ala-Asp) box polypeptide 39B | 1.23E-08 | 2.65 |
| ***EPPIN*** | epididymal peptidase inhibitor | 1.30E-08 | -2.44 |
| ***RPS27A*** | ribosomal protein S27a | 1.32E-08 | 3.08 |
| ***MMP2*** | matrix metallopeptidase 2 (gelatinase A, 72kDa gelatinase, 72kDa type IV collagenase) | 1.53E-08 | -2.40 |
| ***HSP90AB1*** | heat shock protein 90kDa alpha (cytosolic), class B member 1 | 1.55E-08 | 2.35 |
| ***ZNF146*** | zinc finger protein 146 | 1.57E-08 | 2.36 |
| ***RPS7*** | ribosomal protein S7 | 1.61E-08 | 4.09 |
| ***MEP1A*** | meprin A, alpha (PABA peptide hydrolase) | 1.61E-08 | -2.24 |
| ***PGC*** | progastricsin (pepsinogen C) | 1.63E-08 | -2.07 |
| ***MAP2K2*** | mitogen-activated protein kinase kinase 2 | 1.66E-08 | 2.79 |
| ***PCSK6*** | proprotein convertase subtilisin/kexin type 6 | 1.67E-08 | -2.05 |
| ***ADAMDEC1*** | ADAM-like, decysin 1 | 2.09E-08 | -2.48 |
| ***CPA5*** | carboxypeptidase A5 | 2.16E-08 | -2.02 |
| ***CAST*** | calpastatin | 2.16E-08 | 2.54 |
| ***PSMD11*** | proteasome (prosome, macropain) 26S subunit, non-ATPase, 11 | 2.31E-08 | 2.22 |
| ***RPL35*** | ribosomal protein L35 | 2.51E-08 | 8.68 |
| ***RPL11*** | ribosomal protein L11 | 2.64E-08 | 4.10 |
| ***RPL9*** | ribosomal protein L9 | 2.72E-08 | 4.09 |
| ***PRPF8*** | PRP8 pre-mRNA processing factor 8 homolog (S. cerevisiae) | 3.20E-08 | 3.03 |
| ***PLAU*** | plasminogen activator, urokinase | 3.43E-08 | 5.67 |
| ***CST1*** | cystatin SN | 3.86E-08 | 24.7 |
| ***PSMA7*** | proteasome (prosome, macropain) subunit, alpha type, 7 | 3.89E-08 | 11.1 |
| ***RPS11*** | ribosomal protein S11 | 3.98E-08 | 6.27 |
| ***STARD7*** | StAR-related lipid transfer (START) domain containing 7 | 4.14E-08 | 2.71 |
| ***PRSS16*** | protease, serine, 16 (thymus) | 4.31E-08 | -2.41 |
| ***SPOCK2*** | sparc/osteonectin, cwcv and kazal-like domains proteoglycan (testican) 2 | 4.46E-08 | -2.01 |
| ***ELANE*** | elastase, neutrophil expressed | 4.49E-08 | -2.06 |
| ***MMP16*** | matrix metallopeptidase 16 (membrane-inserted) | 4.60E-08 | -2.16 |
| ***CTRC*** | chymotrypsin C (caldecrin) | 4.63E-08 | -2.02 |
| ***OAZ1*** | ornithine decarboxylase antizyme 1 | 4.93E-08 | 9.67 |
| ***SLC25A3*** | solute carrier family 25 (mitochondrial carrier; phosphate carrier), member 3 | 5.20E-08 | 2.33 |
| ***KLK11*** | kallikrein-related peptidase 11 | 5.31E-08 | -2.13 |
| ***ADAM12*** | ADAM metallopeptidase domain 12 | 5.58E-08 | -2.22 |
| ***ADAMTS19*** | ADAM metallopeptidase with thrombospondin type 1 motif, 19 | 5.60E-08 | -2.19 |
| ***MASP2*** | mannan-binding lectin serine peptidase 2 | 5.78E-08 | -2.16 |
| ***KARS*** | lysyl-tRNA synthetase | 6.18E-08 | 2.81 |
| ***FAU*** | Finkel-Biskis-Reilly murine sarcoma virus (FBR-MuSV) ubiquitously expressed | 6.59E-08 | 2.65 |
| ***ADAMTS6*** | ADAM metallopeptidase with thrombospondin type 1 motif, 6 | 7.43E-08 | -2.13 |
| ***RPS13*** | ribosomal protein S13 | 7.82E-08 | 6.27 |
| ***PROZ*** | protein Z, vitamin K-dependent plasma glycoprotein | 7.83E-08 | -2.05 |
| ***COL4A6*** | collagen, type IV, alpha 6 | 8.23E-08 | -2.45 |
| ***TIMP2*** | TIMP metallopeptidase inhibitor 2 | 9.67E-08 | 2.62 |
| ***RPS10*** | ribosomal protein S10 | 1.25E-07 | 4.74 |
| ***PSMD10*** | proteasome (prosome, macropain) 26S subunit, non-ATPase, 10 | 1.56E-07 | 2.06 |
| ***MMP17*** | matrix metallopeptidase 17 (membrane-inserted) | 1.82E-07 | -2.06 |
| ***CMA1*** | chymase 1, mast cell | 1.88E-07 | -2.25 |
| ***CST3*** | cystatin C | 2.02E-07 | 11.4 |
| ***SERPINA2*** | serpin peptidase inhibitor, clade A (alpha-1 antiproteinase, antitrypsin), member 2 | 2.11E-07 | -2.09 |
| ***RPL28*** | ribosomal protein L28 | 2.13E-07 | 9.88 |
| ***MEP1B*** | meprin A, beta | 2.28E-07 | -2.22 |
| ***ADAMTS2*** | ADAM metallopeptidase with thrombospondin type 1 motif, 2 | 2.32E-07 | -2.01 |
| ***CTSZ*** | cathepsin Z | 2.45E-07 | 2.49 |
| ***CAPN6*** | calpain 6 | 2.62E-07 | -2.06 |
| ***FNTA*** | farnesyltransferase, CAAX box, alpha | 2.70E-07 | 2.32 |
| ***HRG*** | histidine-rich glycoprotein | 4.51E-07 | -2.12 |
| ***CASP2*** | caspase 2, apoptosis-related cysteine peptidase | 4.70E-07 | -2.04 |
| ***GUK1*** | guanylate kinase 1 | 4.77E-07 | 2.37 |
| ***SERPINB3*** | serpin peptidase inhibitor, clade B (ovalbumin), member 3 | 4.94E-07 | -2.17 |
| ***CCSER2*** | coiled-coil serine-rich protein 2 | 5.66E-07 | 4.14 |
| ***PCSK1*** | proprotein convertase subtilisin/kexin type 1 | 5.69E-07 | -2.10 |
| ***RPL6*** | ribosomal protein L6 | 5.79E-07 | 9.77 |
| ***HTRA1*** | HtrA serine peptidase 1 | 9.41E-07 | 3.10 |
| ***NONO*** | non-POU domain containing, octamer-binding | 1.23E-06 | 3.99 |
| ***RPL37*** | ribosomal protein L37 | 1.28E-06 | 3.17 |
| ***CTSW*** | cathepsin W | 1.31E-06 | -2.04 |
| ***PSMD7*** | proteasome (prosome, macropain) 26S subunit, non-ATPase, 7 | 1.60E-06 | 2.07 |
| ***PLAT*** | plasminogen activator, tissue | 2.85E-06 | 5.09 |
| ***HNRNPA1*** | heterogeneous nuclear ribonucleoprotein A1 | 3.26E-06 | 5.29 |
| ***RPL34*** | ribosomal protein L34 | 7.18E-06 | 2.56 |
| ***NPM1*** | nucleophosmin (nucleolar phosphoprotein B23, numatrin) | 7.49E-06 | 3.48 |
| ***ADAM21*** | ADAM metallopeptidase domain 21 | 7.52E-06 | -2.08 |
| ***RPS24*** | ribosomal protein S24 | 7.88E-06 | 5.69 |
| ***UBE2D3*** | ubiquitin-conjugating enzyme E2D 3 | 1.17E-05 | -2.08 |
| ***TIMP3*** | TIMP metallopeptidase inhibitor 3 | 5.78E-03 | 2.26 |
| ***SPOCK1*** | sparc/osteonectin, cwcv and kazal-like domains proteoglycan (testican) 1 | 7.64E-03 | -2.09 |

| **c. Tumor-derived genes that show stage-specific expression in bone metastasis** | | | |
|---|---|---|---|
| **Symbol** | Name | *P* Value | Fold Change Late vs Early |
| ***PSMB1*** | proteasome (prosome, macropain) subunit, beta type, 1 | 1.99E-15 | 9.56 |
| ***SERPINE1*** | serpin peptidase inhibitor, clade E (nexin, plasminogen activator inhibitor type 1), member 1 | 2.24E-15 | 13.9 |
| ***PSMD6*** | proteasome (prosome, macropain) 26S subunit, non-ATPase, 6 | 5.17E-15 | 5.18 |
| ***SEC11A*** | SEC11 homolog A (S. cerevisiae) | 5.36E-15 | 5.14 |
| ***ADAM9*** | ADAM metallopeptidase domain 9 | 1.79E-14 | 5.58 |
| ***PSMA3*** | proteasome (prosome, macropain) subunit, alpha type, 3 | 3.95E-14 | 5.75 |
| ***AZIN1*** | antizyme inhibitor 1 | 1.23E-13 | 3.40 |
| ***PSMB6*** | proteasome (prosome, macropain) subunit, beta type, 6 | 2.09E-13 | 6.50 |
| ***PSMA2*** | proteasome (prosome, macropain) subunit, alpha type, 2 | 2.33E-13 | 8.43 |
| ***RPS6*** | ribosomal protein S6 | 2.40E-13 | 7.00 |
| ***APP*** | amyloid beta (A4) precursor protein | 3.30E-13 | 4.31 |
| ***GD*/*2*** | GDP dissociation inhibitor 2 | 3.41E-13 | 4.68 |
| ***CBX3*** | chromobox homolog 3 | 4.11E-13 | 4.18 |
| ***SNRNP200*** | small nuclear ribonucleoprotein 200kDa (U5) | 5.36E-13 | 5.61 |
| ***PSMC2*** | proteasome (prosome, macropain) 26S subunit, ATPase, 2 | 7.09E-13 | 5.28 |
| ***TMED2*** | transmembrane emp24 domain trafficking protein 2 | 7.22E-13 | 6.00 |
| ***EEF2*** | eukaryotic translation elongation factor 2 | 7.27E-13 | 4.25 |
| ***PARK7*** | parkinson protein 7 | 8.82E-13 | 8.05 |
| ***ILF2*** | interleukin enhancer binding factor 2, 45kDa | 9.33E-13 | 3.47 |
| ***EIF3F*** | eukaryotic translation initiation factor 3, subunit F | 1.08E-12 | 4.26 |
| ***PSMC1*** | proteasome (prosome, macropain) 26S subunit, ATPase, 1 | 1.75E-12 | 3.83 |
| ***PSMA4*** | proteasome (prosome, macropain) subunit, alpha type, 4 | 1.75E-12 | 5.99 |
| ***PSMD2*** | proteasome (prosome, macropain) 26S subunit, non-ATPase, 2 | 1.95E-12 | 7.55 |
| ***COX411*** | cytochrome c oxidase subunit IV isoform 1 | 1.96E-12 | 3.11 |
| ***METAP2*** | methionyl aminopeptidase 2 | 2.03E-12 | 3.27 |
| ***MMP24*** | matrix metallopeptidase 24 (membrane-inserted) | 2.23E-12 | -2.58 |
| ***RNPS1*** | RNA binding protein S1, serine-rich domain | 2.28E-12 | 3.48 |
| ***ADAMTS13*** | ADAM metallopeptidase with thrombospondin type 1 motif, 13 | 2.41E-12 | -2.56 |
| ***CAV1*** | caveolin 1, caveolae protein, 22kDa | 2.56E-12 | 7.38 |
| ***RPL 18*** | ribosomal protein L18 | 2.59E-12 | 6.76 |
| ***PSMB3*** | proteasome (prosome, macropain) subunit, beta type, 3 | 2.81E-12 | 4.32 |
| ***HNRNPC*** | heterogeneous nuclear ribonucleoprotein C (C1/C2) | 3.07E-12 | 3.25 |
| ***SRSF9*** | serine/arginine-rich splicing factor 9 | 3.13E-12 | 2.97 |
| ***JTB*** | jumping translocation breakpoint | 3.27E-12 | 4.49 |
| ***RPL4*** | ribosomal protein L4 | 3.71E-12 | 6.21 |
| ***PSMB5*** | proteasome (prosome, macropain) subunit, beta type, 5 | 3.80E-12 | 4.53 |
| ***PSMA6*** | proteasome (prosome, macropain) subunit, alpha type, 6 | 3.91E-12 | 3.29 |
| ***ERH*** | enhancer of rudimentary homolog (Drosophila) | 4.53E-12 | 4.85 |
| ***CSTB*** | cystatin B (stefin B) | 4.64E-12 | 3.61 |
| ***KIFAP3*** | kinesin-associated protein 3 | 4.73E-12 | 7.82 |
| ***NARS*** | asparaginyl-tRNA synthetase | 5.50E-12 | 5.08 |
| ***COPS3*** | COP9 constitutive photomorphogenic homolog subunit 3 (Arabidopsis) | 5.99E-12 | 2.94 |
| ***PSMC5*** | proteasome (prosome, macropain) 26S subunit, ATPase, 5 | 7.22E-12 | 6.09 |
| ***RPL14*** | ribosomal protein L14 | 7.34E-12 | 8.81 |
| ***PSMD7*** | proteasome (prosome, macropain) 26S subunit, non-ATPase, 7 | 8.23E-12 | 4.20 |
| ***SNX3*** | sorting nexin 3 | 8.90E-12 | 2.88 |
| ***ANXA5*** | annexin A5 | 9.80E-12 | 6.86 |
| ***PRSS22*** | protease, serine, 22 | 1.09E-11 | -2.53 |
| ***PSMC3*** | proteasome (prosome, macropain) 26S subunit, ATPase, 3 | 1.23E-11 | 3.91 |
| ***MME*** | membrane metallo-endopeptidase | 1.25E-11 | -2.32 |
| ***PRSS8*** | protease, serine, 8 | 1.33E-11 | -2.57 |
| ***PSMC4*** | proteasome (prosome, macropain) 26S subunit, ATPase, 4 | 1.36E-11 | 2.47 |
| ***HNRNPK*** | heterogeneous nuclear ribonucleoprotein K | 1.49E-11 | 6.47 |
| ***HSP90AB1*** | heat shock protein 90kDa alpha (cytosolic), class B member 1 | 1.61E-11 | 3.41 |
| ***ANXA9*** | annexin A9 | 1.76E-11 | -2.30 |
| ***PSMB4*** | proteasome (prosome, macropain) subunit, beta type, 4 | 2.05E-11 | 6.23 |
| ***PSMD11*** | proteasome (prosome, macropain) 26S subunit, non-ATPase, 11 | 2.06E-11 | 3.18 |
| ***PSMB7*** | proteasome (prosome, macropain) subunit, beta type, 7 | 2.16E-11 | 3.39 |
| ***HP*** | haptoglobin | 2.32E-11 | -2.43 |
| ***REEP5*** | receptor accessory protein 5 | 2.39E-11 | 3.45 |
| ***KLK14*** | kallikrein-related peptidase 14 | 3.54E-11 | -2.27 |
| ***REN*** | renin | 3.69E-11 | -2.73 |
| ***FNTA*** | farnesyltransferase, CAAX box, alpha | 3.75E-11 | 3.95 |
| ***ADAMTS14*** | ADAM metallopeptidase with thrombospondin type 1 motif, 14 | 3.86E-11 | -2.34 |
| ***SPINT1*** | serine peptidase inhibitor, Kunitz type 1 | 3.98E-11 | -2.59 |
| ***PSMA1*** | proteasome (prosome, macropain) subunit, alpha type, 1 | 4.01E-11 | 3.56 |
| ***ZNF146*** | zinc finger protein 146 | 4.34E-11 | 3.25 |
| ***COPS4*** | COP9 constitutive photomorphogenic homolog subunit 4 (Arabidopsis) | 4.48E-11 | 3.14 |
| ***MMP25*** | matrix metallopeptidase 25 | 4.49E-11 | -2.55 |
| ***ANXA1*** | annexin A1 | 4.54E-11 | 14.3 |
| ***HPX*** | hemopexin | 4.74E-11 | -2.75 |
| ***PSMC6*** | proteasome (prosome, macropain) 26S subunit, ATPase, 6 | 5.76E-11 | 3.15 |
| ***PSMB2*** | proteasome (prosome, macropain) subunit, beta type, 2 | 6.05E-11 | 3.94 |
| ***TIMP1*** | TIMP metallopeptidase inhibitor 1 | 6.08E-11 | 4.16 |
| ***SEPT2*** | septin 2 | 6.23E-11 | 4.85 |
| ***SPINK2*** | serine peptidase inhibitor, Kazal type 2 (acrosin-trypsin inhibitor) | 6.23E-11 | -2.25 |
| ***SERPINF2*** | serpin peptidase inhibitor, clade F (alpha-2 antiplasmin, pigment epithelium derived factor), member 2 | 7.02E-11 | -2.38 |
| ***KLK12*** | kallikrein-related peptidase 12 | 7.76E-11 | -2.01 |
| ***FSTL1*** | follistatin-like 1 | 8.10E-11 | 4.13 |
| ***RPL27*** | ribosomal protein L27 | 8.68E-11 | 4.74 |
| ***CELA2B*** | chymotrypsin-like elastase family, member 2B | 8.86E-11 | -2.26 |
| ***SLC25A3*** | solute carrier family 25 (mitochondrial carrier; phosphate carrier), member 3 | 9.06E-11 | 3.31 |
| ***CFLAR*** | CASP8 and FADD-like apoptosis regulator | 9.24E-11 | -2.11 |
| ***RPL10A*** | ribosomal protein L10a | 1.02E-10 | 6.72 |
| ***PI3*** | peptidase inhibitor 3, skin-derived | 1.10E-10 | -2.13 |
| ***SERPINA4*** | serpin peptidase inhibitor, clade A (alpha-1 antiproteinase, antitrypsin), member 4 | 1.10E-10 | -2.35 |
| ***USP1*** | ubiquitin specific peptidase 1 | 1.10E-10 | 7.68 |
| ***PSMD3*** | proteasome (prosome, macropain) 26S subunit, non-ATPase, 3 | 1.17E-10 | 3.54 |
| ***SPINK4*** | serine peptidase inhibitor, Kazal type 4 | 1.20E-10 | -2.38 |
| ***RPL19*** | ribosomal protein L19 | 1.22E-10 | 3.86 |
| ***HINT1*** | histidine triad nucleotide binding protein 1 | 1.39E-10 | 4.83 |
| ***TMPRSS5*** | transmembrane protease, serine 5 | 1.39E-10 | -2.34 |
| ***SRP14*** | signal recognition particle 14kDa (homologous Alu RNA binding protein) | 1.58E-10 | 8.26 |
| ***USP22*** | ubiquitin specific peptidase 22 | 1.59E-10 | 4.58 |
| ***CAPN13*** | calpain 13 | 1.60E-10 | -2.07 |
| ***ADAMTS8*** | ADAM metallopeptidase with thrombospondin type 1 motif, 8 | 1.75E-10 | -2.18 |
| ***CELA3B*** | chymotrypsin-like elastase family, member 3B | 1.87E-10 | -2.22 |
| ***SERPINA3*** | serpin peptidase inhibitor, clade A (alpha-1 antiproteinase, antitrypsin), member 3 | 1.93E-10 | -2.25 |
| ***PSME1*** | proteasome (prosome, macropain) activator subunit 1 (PA28 alpha) | 1.96E-10 | 3.13 |
| ***F12*** | coagulation factor XII (Hageman factor) | 2.02E-10 | -2.25 |
| ***F10*** | coagulation factor X | 2.15E-10 | -2.05 |
| ***FURIN*** | furin (paired basic amino acid cleaving enzyme) | 2.21E-10 | -2.26 |
| ***CST5*** | cystatin D | 2.31E-10 | -2.00 |
| ***ADAMTS15*** | ADAM metallopeptidase with thrombospondin type 1 motif, 15 | 2.47E-10 | -2.13 |
| ***ARF1*** | ADP-ribosylation factor 1 | 3.01E-10 | 4.99 |
| ***CAPNS1*** | calpain, small subunit 1 | 3.03E-10 | 4.58 |
| ***SMNDC1*** | survival motor neuron domain containing 1 | 3.15E-10 | 3.57 |
| ***ADAM29*** | ADAM metallopeptidase domain 29 | 3.27E-10 | -2.48 |
| ***ANXA3*** | annexin A3 | 3.41E-10 | 5.85 |
| ***CANX*** | calnexin | 3.55E-10 | 6.66 |
| ***MMP15*** | matrix metallopeptidase 15 (membrane-inserted) | 4.34E-10 | -2.38 |
| ***ANAPC5*** | anaphase promoting complex subunit 5 | 4.53E-10 | 2.07 |
| ***TPSG1*** | tryptase gamma 1 | 4.70E-10 | -2.24 |
| ***LAP3*** | leucine aminopeptidase 3 | 4.96E-10 | 2.63 |
| ***FETUB*** | fetuin B | 4.99E-10 | -2.19 |
| ***GZMM*** | granzyme M (lymphocyte met-ase 1) | 5.60E-10 | -2.33 |
| ***KLK13*** | kallikrein-related peptidase 13 | 5.68E-10 | -2.22 |
| ***F2*** | coagulation factor II (thrombin) | 6.87E-10 | -2.12 |
| ***RPS5*** | ribosomal protein S5 | 7.11E-10 | 5.26 |
| ***PSMD8*** | proteasome (prosome, macropain) 26S subunit, non-ATPase, 8 | 7.62E-10 | 2.62 |
| ***NAPSA*** | napsin A aspartic peptidase | 7.76E-10 | -2.26 |
| ***CTRB1*** | chymotrypsinogen B1 | 7.87E-10 | -2.26 |
| ***CAPN12*** | calpain 12 | 8.39E-10 | -2.35 |
| ***PRSS21*** | protease, serine, 21 (testisin) | 8.40E-10 | -2.36 |
| ***RPS25*** | ribosomal protein S25 | 8.65E-10 | 5.81 |
| ***PSMD13*** | proteasome (prosome, macropain) 26S subunit, non-ATPase, 13 | 9.78E-10 | 2.73 |
| ***ADAMTS7*** | ADAM metallopeptidase with thrombospondin type 1 motif, 7 | 9.79E-10 | -2.34 |
| ***PRTN3*** | proteinase 3 | 1.01E-09 | -2.08 |
| ***MMP11*** | matrix metallopeptidase 11 (stromelysin 3) | 1.05E-09 | -2.16 |
| ***ADAM6*** | ADAM metallopeptidase domain 6, pseudogene | 1.12E-09 | -2.36 |
| ***ADAM11*** | ADAM metallopeptidase domain 11 | 1.28E-09 | -2.40 |
| ***RHOA*** | ras homolog family member A | 1.33E-09 | 4.45 |
| ***FBLN1*** | fibulin 1 | 1.51E-09 | -2.40 |
| ***EIF3M*** | eukaryotic translation initiation factor 3, subunit M | 1.52E-09 | 2.62 |
| ***RPL9*** | ribosomal protein L9 | 1.63E-09 | 5.20 |
| ***LY6H*** | lymphocyte antigen 6 complex, locus | 1.68E-09 | -2.09 |
| ***KLK1*** | kallikrein 1 | 1.80E-09 | -2.20 |
| ***PRPF8*** | PRP8 pre-mRNA processing factor 8 homolog (S. cerevisiae) | 1.87E-09 | 3.67 |
| ***CELA3A*** | chymotrypsin-like elastase family, member 3A | 1.98E-09 | -2.10 |
| ***S100A10*** | S100 calcium binding protein A10 | 2.06E-09 | 7.49 |
| ***PSMF1*** | proteasome (prosome, macropain) inhibitor subunit 1 (PI31) | 2.09E-09 | 2.09 |
| ***PRSS23*** | protease, serine, 23 | 2.13E-09 | 6.50 |
| ***KARS*** | lysyl-tRNA synthetase | 2.15E-09 | 3.49 |
| ***SERPINC1*** | serpin peptidase inhibitor, clade C (antithrombin), member 1 | 2.48E-09 | -2.31 |
| ***PRSS1*** | protease, serine, 1 (trypsin 1) | 2.61E-09 | -2.02 |
| ***PSMD10*** | proteasome (prosome, macropain) 26S subunit, non-ATPase, 10 | 2.68E-09 | 2.49 |
| ***CAPN5*** | calpain 5 | 2.78E-09 | -2.26 |
| ***EIF3D*** | eukaryotic translation initiation factor 3, subunit D | 2.88E-09 | 2.50 |
| ***TMPRSS7*** | transmembrane protease, serine 7 | 2.95E-09 | -2.00 |
| ***RPL21*** | ribosomal protein L21 | 3.04E-09 | 3.18 |
| ***CST11*** | cystatin 11 | 3.10E-09 | -2.12 |
| ***SPINT2*** | serine peptidase inhibitor, Kunitz type, 2 | 3.23E-09 | 4.18 |
| ***DPP8*** | dipeptidyl-peptidase 8 | 3.38E-09 | 2.99 |
| ***RPS7*** | ribosomal protein S7 | 3.39E-09 | 4.65 |
| ***KLK6*** | kallikrein-related peptidase 6 | 3.40E-09 | -2.04 |
| ***PLAU*** | plasminogen activator, urokinase | 3.45E-09 | 7.21 |
| ***SERPINB10*** | serpin peptidase inhibitor, clade B (ovalbumin), member 10 | 3.63E-09 | -2.39 |
| ***CRNKL1*** | crooked neck pre-mRNA splicing factor-like 1 (Drosophila) | 4.05E-09 | -2.17 |
| ***KLK15*** | kallikrein-related peptidase 15 | 4.17E-09 | -2.07 |
| ***PSMD1*** | proteasome (prosome, macropain) 26S subunit, non-ATPase, 1 | 4.69E-09 | 2.85 |
| ***IGFBP7*** | insulin-like growth factor binding protein 7 | 4.73E-09 | 7.99 |
| ***KLK9*** | kallikrein-related peptidase 9 | 5.42E-09 | -2.24 |
| ***DAD1*** | defender against cell death 1 | 5.66E-09 | 2.41 |
| ***SLPI*** | secretory leukocyte peptidase inhibitor | 6.12E-09 | -2.12 |
| ***ADAMTS2*** | ADAM metallopeptidase with thrombospondin type 1 motif, 2 | 6.43E-09 | -2.37 |
| ***GAL*** | galanin prepropeptide | 6.46E-09 | -2.01 |
| ***CAPN2*** | calpain 2, (m/ll) large subunit | 6.67E-09 | 4.42 |
| ***CPA5*** | carboxypeptidase A5 | 6.76E-09 | -2.12 |
| ***RPL30*** | ribosomal protein L30 | 6.76E-09 | 4.26 |
| ***GZMB*** | granzyme B (granzyme 2, cytotoxic T-lymphocyte-associated serine esterase 1) | 7.05E-09 | -2.22 |
| ***CTSS*** | cathepsin S | 7.13E-09 | -2.11 |
| ***HTRA3*** | HtrA serine peptidase 3 | 7.86E-09 | -2.18 |
| ***TRAF2*** | TNF receptor-associated factor 2 | 8.79E-09 | -2.03 |
| ***CASP14*** | caspase 14, apoptosis-related cysteine peptidase | 9.86E-09 | -2.01 |
| ***C6*** | complement component 6 | 1.15E-08 | -2.01 |
| ***KRTAP4-5*** | keratin associated protein 4-5 | 1.42E-08 | -2.11 |
| ***RPS11*** | ribosomal protein S11 | 1.53E-08 | 6.96 |
| ***CELA1*** | chymotrypsin-like elastase family, member 1 | 1.65E-08 | -2.22 |
| ***SPG7*** | spastic paraplegia 7 (pure and complicated autosomal recessive) | 1.73E-08 | 3.00 |
| ***MMP17*** | matrix metallopeptidase 17 (membrane-inserted) | 1.74E-08 | -2.29 |
| ***SERPINA5*** | serpin peptidase inhibitor, clade A (alpha-1 antiproteinase, antitrypsin), member 5 | 1.79E-08 | -2.07 |
| ***TMPRSS3*** | transmembrane protease, serine 3 | 2.26E-08 | -2.02 |
| ***ITIH1*** | inter-alpha-trypsin inhibitor heavy chain 1 | 2.28E-08 | -2.06 |
| ***KLK10*** | kallikrein-related peptidase 10 | 2.29E-08 | -2.13 |
| ***RPS13*** | ribosomal protein S13 | 2.33E-08 | 7.17 |
| ***RPL35*** | ribosomal protein L35 | 2.47E-08 | 8.70 |
| ***ANXA6*** | annexin A6 | 2.88E-08 | 2.30 |
| ***STARD7*** | StAR-related lipid transfer (START) domain containing 7 | 3.63E-08 | 2.73 |
| ***PSMD12*** | proteasome (prosome, macropain) 26S subunit, non-ATPase, 12 | 3.91E-08 | 2.47 |
| ***CAST*** | calpastatin | 4.84E-08 | 2.43 |
| ***RPL28*** | ribosomal protein L28 | 5.24E-08 | 12.1 |
| ***CTSB*** | cathepsin B | 5.27E-08 | 2.92 |
| ***RPS27A*** | ribosomal protein S27a | 5.65E-08 | 2.81 |
| ***CTRC*** | chymotrypsin C (caldecrin) | 6.19E-08 | -2.00 |
| ***KRTAP4-7*** | keratin associated protein 4-7 | 7.10E-08 | -2.01 |
| ***ADAMTS19*** | ADAM metallopeptidase with thrombospondin type 1 motif, 19 | 7.18E-08 | -2.17 |
| ***RPL11*** | ribosomal protein L11 | 7.75E-08 | 3.76 |
| ***ARF3*** | ADP-ribosylation factor 3 | 7.85E-08 | 2.71 |
| ***CTSD*** | cathepsin D | 8.09E-08 | 4.74 |
| ***ZMPSTE24*** | zinc metallopeptidase STE24 homolog (S. cerevisiae) | 8.26E-08 | 2.58 |
| ***RPS10*** | ribosomal protein S10 | 8.52E-08 | 4.92 |
| ***PSMA7*** | proteasome (prosome, macropain) subunit, alpha type, 7 | 8.96E-08 | 9.91 |
| ***ADAMTS16*** | ADAM metallopeptidase with thrombospondin type 1 motif, 16 | 1.11E-07 | -2.08 |
| ***CTSC*** | cathepsin C | 1.18E-07 | 2.32 |
| ***HTRA1*** | HtrA serine peptidase 1 | 1.37E-07 | 3.58 |
| ***MMP2*** | matrix metallopeptidase 2 (gelatinase A, 72kDa gelatinase, 72kDa type IV collagenase) | 1.41E-07 | -2.15 |
| ***MMP16*** | matrix metallopeptidase 16 (membrane-inserted) | 1.81E-07 | -2.03 |
| ***PRSS2*** | protease, serine, 2 (trypsin 2) | 1.86E-07 | -2.42 |
| ***OAZ1*** | ornithine decarboxylase antizyme 1 | 1.87E-07 | 8.12 |
| ***RPL17*** | ribosomal protein L17 | 1.88E-07 | 5.06 |
| ***PRSS16*** | protease, serine, 16 (thymus) | 2.25E-07 | -2.22 |
| ***HRG*** | histidine-rich glycoprotein | 2.26E-07 | -2.19 |
| ***RPL12P35*** | ribosomal protein L12 pseudogene 35 | 3.15E-07 | 3.95 |
| ***RPL6*** | ribosomal protein L6 | 3.91E-07 | 10.3 |
| ***USP4*** | ubiquitin specific peptidase 4 (proto-oncogene) | 4.39E-07 | 2.02 |
| ***CTSW*** | cathepsin W | 4.66E-07 | -2.14 |
| ***MMP8*** | matrix metallopeptidase 8 (neutrophil collagenase) | 4.91E-07 | -2.10 |
| ***PSMD4*** | proteasome (prosome, macropain) 26S subunit, non-ATPase, 4 | 6.00E-07 | 2.32 |
| ***TCEB2*** | transcription elongation factor B (SIII), polypeptide 2 (18kDa, elongin B) | 8.75E-07 | 2.01 |
| ***UBE2D3*** | ubiquitin-conjugating enzyme E2D 3 | 8.92E-07 | -2.40 |
| ***DDX39B*** | DEAD (Asp-Glu-Ala-Asp) box polypeptide 39B | 1.04E-06 | 2.11 |
| ***CASP3*** | caspase 3, apoptosis-related cysteine peptidase | 1.06E-06 | 2.04 |
| ***PSME4*** | proteasome (prosome, macropain) activator subunit 4 | 1.22E-06 | 2.26 |
| ***C1D*** | C1 D nuclear receptor corepressor | 1.26E-06 | 2.54 |
| ***MAP2K2*** | mitogen-activated protein kinase kinase 2 | 1.33E-06 | 2.19 |
| ***UBA1*** | ubiquitin-like modifier activating enzyme 1 | 1.36E-06 | 2.04 |
| ***RPL37*** | ribosomal protein L37 | 1.38E-06 | 3.15 |
| ***HNRNPA1*** | heterogeneous nuclear ribonucleoprotein A1 | 1.75E-06 | 5.68 |
| ***GUK1*** | guanylate kinase 1 | 2.05E-06 | 2.19 |
| ***ADAM17*** | ADAM metallopeptidase domain 17 | 3.06E-06 | 2.01 |
| ***CST3*** | cystatin C | 3.24E-06 | 7.65 |
| ***ATP6V0E1*** | ATPase, H+ transporting, lysosomal 9kDa, V0 subunit e1 | 5.36E-06 | 2.14 |
| ***NPM1*** | nucleophosmin (nucleolar phosphoprotein B23, numatrin) | 7.25E-06 | 3.49 |
| ***NONO*** | non-POU domain containing, octamer-binding | 8.73E-06 | 3.35 |
| ***PLAT*** | plasminogen activator, tissue | 1.03E-05 | 4.42 |
| ***CAPN7*** | calpain 7 | 1.19E-05 | 2.05 |
| ***CTSL1*** | cathepsin L1 | 1.41E-05 | 2.16 |
| ***RPS24*** | ribosomal protein S24 | 2.73E-05 | 4.88 |
| ***CCSER2*** | coiled-coil serine-rich protein 2 | 2.38E-04 | 2.47 |
| ***SPOCK1*** | sparc/osteonectin, cwcv and kazal-like domains proteoglycan (testican) 1 | 3.84E-04 | 2.85 |
| ***CST1*** | cystatin SN | 1.17E-02 | 2.97 |

| **d. Tumor-derived genes that show stage-specific expression in lung metastasis** | | | |
|---|---|---|---|
| **Symbol** | Name | *P* Value | Fold Change Late vs Early |
| ***SEC11A*** | SEC11 homolog A (S. cerevisiae) | 2.08E-16 | 6.70 |
| ***EEF2*** | eukaryotic translation elongation factor 2 | 4.74E-15 | 6.29 |
| ***PSMB1*** | proteasome (prosome, macropain) subunit, beta type, 1 | 5.22E-15 | 8.66 |
| ***PSMA3*** | proteasome (prosome, macropain) subunit, alpha type, 3 | 2.04E-14 | 6.08 |
| ***PSMA4*** | proteasome (prosome, macropain) subunit, alpha type, 4 | 4.41E-14 | 8.47 |
| ***PARK7*** | parkinson protein 7 | 5.45E-14 | 10.8 |
| ***TIMP1*** | TIMP metallopeptidase inhibitor 1 | 1.08E-13 | 7.00 |
| ***RPL18*** | ribosomal protein L18 | 2.46E-13 | 8.51 |
| ***CTSB*** | cathepsin B | 3.00E-13 | 7.48 |
| ***ERH*** | enhancer of rudimentary homolog (Drosophila) | 3.26E-13 | 6.02 |
| ***PSMB4*** | proteasome (prosome, macropain) subunit, beta type, 4 | 3.30E-13 | 9.40 |
| ***SPARC*** | secreted protein, acidic, cysteine-rich (osteonectin) | 3.46E-13 | 26.3 |
| ***CAV1*** | caveolin 1, caveolae protein, 22kDa | 4.68E-13 | 8.77 |
| ***CBX3*** | chromobox homolog 3 | 5.79E-13 | 4.08 |
| ***APP*** | amyloid beta (A4) precursor protein | 6.01E-13 | 4.14 |
| ***PSMB6*** | proteasome (prosome, macropain) subunit, beta type, 6 | 8.65E-13 | 5.74 |
| ***PSMD6*** | proteasome (prosome, macropain) 26S subunit, non-ATPase, 6 | 1.07E-12 | 3.58 |
| ***KLK12*** | kallikrein-related peptidase 12 | 1.12E-12 | -2.37 |
| ***EIF3F*** | eukaryotic translation initiation factor 3, subunit F | 1.51E-12 | 4.16 |
| ***PSMC2*** | proteasome (prosome, macropain) 26S subunit, ATPase, 2 | 1.75E-12 | 4.91 |
| ***ANXA5*** | annexin A5 | 2.07E-12 | 8.00 |
| ***TMED2*** | transmembrane emp24 domain trafficking protein 2 | 2.18E-12 | 5.46 |
| ***GDI2*** | GDP dissociation inhibitor 2 | 2.24E-12 | 4.09 |
| ***IGFBP7*** | insulin-like growth factor binding protein 7 | 2.27E-12 | 21.9 |
| ***SPINT2*** | serine peptidase inhibitor, Kunitz type, 2 | 2.71E-12 | 7.86 |
| ***NARS*** | asparaginyl-tRNA synthetase | 3.61E-12 | 5.26 |
| ***PSMB5*** | proteasome (prosome, macropain) subunit, beta type, 5 | 3.75E-12 | 4.54 |
| ***OTUB1*** | OTU domain, ubiquitin aldehyde binding 1 | 5.65E-12 | -2.02 |
| ***HNRNPC*** | heterogeneous nuclear ribonucleoprotein C (C1/C2) | 6.59E-12 | 3.11 |
| ***RPL4*** | ribosomal protein L4 | 6.64E-12 | 5.89 |
| ***ANXA1*** | annexin A1 | 7.33E-12 | 18.5 |
| ***PSMB7*** | proteasome (prosome, macropain) subunit, beta type, 7 | 7.55E-12 | 3.62 |
| ***RPS6*** | ribosomal protein S6 | 1.16E-11 | 5.01 |
| ***ANXA9*** | annexin A9 | 1.24E-11 | -2.34 |
| ***SNX3*** | sorting nexin 3 | 1.74E-11 | 2.78 |
| ***RPL 19*** | ribosomal protein L19 | 1.86E-11 | 4.41 |
| ***RPL27*** | ribosomal protein L27 | 2.20E-11 | 5.30 |
| ***JTB*** | jumping translocation breakpoint | 3.45E-11 | 3.80 |
| ***MMP24*** | matrix metallopeptidase 24 (membrane-inserted) | 3.53E-11 | -2.29 |
| ***CTSD*** | cathepsin D | 3.89E-11 | 10.6 |
| ***PSMC1*** | proteasome (prosome, macropain) 26S subunit, ATPase, 1 | 4.35E-11 | 3.14 |
| ***KIFAP3*** | kinesin-associated protein 3 | 4.47E-11 | 6.29 |
| ***CANX*** | calnexin | 4.63E-11 | 8.22 |
| ***ADAMTS13*** | ADAM metallopeptidase with thrombospondin type 1 motif, 13 | 4.70E-11 | -2.25 |
| ***PRSS50*** | protease, serine, 50 | 4.84E-11 | -2.13 |
| ***PI3*** | peptidase inhibitor 3, skin-derived | 5.52E-11 | -2.18 |
| ***SERPINF2*** | serpin peptidase inhibitor, clade F (alpha-2 antiplasmin, pigment epithelium derived factor), member 2 | 7.01E-11 | -2.38 |
| ***S100A10*** | S100 calcium binding protein A10 | 7.76E-11 | 10.9 |
| ***PSMA6*** | proteasome (prosome, macropain) subunit, alpha type, 6 | 7.86E-11 | 2.79 |
| ***SRP14*** | signal recognition particle 14kDa (homologous Alu RNA binding protein) | 8.07E-11 | 8.89 |
| ***CAPN2*** | calpain 2, (m/ll) large subunit | 8.28E-11 | 6.53 |
| ***PSMC3*** | proteasome (prosome, macropain) 26S subunit, ATPase, 3 | 8.54E-11 | 3.44 |
| ***ILF2*** | interleukin enhancer binding factor 2, 45kDa | 8.65E-11 | 2.70 |
| ***PSMD4*** | proteasome (prosome, macropain) 26S subunit, non-ATPase, 4 | 8.83E-11 | 4.01 |
| ***MME*** | membrane metallo-endopeptidase | 9.49E-11 | -2.14 |
| ***CPB1*** | carboxypeptidase B1 (tissue) | 1.06E-10 | -2.26 |
| ***ST14*** | suppression of tumorigenicity 14 (colon carcinoma) | 1.16E-10 | -2.17 |
| ***HP*** | haptoglobin | 1.18E-10 | -2.26 |
| ***PSMC5*** | proteasome (prosome, macropain) 26S subunit, ATPase, 5 | 1.37E-10 | 4.75 |
| ***RNPS1*** | RNA binding protein S1, serine-rich domain | 1.55E-10 | 2.74 |
| ***PCSK4*** | proprotein convertase subtilisin/kexin type 4 | 1.66E-10 | -2.03 |
| ***USP22*** | ubiquitin specific peptidase 22 | 1.80E-10 | 4.54 |
| ***ADAM11*** | ADAM metallopeptidase domain 11 | 1.92E-10 | -2.63 |
| ***KLK6*** | kallikrein-related peptidase 6 | 1.96E-10 | -2.29 |
| ***PRTN3*** | proteinase 3 | 1.98E-10 | -2.22 |
| ***PRSS8*** | protease, serine, 8 | 1.98E-10 | -2.28 |
| ***CELA2B*** | chymotrypsin-like elastase family, member 2B | 2.02E-10 | -2.18 |
| ***SERPINA4*** | serpin peptidase inhibitor, clade A (alpha-1 antiproteinase, antitrypsin), member 4 | 2.05E-10 | -2.29 |
| ***LY6H*** | lymphocyte antigen 6 complex, locus H | 2.18E-10 | -2.27 |
| ***KLK14*** | kallikrein-related peptidase 14 | 2.33E-10 | -2.11 |
| ***CSTB*** | cystatin B (stefin B) | 2.40E-10 | 2.86 |
| ***KLK2*** | kallikrein-related peptidase 2 | 2.44E-10 | -2.08 |
| ***ADAMTS15*** | ADAM metallopeptidase with thrombospondin type 1 motif, 15 | 2.65E-10 | -2.13 |
| ***TMPRSS4*** | transmembrane protease, serine 4 | 2.66E-10 | -2.45 |
| ***PSMA1*** | proteasome (prosome, macropain) subunit, alpha type, 1 | 2.66E-10 | 3.16 |
| ***MMP15*** | matrix metallopeptidase 15 (membrane-inserted) | 2.74E-10 | -2.43 |
| ***GZMM*** | granzyme M (lymphocyte met-ase 1) | 2.90E-10 | -2.40 |
| ***DAD1*** | defender against cell death 1 | 3.03E-10 | 2.80 |
| ***SPINK2*** | serine peptidase inhibitor, Kazal type 2 (acrosin-trypsin inhibitor) | 3.16E-10 | -2.11 |
| ***ITIH1*** | inter-alpha-trypsin inhibitor heavy chain 1 | 3.22E-10 | -2.49 |
| ***PRSS22*** | protease, serine, 22 | 3.34E-10 | -2.18 |
| ***ZNF146*** | zinc finger protein 146 | 3.38E-10 | 2.89 |
| ***MMP25*** | matrix metallopeptidase 25 | 3.57E-10 | -2.32 |
| ***RPL35*** | ribosomal protein L35 | 3.69E-10 | 15.2 |
| ***CELA3B*** | chymotrypsin-like elastase family, member 3B | 3.82E-10 | -2.16 |
| ***F10*** | coagulation factor X | 4.04E-10 | -2.01 |
| ***ADAMTS7*** | ADAM metallopeptidase with thrombospondin type 1 motif, 7 | 4.15E-10 | -2.43 |
| ***RPL14*** | ribosomal protein L14 | 4.15E-10 | 5.88 |
| ***HNRNPK*** | heterogeneous nuclear ribonucleoprotein K | 4.24E-10 | 4.82 |
| ***KLK13*** | kallikrein-related peptidase 13 | 4.43E-10 | -2.24 |
| ***ACR*** | acrosin | 4.54E-10 | -2.20 |
| ***CTSS*** | cathepsin S | 4.60E-10 | -2.37 |
| ***PLAU*** | plasminogen activator, urokinase | 4.74E-10 | 9.01 |
| ***RPS11*** | ribosomal protein S11 | 4.76E-10 | 10.4 |
| ***PCSK6*** | proprotein convertase subtilisin/kexin type 6 | 5.02E-10 | -2.38 |
| ***ADAM9*** | ADAM metallopeptidase domain 9 | 5.07E-10 | 2.82 |
| ***PSMA2*** | proteasome (prosome, macropain) subunit, alpha type, 2 | 5.49E-10 | 4.24 |
| ***REN*** | renin | 5.54E-10 | -2.39 |
| ***RPS13*** | ribosomal protein S13 | 5.54E-10 | 11.3 |
| ***RPL10A*** | ribosomal protein L10a | 5.57E-10 | 5.72 |
| ***HPX*** | hemopexin | 6.08E-10 | -2.43 |
| ***MMP11*** | matrix metallopeptidase 11 (stromelysin 3) | 6.70E-10 | -2.20 |
| ***SNRNP200*** | small nuclear ribonucleoprotein 200kDa (U5) | 6.81E-10 | 3.33 |
| ***ADAM6*** | ADAM metallopeptidase domain 6, pseudogene | 6.82E-10 | -2.42 |
| ***C6*** | complement component 6 | 7.23E-10 | -2.25 |
| ***SPINK4*** | serine peptidase inhibitor, Kazal type 4 | 7.24E-10 | -2.21 |
| ***RPL21*** | ribosomal protein L21 | 7.44E-10 | 3.48 |
| ***TPSG1*** | tryptase gamma 1 | 8.13E-10 | -2.19 |
| ***RPL9*** | ribosomal protein L9 | 8.28E-10 | 5.53 |
| ***COPS7B*** | COP9 constitutive photomorphogenic homolog subunit 7B (Arabidopsis) | 9.26E-10 | -2.03 |
| ***PSMA7*** | proteasome (prosome, macropain) subunit, alpha type, 7 | 9.44E-10 | 19.4 |
| ***ADAMTS8*** | ADAM metallopeptidase with thrombospondin type 1 motif, 8 | 1.01E-09 | -2.03 |
| ***TMPRSS5*** | transmembrane protease, serine 5 | 1.03E-09 | -2.15 |
| ***PSMB3*** | proteasome (prosome, macropain) subunit, beta type, 3 | 1.07E-09 | 2.95 |
| ***RPL28*** | ribosomal protein L28 | 1.12E-09 | 21.9 |
| ***GZMK*** | granzyme K (granzyme 3; tryptase II) | 1.26E-09 | -2.15 |
| ***NAPSA*** | napsin A aspartic peptidase | 1.32E-09 | -2.21 |
| ***FSTL1*** | follistatin-like 1 | 1.33E-09 | 3.40 |
| ***CTRL*** | chymotrypsin-like | 1.47E-09 | -2.28 |
| ***USP1*** | ubiquitin specific peptidase 1 | 1.53E-09 | 5.90 |
| ***RPS5*** | ribosomal protein S5 | 1.68E-09 | 4.88 |
| ***TMPRSS7*** | transmembrane protease, serine 7 | 1.69E-09 | -2.05 |
| ***F12*** | coagulation factor XII (Hageman factor) | 1.82E-09 | -2.06 |
| ***PRSS1*** | protease, serine, 1 (trypsin 1) | 1.86E-09 | -2.05 |
| ***NONO*** | non-POU domain containing, octamer-binding | 1.86E-09 | 7.61 |
| ***PSMD10*** | proteasome (prosome, macropain) 26S subunit, non-ATPase, 10 | 1.89E-09 | 2.53 |
| ***ADAMTS12*** | ADAM metallopeptidase with | 1.90E-09 | -2.01 |
| | thrombospondin type 1 motif, 12 | | |
| ***FETUB*** | fetuin B | 1.94E-09 | -2.07 |
| ***SRSF9*** | serine/arginine-rich splicing factor 9 | 2.12E-09 | 2.18 |
| ***OAZ1*** | ornithine decarboxylase antizyme 1 | 2.21E-09 | 14.9 |
| ***FBLN1*** | fibulin 1 | 2.58E-09 | -2.34 |
| ***CTRB1*** | chymotrypsinogen B1 | 2.70E-09 | -2.14 |
| ***PRSS23*** | protease, serine, 23 | 2.73E-09 | 6.34 |
| ***KRTAP4-7*** | keratin associated protein 4-7 | 2.88E-09 | -2.30 |
| ***MASP1*** | mannan-binding lectin serine peptidase 1 (C4/C2 activating component of Ra-reactive factor) | 3.01E-09 | -2.14 |
| ***CAPN5*** | calpain 5 | 3.23E-09 | -2.24 |
| ***PSMD2*** | proteasome (prosome, macropain) 26S subunit, non-ATPase, 2 | 3.25E-09 | 3.98 |
| ***CTSC*** | cathepsin C | 3.32E-09 | 2.82 |
| ***PSMB2*** | proteasome (prosome, macropain) subunit, beta type, 2 | 3.49E-09 | 3.02 |
| ***AZIN1*** | antizyme inhibitor 1 | 3.52E-09 | 2.07 |
| ***CST8*** | cystatin 8 (cystatin-related epididymal specific) | 3.67E-09 | -2.08 |
| ***KAT7*** | K(lysine) acetyltransferase 7 | 3.79E-09 | -2.24 |
| ***RPL12P35*** | ribosomal protein L12 pseudogene 35 | 3.83E-09 | 5.92 |
| ***CELA3A*** | chymotrypsin-like elastase family, member 3A | 3.86E-09 | -2.04 |
| ***PSMD13*** | proteasome (prosome, macropain) 26S subunit, non-ATPase, 13 | 4.03E-09 | 2.53 |
| ***ADAMTS18*** | ADAM metallopeptidase with thrombospondin type 1 motif, 18 | 4.33E-09 | -2.03 |
| ***MMP16*** | matrix metallopeptidase 16 (membrane-inserted) | 4.38E-09 | -2.41 |
| ***KRTAP4-5*** | keratin associated protein 4-5 | 4.38E-09 | -2.22 |
| ***CST7*** | cystatin F (leukocystatin) | 4.41E-09 | -2.03 |
| ***METAP2*** | methionyl aminopeptidase 2 | 4.48E-09 | 2.22 |
| ***TMPRSS3*** | transmembrane protease, serine 3 | 5.26E-09 | -2.14 |
| ***KLK9*** | kallikrein-related peptidase 9 | 5.27E-09 | -2.24 |
| ***PRSS55*** | protease, serine, 55 | 5.36E-09 | -2.08 |
| ***HNRNPA1*** | heterogeneous nuclear ribonucleoprotein A1 | 5.41E-09 | 11.7 |
| ***PSMC6*** | proteasome (prosome, macropain) 26S subunit, ATPase, 6 | 5.61E-09 | 2.46 |
| ***PCSK1N*** | proprotein convertase subtilisin/kexin type 1 inhibitor | 5.85E-09 | -2.02 |
| ***ADAMTS19*** | ADAM metallopeptidase with thrombospondin type 1 motif, 19 | 5.99E-09 | -2.44 |
| ***PSME1*** | proteasome (prosome, macropain) activator subunit 1 (PA28 alpha) | 6.15E-09 | 2.58 |
| ***ADAMTS2*** | ADAM metallopeptidase with thrombospondin type 1 motif, 2 | 7.74E-09 | -2.35 |
| ***COPS3*** | COP9 constitutive photomorphogenic homolog subunit 3 (Arabidopsis) | 7.99E-09 | 2.10 |
| ***SERPINB10*** | serpin peptidase inhibitor, clade B (ovalbumin), member 10 | 8.14E-09 | -2.30 |
| ***ADAMTS9*** | ADAM metallopeptidase with thrombospondin type 1 motif, 9 | 9.31E-09 | -2.22 |
| ***REEP5*** | receptor accessory protein 5 | 9.81E-09 | 2.46 |
| ***SERPINC1*** | serpin peptidase inhibitor, clade C (antithrombin), member 1 | 1.01E-08 | -2.17 |
| ***CTRC*** | chymotrypsin C (caldecrin) | 1.02E-08 | -2.16 |
| ***SPINT1*** | serine peptidase inhibitor, Kunitz type 1 | 1.03E-08 | -2.03 |
| ***RPL17*** | ribosomal protein L17 | 1.05E-08 | 6.82 |
| ***SLPI*** | secretory leukocyte peptidase inhibitor | 1.10E-08 | -2.07 |
| ***ELANE*** | elastase, neutrophil expressed | 1.26E-08 | -2.17 |
| ***CAPNS1*** | calpain, small subunit 1 | 1.55E-08 | 3.42 |
| ***GZMB*** | granzyme B (granzyme 2, cytotoxic T-lymphocyte-associated serine esterase 1) | 1.67E-08 | -2.14 |
| ***KLK10*** | kallikrein-related peptidase 10 | 1.72E-08 | -2.15 |
| ***SEPT2*** | septin 2 | 1.77E-08 | 3.21 |
| ***CELA1*** | chymotrypsin-like elastase family, member 1 | 1.82E-08 | -2.21 |
| ***PROZ*** | protein Z, vitamin K-dependent plasma glycoprotein | 2.33E-08 | -2.16 |
| ***SERPIND1*** | serpin peptidase inhibitor, clade D (heparin cofactor), member 1 | 2.43E-08 | -2.08 |
| ***RPS25*** | ribosomal protein S25 | 2.46E-08 | 4.32 |
| ***FNTA*** | farnesyltransferase, CAAX box, alpha | 2.56E-08 | 2.63 |
| ***CPB2*** | carboxypeptidase B2 (plasma) | 2.58E-08 | -2.11 |
| ***RPL30*** | ribosomal protein L30 | 2.70E-08 | 3.81 |
| ***HINT1*** | histidine triad nucleotide binding protein 1 | 2.96E-08 | 3.24 |
| ***RPL6*** | ribosomal protein L6 | 3.07E-08 | 15.2 |
| ***RPS10*** | ribosomal protein S10 | 3.32E-08 | 5.39 |
| ***ZMPSTE24*** | zinc metallopeptidase STE24 homolog (S. cerevisiae) | 3.32E-08 | 2.72 |
| ***CTSL1*** | cathepsin L1 | 3.43E-08 | 3.13 |
| ***MMP9*** | matrix metallopeptidase 9 (gelatinase B, 92kDa gelatinase, 92kDa type IV collagenase) | 3.63E-08 | -2.18 |
| ***HTRA3*** | HtrA serine peptidase 3 | 3.65E-08 | -2.05 |
| ***HRG*** | histidine-rich glycoprotein | 3.65E-08 | -2.40 |
| ***ADAMTS6*** | ADAM metallopeptidase with thrombospondin type 1 motif, 6 | 3.74E-08 | -2.20 |
| ***MASP2*** | mannan-binding lectin serine peptidase 2 | 3.95E-08 | -2.20 |
| ***PSMD3*** | proteasome (prosome, macropain) 26S subunit, non-ATPase, 3 | 4.32E-08 | 2.50 |
| ***DDX39B*** | DEAD (Asp-Glu-Ala-Asp) box polypeptide 39B | 4.82E-08 | 2.46 |
| ***KLK7*** | kallikrein-related peptidase 7 | 4.85E-08 | -2.15 |
| ***COPS4*** | COP9 constitutive photomorphogenic homolog subunit 4 (Arabidopsis) | 4.99E-08 | 2.19 |
| ***F7*** | coagulation factor VII (serum prothrombin conversion accelerator) | 5.03E-08 | -2.04 |
| ***SLC25A3*** | solute carrier family 25 (mitochondrial carrier; phosphate carrier), member 3 | 5.84E-08 | 2.32 |
| ***MEP1A*** | meprin A, alpha (PABA peptide hydrolase) | 6.18E-08 | -2.11 |
| ***MEP1B*** | meprin A, beta | 6.45E-08 | -2.36 |
| ***EPPIN*** | epididymal peptidase inhibitor | 6.96E-08 | -2.25 |
| ***SERPINA2*** | serpin peptidase inhibitor, clade A (alpha-1 antiproteinase, antitrypsin), member 2 | 8.23E-08 | -2.18 |
| ***ADAMTS16*** | ADAM metallopeptidase with thrombospondin type 1 motif, 16 | 9.83E-08 | -2.09 |
| ***CTSW*** | cathepsin W | 1.26E-07 | -2.28 |
| ***CCSER2*** | coiled-coil serine-rich protein 2 | 1.30E-07 | 4.75 |
| ***ANXA6*** | annexin A6 | 1.36E-07 | 2.14 |
| ***CPA3*** | carboxypeptidase A3 (mast cell) | 1.54E-07 | -2.13 |
| ***PRSS16*** | protease, serine, 16 (thymus) | 1.64E-07 | -2.25 |
| ***ARF1*** | ADP-ribosylation factor 1 | 1.66E-07 | 3.09 |
| ***MMP26*** | matrix metallopeptidase 26 | 1.71E-07 | -2.04 |
| ***UBE2D3*** | ubiquitin-conjugating enzyme E2D 3 | 1.75E-07 | -2.63 |
| ***NPM1*** | nucleophosmin (nucleolar phosphoprotein B23, numatrin) | 2.12E-07 | 4.86 |
| ***HSP90AB1*** | heat shock protein 90kDa alpha (cytosolic), class B member 1 | 2.27E-07 | 2.07 |
| ***SPINK5*** | serine peptidase inhibitor, Kazal type 5 | 2.40E-07 | -2.16 |
| ***PSMD1*** | proteasome (prosome, macropain) 26S subunit, non-ATPase, 1 | 2.48E-07 | 2.29 |
| ***CAPN6*** | calpain 6 | 3.07E-07 | -2.05 |
| ***UBA1*** | ubiquitin-like modifier activating enzyme 1 | 3.12E-07 | 2.19 |
| ***PSMD12*** | proteasome (prosome, macropain) 26S subunit, non-ATPase, 12 | 3.32E-07 | 2.22 |
| ***ADAMDEC1*** | ADAM-like, decysin 1 | 3.45E-07 | -2.15 |
| ***CAPN3*** | calpain 3, (p94) | 4.10E-07 | -2.02 |
| ***STARD7*** | StAR-related lipid transfer (START) domain containing 7 | 5.44E-07 | 2.35 |
| ***MAP2K2*** | mitogen-activated protein kinase kinase 2 | 5.48E-07 | 2.30 |
| ***MMP8*** | matrix metallopeptidase 8 (neutrophil collagenase) | 7.06E-07 | -2.06 |
| ***ATP6V0E1*** | ATPase, H+ transporting, lysosomal 9kDa, V0 subunit e1 | 7.59E-07 | 2.38 |
| ***ANXA3*** | annexin A3 | 8.73E-07 | 3.08 |
| ***RPL37*** | ribosomal protein L37 | 1.18E-06 | 3.19 |
| ***CMA1*** | chymase 1, mast cell | 1.22E-06 | -2.05 |
| ***RPS24*** | ribosomal protein S24 | 1.24E-06 | 7.21 |
| ***SERPINB3*** | serpin peptidase inhibitor, clade B (ovalbumin), member 3 | 1.30E-06 | -2.07 |
| ***RPL11*** | ribosomal protein L11 | 1.50E-06 | 3.00 |
| ***COL4A6*** | collagen, type IV, alpha 6 | 1.67E-06 | -2.10 |
| ***CST3*** | cystatin C | 1.80E-06 | 8.31 |
| ***SERPINE1*** | serpin peptidase inhibitor, clade E (nexin, plasminogen activator inhibitor type 1), member 1 | 1.82E-06 | 2.44 |
| ***RPS7*** | ribosomal protein S7 | 2.59E-06 | 2.80 |
| ***GUK1*** | guanylate kinase 1 | 3.08E-06 | 2.14 |
| ***SERPINE2*** | serpin peptidase inhibitor, clade E (nexin, plasminogen activator inhibitor type 1), member 2 | 3.22E-06 | 3.11 |
| ***PRPF8*** | PRP8 pre-mRNA processing factor 8 homolog (S. cerevisiae) | 3.88E-06 | 2.27 |
| ***PRSS2*** | protease, serine, 2 (trypsin 2) | 4.80E-06 | -2.04 |
| ***CST4*** | cystatin S | 5.64E-06 | -2.25 |
| ***CST6*** | cystatin E/M | 8.01E-06 | 2.07 |
| ***HTRA1*** | HtrA serine peptidase 1 | 2.64E-05 | 2.44 |
| ***RHOA*** | ras homolog family member A | 5.28E-05 | 2.14 |
| ***SERPING1*** | serpin peptidase inhibitor, clade G (C1 inhibitor), member 1 | 6.80E-05 | -2.16 |
| ***TIMP3*** | TIMP metallopeptidase inhibitor 3 | 9.16E-05 | 3.55 |
| ***RPL34*** | ribosomal protein L34 | 0.000261 | 2.02 |
| ***SERPINF1*** | serpin peptidase inhibitor, clade F (alpha-2 antiplasmin, pigment epithelium derived factor), member 1 | 0.000272 | 2.76 |
| ***PLAT*** | plasminogen activator, tissue | 0.00033 | 3.05 |

| **e. Stroma-derived genes that show stage-specific expression in brain metastasis** | | | |
|---|---|---|---|
| **Symbol** | Name | *P* Value | Fold Change Late vs Early |
| ***Pcsk1n*** | proprotein convertase subtilisin/kexin type 1 inhibitor | 1.00E-08 | -3.16 |
| ***Serpina3n*** | serine (or cysteine) peptidase inhibitor, clade A, member 3N | 1.55E-08 | 6.83 |
| ***Anxa3*** | annexin A3 | 2.81E-08 | 2.86 |
| ***Nrip3*** | nuclear receptor interacting protein 3 | 3.48E-08 | -3.09 |
| ***BC031181*** | cDNA sequence BC031181 | 7.87E-08 | -2.27 |
| ***Usp18*** | ubiquitin specific peptidase 18 | 1.12E-07 | 2.23 |
| ***Naa60*** | N(alpha)-acetyltransferase 60, NatF catalytic subunit | 3.17E-07 | -2.19 |
| ***Ctsh*** | cathepsin H | 4.90E-07 | 3.15 |
| ***Aplp2*** | amyloid beta (A4) precursor-like protein 2 | 6.60E-07 | -2.03 |
| ***Psmc5*** | protease (prosome, macropain) 26S subunit, ATPase 5 | 6.76E-07 | -2.41 |
| ***Anxa2*** | annexin A2 | 1.00E-06 | 2.21 |
| ***Psmb6*** | proteasome (prosome, macropain) subunit, beta type 6 | 1.06E-06 | -2.12 |
| ***Ctss*** | cathepsin S | 1.52E-06 | 2.60 |
| ***Usp9x*** | ubiquitin specific peptidase 9, X chromosome | 2.02E-06 | -2.01 |
| ***Igfbp7*** | insulin-like growth factor binding protein 7 | 2.56E-06 | 3.33 |
| ***Ctsf*** | cathepsin F | 5.12E-06 | -2.08 |
| ***Ctsz*** | cathepsin Z | 7.13E-06 | 2.62 |
| ***Timp1*** | tissue inhibitor of metalloproteinase 1 | 9.29E-06 | 3.25 |
| ***Ddx24*** | DEAD (Asp-Glu-Ala-Asp) box | 1.28E-05 | -2.38 |
| | polypeptide 24 | | |
| ***Sec22b*** | SEC22 vesicle trafficking protein homolog B (S. cerevisiae) | 1.61E-05 | -2.20 |
| ***Psmd11*** | proteasome (prosome, macropain) 26S subunit, non-ATPase, 11 | 1.64E-05 | -3.06 |
| ***Mrp*/*27*** | mitochondrial ribosomal protein L27 | 2.18E-05 | -2.22 |
| ***Bace1*** | beta-site APP cleaving enzyme 1 | 2.70E-05 | -2.09 |
| ***Snapin*** | SNAP-associated protein | 2.98E-05 | -2.45 |
| ***Ube2g1*** | ubiquitin-conjugating enzyme E2G 1 | 3.43E-05 | -2.09 |
| ***Zranb1*** | zinc finger, RAN-binding domain containing 1 | 3.50E-05 | -2.43 |
| ***Psmb8*** | proteasome (prosome, macropain) subunit, beta type 8 (large multifunctional peptidase 7) | 5.20E-05 | 3.46 |
| ***Psmc6*** | proteasome (prosome, macropain) 26S subunit, ATPase, 6 | 6.76E-05 | -2.06 |
| ***Serpine1*** | serine (or cysteine) peptidase inhibitor, clade E, member 1 | 1.42E-04 | 2.39 |
| ***Serping1*** | serine (or cysteine) peptidase inhibitor, clade G, member 1 | 1.98E-04 | 2.16 |
| ***Hp*** | haptoglobin | 2.37E-04 | 2.16 |
| ***Serpini1*** | serine (or cysteine) peptidase inhibitor, clade I, member 1 | 2.57E-04 | -2.87 |
| ***Tfdp1*** | transcription factor Dp 1 | 2.79E-04 | -2.16 |
| ***Itih3*** | inter-alpha trypsin inhibitor, heavy chain 3 | 3.00E-04 | -3.59 |
| ***Dig1*** | discs, large homolog 1 (Drosophila) | 4.66E-04 | -2.07 |
| ***Usp2*** | ubiquitin specific peptidase 2 | 5.64E-04 | -2.15 |
| ***Cst7*** | cystatin F (leukocystatin) | 7.19E-04 | 2.19 |
| ***Psmb9*** | proteasome (prosome, macropain) subunit, beta type 9 (large | 1.37E-03 | 2.34 |
| | multifunctional peptidase 2) | | |
| ***Timp4*** | tissue inhibitor of metalloproteinase 4 | 3.36E-03 | -2.59 |
| ***Mmp3*** | matrix metallopeptidase 3 | 8.06E-03 | 3.25 |

| **f. Stroma-derived genes that show stage-specific expression in bone metastasis** | | | |
|---|---|---|---|
| **Symbol** | Name | *P* Value | Fold Change Late vs Early |
| ***Ctse*** | cathepsin E | 7.64E-16 | -12.2 |
| ***Xpo7*** | exportin 7 | 8.31E-12 | -3.65 |
| ***Fryl*** | furry homolog-like (Drosophila) | 9.72E-12 | -2.61 |
| ***Atg4a*** | autophagy related 4A, cysteine peptidase | 1.94E-10 | -3.24 |
| ***Psme3*** | proteaseome (prosome, macropain) 28 subunit, 3 | 2.42E-10 | -3.36 |
| ***Mcpt8*** | mast cell protease 8 | 4.31E-10 | -2.64 |
| ***Rfc1*** | replication factor C (activator 1) 1 | 3.03E-09 | -2.08 |
| ***Ube2e3*** | ubiquitin-conjugating enzyme E2E 3 | 8.00E-09 | 3.22 |
| ***Adamts4*** | a disintegrin-like and metallopeptidase (reprolysin type) with thrombospondin type 1 motif, 4 | 8.50E-09 | 2.45 |
| ***Metap2*** | methionine aminopeptidase 2 | 8.82E-09 | -3.30 |
| ***Dpp4*** | dipeptidylpeptidase 4 | 2.55E-08 | -2.86 |
| ***Ube2r2*** | ubiquitin-conjugating enzyme E2R 2 | 2.62E-08 | -2.45 |
| ***Ctbp1*** | C-terminal binding protein 1 | 3.64E-08 | -2.31 |
| ***Serpina3n*** | serine (or cysteine) peptidase inhibitor, clade A, member 3N | 3.93E-08 | 6.16 |
| ***Casp2*** | caspase 2 | 5.69E-08 | -2.06 |
| ***Psmd13*** | proteasome (prosome, macropain) 26S subunit, non-ATPase, 13 | 5.13E-07 | -2.15 |
| ***Psma1*** | proteasome (prosome, macropain) subunit, alpha type 1 | 5.71E-07 | -2.17 |
| ***Ctss*** | cathepsin S | 9.66E-07 | 2.67 |
| ***Hp1bp3*** | heterochromatin protein 1, binding protein 3 | 1.63E-06 | -2.41 |
| ***Cdv3*** | carnitine deficiency-associated gene expressed in ventricle 3 | 1.95E-06 | -2.29 |
| ***Usp25*** | ubiquitin specific peptidase 25 | 3.03E-06 | -2.18 |
| ***Adamts12*** | a disintegrin-like and metallopeptidase (reprolysin type) with thrombospondin type 1 motif, 12 | 3.10E-06 | 2.30 |
| ***Stfa3*** | stefin A3 | 9.41E-06 | 2.59 |
| ***Ctsf*** | cathepsin F | 1.07E-05 | -2.00 |
| ***Anapc2*** | anaphase promoting complex subunit 2 | 1.19E-05 | -2.06 |
| ***Prtn3*** | proteinase 3 | 1.29E-05 | -2.98 |
| ***Phb2*** | prohibitin 2 | 1.52E-05 | -2.07 |
| ***Timp1*** | tissue inhibitor of metalloproteinase 1 | 2.41E-05 | 2.99 |
| ***Mmp8*** | matrix metallopeptidase 8 | 4.73E-05 | -2.90 |
| ***Rps27*** | ribosomal protein S27 | 5.11E-05 | -2.22 |
| ***Eif5*** | eukaryotic translation initiation factor 5 | 5.77E-05 | -2.12 |
| ***Capn3*** | calpain 3 | 8.77E-05 | -2.53 |
| ***Serpine1*** | serine (or cysteine) peptidase inhibitor, clade E, member 1 | 1.12E-04 | 2.43 |
| ***Plau*** | plasminogen activator, urokinase | 3.09E-04 | 2.66 |
| ***Ube2b*** | ubiquitin-conjugating enzyme E2B | 3.31E-04 | -2.02 |
| ***Mmp3*** | matrix metallopeptidase 3 | 3.47E-04 | 5.54 |
| ***Psmd11*** | proteasome (prosome, macropain) 26S subunit, non-ATPase, 11 | 3.77E-04 | -2.35 |
| ***Elane*** | elastase, neutrophil expressed | 4.93E-04 | -2.81 |
| ***Serping1*** | serine (or cysteine) peptidase inhibitor, clade G, member 1 | 4.95E-04 | 2.02 |
| ***Tug1*** | taurine upregulated gene 1 | 5.16E-04 | -2.04 |
| ***Ctsg*** | cathepsin G | 5.81E-04 | -2.52 |
| ***Mmp13*** | matrix metallopeptidase 13 | 9.98E-04 | 3.17 |
| ***Igfbp7*** | insulin-like growth factor binding protein 7 | 1.03E-03 | 2.06 |
| ***Slpi*** | secretory leukocyte peptidase inhibitor | 2.89E-03 | -2.13 |
| | | | |

| **g. Stroma-derived genes that show stage-specific expression in lung metastasis** | | | |
|---|---|---|---|
| **Symbol** | Name | *P* Value | Fold Change Late vs Early |
| ***Hp*** | haptoglobin | 5.66E-06 | -2.85 |

**Table 2: Tumor-derived stage-specific genes that are associated with metastasis-free survival (MFS) in patient datasets.**

| **a.** Tumor-derived genes that change by stage in brain metastases and are associated with brain MFS. | | | | |
|---|---|---|---|---|
| **Gene** | **Hazard Ratio** | **95% CI** | **Nominal *P* Value** | **Metastatic Site Association** |
| ***TPSG1*** | 0.1282 | (0.01678 - 0.9793) | 0.04769 | Brain and Lung |
| ***HNRNPC*** | 0.4147 | (0.2335 - 0.7366) | 0.002673 | Brain only |
| ***SEPT2*** | 0.5287 | (0.299 - 0.9349) | 0.02842 | Brain only |
| ***SERPINB3*** | 1.268 | (1.066 - 1.507) | 0.007274 | Brain and Lung |
| ***PI3*** | 1.295 | (1.062 - 1.58) | 0.01059 | Brain and Lung |
| ***SPOCK2*** | 1.362 | (1.045 - 1.773) | 0.02211 | Brain only |
| ***PSMB6*** | 1.377 | (1 - 1.894) | 0.04968 | Brain only |
| ***PRSS22*** | 1.396 | (1.015 - 1.921) | 0.04036 | Brain only |
| ***CTSS*** | 1.411 | (1.053 - 1.889) | 0.02094 | Brain only |
| ***KLK10*** | 1.414 | (1.018 - 1.964) | 0.03896 | Brain only |
| ***GZMK*** | 1.483 | (1.127 - 1.952) | 0.004925 | Brain only |
| ***ADAMDEC1*** | 1.494 | (1.064 - 2.097) | 0.02037 | Brain and Lung |
| ***ELANE*** | 1.495 | (1.041 - 2.147) | 0.02947 | Brain only |
| ***ILF2*** | 1.516 | (1.009 - 2.278) | 0.04531 | Brain and Lung |
| ***PSMD11*** | 1.553 | (1.155 - 2.09) | 0.003629 | Brain and Bone |
| ***PSMB4*** | 1.584 | (1.074 - 2.334) | 0.02021 | Brain and Lung |
| ***S100A10*** | 1.618 | (1.016 - 2.577) | 0.04285 | Brain and Lung |
| ***APP*** | 1.624 | (1.108 - 2.381) | 0.01291 | Brain and Lung |
| ***COX4I1*** | 1.661 | (1.015 - 2.717) | 0.04345 | Brain only |
| ***CTSC*** | 1.671 | (1.131 - 2.469) | 0.009991 | Brain and Lung |
| ***CTSL1*** | 1.69 | (1.101 - 2.596) | 0.0165 | Brain and Lung |
| ***TIMP2*** | 1.725 | (1.161 - 2.563) | 0.007004 | Brain only |
| ***CANX*** | 1.729 | (1.148 - 2.605) | 0.0088 | Brain and Lung |
| ***SLPI*** | 1.747 | (1.21 - 2.523) | 0.002909 | Brain, Bone, Lung |
| ***ANXA5*** | 1.852 | (1.386 - 2.475) | 3.09E-05 | Brain and Lung |
| ***PSMD2*** | 1.871 | (1.209 - 2.896) | 0.00491 | Brain and Lung |
| ***CTSB*** | 2.249 | (1.476 - 3.425) | 0.0001605 | Brain and Lung |

| **b.** Tumor-derived genes that change by stage in bone metastases and are associated with bone MFS. | | | | |
|---|---|---|---|---|
| Gene | Hazard Ratio | 95% CI | Nominal *P* Value | Metastatic Site Association |
| ***EIF3F*** | 0.6903 | (0.5558 - 0.8574) | 0.000803 | Bone only |
| ***RPS6*** | 0.7257 | (0.5795 - 0.9088) | 0.005221 | Bone only |
| ***GZMB*** | 0.7338 | (0.5516 - 0.9761) | 0.03352 | Bone only |
| ***RPS13*** | 0.7342 | (0.6062 - 0.8893) | 0.001577 | Bone only |
| ***RPS10*** | 0.7387 | (0.603 - 0.9049) | 0.00345 | Bone only |
| ***PSME1*** | 0.7481 | (0.6063 - 0.923) | 0.006784 | Bone and Lung |
| ***RPL21*** | 0.751 | (0.6193 - 0.9107) | 0.003607 | Bone only |
| ***RPL30*** | 0.7514 | (0.614 - 0.9196) | 0.005541 | Bone only |
| ***OAZ1*** | 0.7659 | (0.6241 - 0.9399) | 0.01065 | Bone only |
| ***SERPINF2*** | 0.7679 | (0.6105 - 0.9659) | 0.02406 | Bone only |
| ***RPL27*** | 0.7689 | (0.632 - 0.9354) | 0.008605 | Bone only |
| ***PRTN3*** | 0.7774 | (0.6358 - 0.9506) | 0.01414 | Bone only |
| ***RPS5*** | 0.779 | (0.6476 - 0.9371) | 0.008072 | Bone only |
| ***F2*** | 0.7885 | (0.6475 - 0.9602) | 0.01807 | Bone only |
| ***RPL14*** | 0.7888 | (0.6483 - 0.9596) | 0.0177 | Bone only |
| ***PSMD13*** | 0.7955 | (0.6431 - 0.984) | 0.035 | Bone only |
| ***RPL28*** | 0.7966 | (0.6598 - 0.9618) | 0.01805 | Bone only |
| ***RPS27A*** | 0.7985 | (0.6641 - 0.96) | 0.01665 | Bone only |
| ***TIMP1*** | 0.7991 | (0.649 - 0.984) | 0.0347 | Bone only |
| ***RPS11*** | 0.8005 | (0.6624 - 0.9674) | 0.02131 | Bone only |
| ***USP4*** | 0.8043 | (0.6536 - 0.9897) | 0.03963 | Bone only |
| ***RPS24*** | 0.8129 | (0.6692 - 0.9874) | 0.03683 | Bone only |
| ***CELA2B*** | 0.8144 | (0.6776 - 0.9788) | 0.02863 | Bone only |
| ***RPL11*** | 0.8228 | (0.6801 - 0.9956) | 0.04491 | Bone only |
| ***MME*** | 1.157 | (1.025 - 1.306) | 0.01873 | Bone and Lung |
| ***PSMB3*** | 1.174 | (1.001 - 1.377) | 0.04863 | Bone and Lung |
| ***SNRNP200*** | 1.197 | (1.003 - 1.429) | 0.04677 | Bone only |
| ***SLPI*** | 1.245 | (1.004 - 1.543) | 0.046 | Brain, Bone, Lung |
| ***PSMD10*** | 1.245 | (1.024 - 1.514) | 0.02791 | Bone and Lung |
| ***PSMD11*** | 1.252 | (1.057 - 1.483) | 0.009225 | Brain and Bone |

| **c.** Tumor-derived genes that change by stage in lung metastases and are associated with lung MFS. | | | | |
|---|---|---|---|---|
| **Gene** | **Ratio** | **95% CI** | **Nominal *P* Value** | **Metastatic Site Association** |
| ***SPINK4*** | 0.2861 | (0.1325 - 0.6176) | 0.001437 | Lung only |
| ***ANXA9*** | 0.4323 | (0.2428 - 0.7699) | 0.004397 | Lung only |
| ***PLAT*** | 0.4627 | (0.2329 - 0.9195) | 0.02783 | Lung only |
| ***MMP24*** | 0.4685 | (0.2883 - 0.7612) | 0.002203 | Lung only |
| ***CST3*** | 0.4736 | (0.2974 - 0.7542) | 0.001644 | Lung only |
| ***EEF2*** | 0.4758 | (0.3223 - 0.7023) | 0.0001853 | Lung only |
| ***F7*** | 0.5294 | (0.3196 - 0.8768) | 0.01349 | Lung only |
| ***F10*** | 0.5902 | (0.4 - 0.8709) | 0.007896 | Lung only |
| ***RPL9*** | 0.5994 | (0.4178 - 0.8598) | 0.005432 | Lung only |
| ***TPSG1*** | 0.6108 | (0.3745 - 0.9961) | 0.04818 | Brain and Lung |
| ***PRSS23*** | 0.6253 | (0.3932 - 0.9943) | 0.04723 | Lung only |
| ***MMP26*** | 0.6277 | (0.4384 - 0.8989) | 0.01102 | Lung only |
| ***HTRA1*** | 0.6382 | (0.4541 - 0.897) | 0.009704 | Lung only |
| ***PSME1*** | 0.705 | (0.5073 - 0.9798) | 0.03739 | Bone and Lung |
| ***MME*** | 1.198 | (1.032 - 1.39) | 0.01759 | Bone and Lung |
| ***SERPINB3*** | 1.232 | (1.095 - 1.387) | 0.0005333 | Brain and Lung |
| ***PSMB3*** | 1.254 | (1.028 - 1.529) | 0.02562 | Bone and Lung |
| ***PI3*** | 1.257 | (1.1 - 1.436) | 0.0007963 | Brain and Lung |
| ***PSMA7*** | 1.27 | (1.07 - 1.507) | 0.006299 | Lung only |
| ***TMPRSS5*** | 1.275 | (1.039 - 1.564) | 0.02005 | Lung only |
| ***F12*** | 1.287 | (1.057 - 1.565) | 0.01181 | Lung only |
| ***PSMA6*** | 1.289 | (1.025 - 1.622) | 0.02977 | Lung only |
| ***SPINK2*** | 1.295 | (1.039 - 1.613) | 0.0215 | Lung only |
| ***PSMA3*** | 1.304 | (1.014 - 1.678) | 0.03883 | Lung only |
| ***ADAM9*** | 1.306 | (1.028 - 1.658) | 0.02868 | Lung only |
| ***PLAU*** | 1.309 | (1.033 - 1.659) | 0.02569 | Lung only |
| ***CAPN3*** | 1.316 | (1.016 - 1.703) | 0.03717 | Lung only |
| ***ZNF146*** | 1.317 | (1.088 - 1.593) | 0.004644 | Lung only |
| ***ANXA1*** | 1.342 | (1.085 - 1.659) | 0.006674 | Lung only |
| ***PSMC2*** | 1.343 | (1.043 - 1.729) | 0.02242 | Lung only |
| ***COPS7B*** | 1.347 | (1.013 - 1.792) | 0.04042 | Lung only |
| ***PSMB5*** | 1.354 | (1.094 - 1.676) | 0.005394 | Lung only |
| ***CTSB*** | 1.36 | (1.022 - 1.81) | 0.03485 | Brain and Lung |
| ***PSMD10*** | 1.362 | (1.017 - 1.824) | 0.03845 | Bone and Lung |
| ***PSMC3*** | 1.364 | (1.047 - 1.777) | 0.02142 | Lung only |
| ***ANXA3*** | 1.364 | (1.056 - 1.761) | 0.01725 | Lung only |
| ***PSMA4*** | 1.376 | (1.064 - 1.78) | 0.01499 | Lung only |
| ***ADAMDEC1*** | 1.387 | (1.12 - 1.718) | 0.002683 | Brain and Lung |
| ***USP1*** | 1.398 | (1.11 - 1.762) | 0.004469 | Lung only |
| ***PSMB4*** | 1.405 | (1.098 - 1.799) | 0.006917 | Brain and Lung |
| ***KIFAP3*** | 1.427 | (1.057 - 1.927) | 0.02015 | Lung only |
| ***PSMD4*** | 1.463 | (1.085 - 1.974) | 0.01266 | Lung only |
| ***HSP90AB1*** | 1.487 | (1.139 - 1.942) | 0.003554 | Lung only |
| ***PCSK1N*** | 1.495 | (1.148 - 1.946) | 0.002805 | Lung only |
| ***APP*** | 1.517 | (1.18 - 1.95) | 0.001149 | Brain and Lung |
| ***CANX*** | 1.547 | (1.193 - 2.007) | 0.001015 | Brain and Lung |
| ***CSTB*** | 1.555 | (1.288 - 1.876) | 4.18E-06 | Lung only |
| ***PSMB7*** | 1.556 | (1.206 - 2.008) | 0.0006705 | Lung only |
| ***PSMC1*** | 1.569 | (1.228 - 2.005) | 0.0003191 | Lung only |
| ***ILF2*** | 1.595 | (1.261 - 2.017) | 9.66E-05 | Brain and Lung |
| ***ANXA5*** | 1.615 | (1.32 - 1.976) | 3.19E-06 | Brain and Lung |
| ***PSMD1*** | 1.653 | (1.246 - 2.194) | 0.0004984 | Lung only |
| ***CTSC*** | 1.661 | (1.296 - 2.128) | 6.10E-05 | Brain and Lung |
| ***GDI2*** | 1.664 | (1.312 - 2.111) | 2.75E-05 | Lung only |
| ***CTSL1*** | 1.695 | (1.335 - 2.152) | 1.48E-05 | Brain and Lung |
| ***SERPINE2*** | 1.724 | (1.395 - 2.131) | 4.69E-07 | Lung only |
| ***SLPI*** | 1.757 | (1.411 - 2.19) | 5.02E-07 | Brain, Bone, Lung |
| ***PSMD2*** | 1.793 | (1.384 - 2.324) | 9.98E-06 | Brain and Lung |
| ***S100A10*** | 1.937 | (1.517 - 2.474) | 1.20E-07 | Brain and Lung |

| **d.** Summary of differentially expressed genes (DEGs) and their association with MFS at their respective site. **Association with Brain MFS:** Hypergeometric *P* value < 0.766 | | | | |
|---|---|---|---|---|
| **Gene Set** | **Total Genes** | **Genes on GSE12776 array** | **Non Significant** | **Significant** |
| HuMu Genes | 523 | 476 | 414 | 62 |
| Brain DEG | 242 | 226 | 199 | 27 |

| **Association with Bone MFS:** Hypergeometric *P* value < 3.68x10⁻³ | | | | |
|---|---|---|---|---|
| **Genes Set** | **Total Genes** | **Genes on GSE12776 array** | **Non Significant** | **Significant** |
| HuMu Genes | 523 | 476 | 429 | 47 |
| Bone DEG | 241 | 221 | 191 | 30 |

| **Association with Lung MFS:** Hypergeometric *P* value < 0.728 | | | | |
|---|---|---|---|---|
| **Gene Set** | **Total Genes** | **Genes on GSE12776 array** | **Non Significant** | **Significant** |
| HuMu Genes | 523 | 476 | 346 | 130 |
| Lung DEG | 245 | 225 | 166 | 59 |

| **e.** Summary of differentially expressed genes (DEGs) in the bone, and their association with brain and lung MFS. This demonstrates that the set of genes differentially expressed in the bone is enriched for genes associated with bone MFS only, and not genes associated with brain or lung MFS. | | | | |
|---|---|---|---|---|
| **Association with Brain MFS:** Hypergeometric *P* value < 0.82 | | | | |
| **Gene Set** | **Total Genes** | **Genes on GSE12776 array** | **Non Significant** | **Significant** |
| HuMu Genes | 523 | 476 | 414 | 62 |
| Bone DEG | 241 | 221 | 195 | 26 |

| **Association with Lung MFS:** Hypergeometric *P* value < 0.26 | | | | |
|---|---|---|---|---|
| **Gene Set** | **Total Genes** | **Genes on GSE12776 array** | **Non Significant** | **Significant** |
| HuMu Genes | 523 | 476 | 346 | 130 |
| Bone DEG | 241 | 221 | 157 | 64 |

**Supplementary Table 3: Summary of patient information of primary breast cancer and brain metastases samples**

| | Primary breast cancer | | | | Brain metastasis | | | | |
|---|---|---|---|---|---|---|---|---|---|
| | Tumor grade / Histology | ER | PR | HER2 | Tumor grade / Histology | ER | PR | HER2 | mfs (months) |
| Patient 1 | Poorly differentiated | Pos | Pos | Pos | Poorly differentiated | N/A | N/A | N/A | 47.06 |
| Patient 2 | Moderately differentiated | Neg | N/A | Pos | Grade not reported | Neg | Neg | Neg | 50.07 |
| Patient 3 | Poorly differentiated | Neg | Neg | Pos | Grade not reported | Neg | Neg | Pos | 32.25 |
| Patient 4 | Poorly differentiated | Neg | Neg | Pos | Grade not reported | N/A | N/A | N/A | 41.23 |
| Patient 5 | Poorly differentiated | Neg | Neg | Neg | Poorly differentiated | N/A | N/A | N/A | 13.02 |
| Patient 6 | Poorly differentiated | Neg | Neg | Neg | Grade not reported | N/A | N/A | N/A | 114.25 |
| | | | | | | | | | |
| Patient 7 | Poorly differentiated | Pos | Pos | Pos | Poorly differentiated | Pos | Pos | Pos | 87.12 |
| Patient 8 | Poorly differentiated | Neg | Neg | Pos | Poorly differentiated | Pos | Neg | Pos | 46.10 |
| Patient 9 | Poorly differentiated | Neg | Neg | Pos | Grade not reported | Neg | Neg | Pos | 11.57 |
| Patient 10 | Poorly differentiated | Neg | Neg | Pos | Poorly differentiated | N/A | N/A | Pos | 36.10 |
| Patient 11 | Poorly differentiated | Neg | Neg | Neg | Poorly differentiated | N/A | N/A | N/A | 33.11 |
| Patient 12 | Poorly differentiated | Neg | Neg | Neg | Grade not reported | Neg | Neg | Neg | 40.66 |
| Patient 13 | Histologic Grade II/III | Neg | Neg | Neg | Poorly differentiated | N/A | N/A | N/A | 47.38 |

**Table 4: List of Taqman probes used for qPCR**

| **Taqman Probe** | **Species** | **Assay number** |
|---|---|---|
| *ADAM17* | Human | Hs01041913_m1 |
| *ADAM*21 | Human | Hs01652548_s1 |
| *Adamts4* | Mouse | Mm01295272_m1 |
| *Adamts12* | Mouse | Mm00615603_m1 |
| *B-2 micorglobulin* | Human | Hs99999907_m1 |
| *Casp2* | Mouse | Mm01160321_g1 |
| *CASP3* | Human | Hs00992526_g1 |
| *CD31* | Human | Hs01065279_m1 |
| *CDH5* | Human | Hs00901463_m1 |
| *CLDN3* | Human | Hs00265616_g1 |
| *CLDN5* | Human | Hs00533949_g1 |
| *Cst7* | Mouse | Mm00438349_m1 |
| *Ctse* | Mouse | Mm00456010_m1 |
| *Ctsh* | Mouse | Mm00514455_m1 |
| *Ctss* | Mouse | Mm00457902_m1 |
| *CTSS* | Human | Hs00175407_m1 |
| *DPP8* | Human | Hs01057169_m1 |
| *FAU* | Human | Hs00609871_m1 |
| *Htra1* | Mouse | Mm00479892_m1 |
| *JAM-A* | Human | Hs00170991_m1 |
| *JAM-B* | Human | Hs00221894_m1 |
| *JAM-C* | Human | Hs00230289_m1 |
| *MMP11* | Mouse | Mm01173385_g1 |
| *MMP13* | Mouse | Mm00439491_m1 |
| *MMP24* | Human | Hs01084640_m1 |
| *OCL* | Human | Hs00170162_m1 |
| *Pcsk1n* | Mouse | Mm00457410_m1 |
| *Serpina3n* | Mouse | Mm00776439_m1 |
| *Serpinit* | Mouse | Mm00434740_m1 |
| *SERPINE2* | Human | Hs00385730_m1 |
| *Tïmp1* | Mouse | Mm00441818_m1 |
| *TIMP2* | Human | Hs01099731_g1 |
| *Ubiquitin c* | Mouse | Mm01201237_m1 |
| *18S* | Human/Mouse | Hs999999901_s1 |
| *18S* | Human/Mouse | Hs999999901_s1 |

**Table 5: List of antibodies used for immunofluorescence (IF) staining and Western blotting (WB)**

| **Antibody** | **Reactivity** | **Clone** | **Vendor** | **Dilution** |
|---|---|---|---|---|
| Rabbit anti-ADAM17 | Human, Rat | -- | Abcam | 1:100 |
| Rabbit anti-Sace-1 | Human, Mouse, Rat | -- | Abcam | 1:100 |
| Mouse anti-CD68 | Human | PG-M1 | Dako | Pre-diluted |
| Rat anti-CD68 | Mouse | FA-11 | Serotes | 1:1,000 |
| Gost and-CD31 | Mouse | -- | R&D Systems | 1:100 |
| Rat anti-CD34 | Mouse | Mec14.7 | Biotegend | 1:200 |
| Goat anti-cadherin 5 (Cdh5) | Mouse | -- | R&D Systems | 1:500 |
| Rabbit anti-Claudin 3 (CL3) | Human, Mouse | -- | Invitrogen | 1:1,000 |
| Rabbit anti-Claudin 5 (CL5) | Human, Mouse | -- | Invitrogen | 1:1,000 |
| Goat anti-cathepsin E (CTSE) | Human, Mouse | -- | R&D Systems | 1:100 |
| Goat anti-cathepsin S (CTSS) | Human, Mouse | -- | R&D Systems | 1:100 (IF) |
| | | | | 1:500 (WM) |
| Goat anti-cathepsin Z (Ctsz) | Mouse | -- | R&D Systems | 1:100 |
| Rabbit anti-cystatin A (CSTA) | Human, Mouse | -- | Abcams | |
| Mouse anti-cytokeratin (CK) | Human | AE1/AE3 | Dako | |
| Rabbot anti-glial fibrillary acidic proteïn (GFAP) | Human, Mouse, Rat, Cow | -- | Dako | 1:300 |
| Chicken anti-GFP | Jelly fish | -- | Abcam | 1:1,000 |
| Rabbit anti-lba 1 | Hisrasrs, Mouse, Rat | Mouse | Wako | 1:1,000 |
| Goat anti-IgG1 (FC) | Human | -- | Biodesign | 1:10,000 |
| Goat anti-Junctional Adhesion Molecule A (Jam-A) | Mouse | -- | R&D Systems | 1:500 |
| Goat anti-Junctional Adhesion Molecule B (Jam-B) | Mouse | -- | R&D Systems | 1:500 (WB) |
| Rat anti-Junctional Adhesion Molecule B (Jam-B) | Mouse | CRAM-18 F26 | Pierce | 1:100 (IF) |
| Goat anti-Junctional Adhesion Molecule B (JAM-B) | Human | -- | R&D Systems | 1:500 |
| Goat anti-Junctional Adhesion Molecule C (Jam-C) | Mouse | -- | R&D Systems | 1:500 |
| Rabbit anti-K167 | Human | SP6 | Vector | 1:500 |
| Rabbit anti-Matrix Metallo Proteinase 24 (MMP24) | Human, Rat | -- | Abcam | |
| Rabbit anti-Occludin (OCL) | Human, Mouse | -- | Invitrogen | 1:1,000 |
| Rabbit anti-Serpina3n | Mouse | 13H5 | Custom MSKOC | 1:10 |
| Rabbit anti-SERPINB10 | Human | -- | Abcam | 1:100 |
| Rabbit and-SERPINE2 | Human, Mouse | -- | Abcam | 1:100 |
| Rabbit anti-Timp1 | Mouse | -- | Abcam | 1:100 |
| Rabbit anti-TIMP2 | Human, Goat | -- | Abcam | 1:100 |

**Table 6: List of recombinant proteins used for in vitro cleavage assays**

| **Recombinant protein** | **Species** | **Input (ng / reaction)** | **Vendor** |
|---|---|---|---|
| Cathepsin S (CTSS) | Human | 2.1 | R&8 Systems |
| Cadhesin 5 (Cdh5) | Mouse | 17.5 | R&D Systems |
| CD31 | Mouse | 17.5 | R&D Systems |
| Claudin 3 (CL3) | Human | 15 | Novus |
| Claudin 5 (CL5) | Human | 30 | Novus |
| Jam-A | Mouse | 17.5 | R&D Systems |
| Jam-B | Mouse | 17.5 | R&D Systems |
| Jam-C | Mouse | 17.5 | R&D Systems |
| Occludin (OCL) | Human | 7.5 | Novus |

### References

1. Quail, D.F. & Joyce, J.A. Microenvironmental regulation of tumor progression and metastasis. Nat Med 19, 1423-1437 (2013).
2. Nguyen, D.X., Bos, P.D. & Massague, J. Metastasis: from dissemination to organ-specific colonization. Nat Rev Cancer 9, 274-284 (2009).
3. Goss, P.E. & Chambers, A.F. Does tumour dormancy offer a therapeutic target? Nat Rev Cancer 10, 871-877 (2010).
4. Affara, N.I., Andreu, P. & Coussens, L.M. Delineating protease functions during cancer development. Methods Mol Biol 539, 1-32 (2009).
5. Mason, S.D. & Joyce, J.A. Proteolytic networks in cancer. Trends Cell Biol 21, 228-237 (2011).
6. Gocheva, V. et al. Distinct roles for cysteine cathepsin genes in multistage tumorigenesis. Genes Dev 20, 543-556 (2006).
7. Murphy, G. The ADAMs: signalling scissors in the tumour microenvironment. Nat Rev Cancer 8, 929-941 (2008).
8. Butler, G.S. & Overall, C.M. Proteomic identification of multitasking proteins in unexpected locations complicates drug targeting. Nat Rev Drug Discov 8, 935-948 (2009).
9. Lopez-Otin, C. & Hunter, T. The regulatory crosstalk between kinases and proteases in cancer. Nat Rev Cancer 10, 278-292 (2010).
10. Kessenbrock, K., Plaks, V. & Werb, Z. Matrix metalloproteinases: regulators of the tumor microenvironment. Cell 141, 52-67 (2010).
11. Allinen, M. et al. Molecular characterization of the tumor microenvironment in breast cancer. Cancer Cell 6, 17-32 (2004).
12. Finak, G. et al. Stromal gene expression predicts clinical outcome in breast cancer. Nat Med 14, 518-527 (2008).
13. Ojalvo, L.S., Whittaker, C.A., Condeelis, J.S. & Pollard, J.W. Gene expression analysis of macrophages that facilitate tumor invasion supports a role for Wnt-signaling in mediating their activity in primary mammary tumors. J Immunol 184, 702-712 (2010).
14. Squadrito, M.L., Etzrodt, M., De Palma, M. & Pittet, M.J. MicroRNA-mediated control of macrophages and its implications for cancer. Trends Immunol (2013).
15. Seaman, S. et al. Genes that distinguish physiological and pathological angiogenesis. Cancer Cell 11, 539-554 (2007).
16. Erez, N., Truitt, M., Olson, P., Arron, S.T. & Hanahan, D. Cancer-associated fibroblasts are activated in incipient neoplasia to orchestrate tumor-promoting inflammation in an NF-kappaB-dependent manner. Cancer Cell 17, 135-147 (2010).
17. Aprelikova, O. & Green, J.E. MicroRNA regulation in cancer-associated fibroblasts. Cancer Immunol Immunother 61, 231-237 (2012).
18. Pucci, F. et al. A distinguishing gene signature shared by tumor-infiltrating Tie2-expressing monocytes, blood "resident" monocytes, and embryonic macrophages suggests common functions and developmental relationships. Blood 114, 901-914 (2009).
19. Katz, A.M. et al. Astrocyte-specific expression patterns associated with the PDGF-induced glioma microenvironment. PLoS One 7, e32453 (2012).
20. Paget, S. The distribution of secondary growths in cancer of the breast. 1889. Cancer Metastasis Rev 8, 98-101 (1989).
21. Disibio, G. & French, S.W. Metastatic patterns of cancers: results from a large autopsy study. Arch Pathol Lab Med 132, 931-939 (2008).
22. Lee, Y.T. Breast carcinoma: pattern of metastasis at autopsy. J Surg Oncol 23, 175-180 (1983).
23. Bos, P.D. et al. Genes that mediate breast cancer metastasis to the brain. Nature 459, 1005-1009 (2009).
24. Kang, Y. et al. A multigenic program mediating breast cancer metastasis to bone. Cancer Cell 3, 537-549 (2003).
25. Minn, A.J. et al. Genes that mediate breast cancer metastasis to lung. Nature 436, 518-524 (2005).
26. Schwartz, D.R. et al. Hu/Mu Protln oligonucleotide microarray: dual-species array for profiling protease and protease inhibitor gene expression in tumors and their microenvironment. Mol Cancer Res 5, 443-454 (2007).
27. Ponomarev, V. et al. A novel triple-modality reporter gene for whole-body fluorescent, bioluminescent, and nuclear noninvasive imaging. Eur J Nucl Med Mol Imaging 31, 740-751 (2004).
28. Shi, G.P. et al. Cathepsin S required for normal MHC class II peptide loading and germinal center development. Immunity 10, 197-206 (1999).
29. Lorger, M. & Felding-Habermann, B. Capturing changes in the brain microenvironment during initial steps of breast cancer brain metastasis. Am J Pathol 176, 2958-2971 (2010).
30. Small, D.M. et al. Cathepsin S from both tumor and tumor-associated cells promote cancer growth and neovascularization. Int J Cancer 133, 2102-2112 (2013).
31. Abbott, N.J., Ronnback, L. & Hansson, E. Astrocyte-endothelial interactions at the blood-brain barrier. Nat Rev Neurosci 7, 41-53 (2006).
32. Eugenin, E.A. & Berman, J.W. Chemokine-dependent mechanisms of leukocyte trafficking across a model of the blood-brain barrier. Methods 29, 351-361 (2003).
33. Jia, W., Martin, T.A., Zhang, G. & Jiang, W.G. Junctional adhesion molecules in cerebral endothelial tight junction and brain metastasis. Anticancer Res 33, 2353-2359 (2013).
34. Liu, W.Y., Wang, Z.B., Zhang, L.C., Wei, X. & Li, L. Tight junction in blood-brain barrier: an overview of structure, regulation, and regulator substances. CNS Neurosci Ther 18, 609-615 (2012).
35. Gallagher, F.A. et al. Magnetic resonance imaging of pH in vivo using hyperpolarized 13C-labelled bicarbonate. Nature 453, 940-943 (2008).
36. Kirschke, H., Wiederanders, B., Bromme, D. & Rinne, A. Cathepsin S from bovine spleen. Purification, distribution, intracellular localization and action on proteins. Biochem J 264, 467-473 (1989).
37. Nolan, D.J. et al. Molecular signatures of tissue-specific microvascular endothelial cell heterogeneity in organ maintenance and regeneration. Dev Cell 26, 204-219 (2013).
38. Choe, Y. et al. Substrate profiling of cysteine proteases using a combinatorial peptide library identifies functionally unique specificities. J Biol Chem 281, 12824-12832 (2006).
39. Oliveira, M. et al. Improvement of cathepsin S detection using a designed FRET peptide based on putative natural substrates. Peptides 31, 562-567 (2010).
40. Biniossek, M.L., Nagler, D.K., Becker-Pauly, C. & Schilling, O. Proteomic identification of protease cleavage sites characterizes prime and non-prime specificity of cysteine cathepsins B, L, and S. J Proteome Res 10, 5363-5373 (2011).
41. Oskarsson, T. et al. Breast cancer cells produce tenascin C as a metastatic niche component to colonize the lungs. Nat Med 17, 867-874 (2011).
42. Chapman, H.A. Endosomal proteases in antigen presentation. Curr Opin Immunol 18, 78-84 (2006).
43. Gupta, S., Singh, R.K., Dastidar, S. & Ray, A. Cysteine cathepsin S as an immunomodulatory target: present and future trends. Expert Opin Ther Targets 12, 291-299 (2008).
44. Madsen, C.D. & Sahai, E. Cancer dissemination--lessons from leukocytes. Dev Cell 19, 13-26 (2010).
45. Steeg, P.S., Camphausen, K.A. & Smith, Q.R. Brain metastases as preventive and therapeutic targets. Nat Rev Cancer 11, 352-363 (2011).
46. Eichler, A.F. et al. The biology of brain metastases-translation to new therapies. Nat Rev Clin Oncol 8, 344-356 (2011).
47. Park, E.S. et al. Cross-species hybridization of microarrays for studying tumor transcriptome of brain metastasis. Proc Natl Acad Sci U S A 108, 17456-17461 (2011).
48. Turk, B. Targeting proteases: successes, failures and future prospects. Nat Rev Drug Discov 5, 785-799 (2006).
49. Palermo, C. & Joyce, J.A. Cysteine cathepsin proteases as pharmacological targets in cancer. Trends Pharmacol Sci 29, 22-28 (2008).
50. Drag, M. & Salvesen, G.S. Emerging principles in protease-based drug discovery. Nat Rev Drug Discov 9, 690-701 (2010).
51. Fingleton, B. Matrix metalloproteinases as valid clinical targets. Curr Pharm Des 13, 333-346 (2007).
52. Seidah, N.G. & Prat, A. The biology and therapeutic targeting of the proprotein convertases. Nat Rev Drug Discov 11, 367-383 (2012).
53. Guy, C.T., Cardiff, R.D. & Muller, W.J. Induction of mammary tumors by expression of polyomavirus middle T oncogene: a transgenic mouse model for metastatic disease. Mol Cell Biol 12, 954-961 (1992).
54. Shree, T. et al. Macrophages and cathepsin proteases blunt chemotherapeutic response in breast cancer. Genes Dev 25, 2465-2479 (2011).
55. Gocheva, V. et al. IL-4 induces cathepsin protease activity in tumor-associated macrophages to promote cancer growth and invasion. Genes Dev 24, 241-255 (2010).

## Claims

1. A method of determining the risk that a subject with breast cancer will develop metastasis of said cancer to the brain, bone and/or lung, said method comprising:
(a) detecting in a sample from the subject the level of expression of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX411, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, CSTB, PSMB7, PSMC1, PSMD1, GDI2, SERPINE2, TPSG1, PSMD2* and *PSME1;* and
(b)
(i) predicting that the subject will develop metastasis to the brain, bone and lung if expression of *SLPI* is increased over control;
(ii) predicting that the subject will develop metastasis to brain and bone if expression of *PSMD11* is increased over control;
(iii) predicting that the subject will develop metastasis to brain and lung if expression of one or more of *SERPINB3, PI3, ADAMDEC1, ILF2, PSMB4, APP, S100A10, CTSC, CTSL1, CANX, ANXA5, PSMD2* and *CTSB* is increased over control, but will not develop metastasis to the brain if *TPSG1* is increased over control;
(iv) predicting that the subject will develop metastasis to bone and lung if expression of one or more of *MME, PSMB3,* and *PSMD10* is increased over control;
(v) predicting that the subject will develop metastasis to brain only if expression of one or more of *SPOCK2, PSMB6, PRSS22, CTSS, KLK10, GZMK, ELANE, COX4*/*1,* and *TIMP2* is increased over control, but will not develop metastasis to the brain if *HNRPNPC* and/or *SEPT2* is increased over control;
(vi) predicting that the subject will develop metastasis to bone if expression of SNRNP200 is increased over control, but will not develop metastasis to the bone if one or more of *EIF3F, RPS6, GZMB, RPS13, RPS10, RPL21, RPL30, OAZ1, SerpinF2, RPL27, PRTN3, RPS5, F2, RPL14, PSMD13, RPL28, RPS27A, TIMP1, RPS11, USP4, 1RPS24, CELA2B,* and *RPL11* is increased over control;
(vii) predicting that the subject will develop metastasis to lung only if expression of one or more of *PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, PSMB7, PSMC1, ILF2, PSMD1, GDI2,* and *SERPINE2* is increased over control, but will not develop metastasis to the lung if one or more of *SPINK4, ANXA9, PLAT, MMP24, CST3, EEF2, F7, F10, RPL9, PRSS23, MMP26,* and *HTRA1* is increased over control.

2. A method of determining the risk that a subject with breast cancer will develop metastasis of said cancer to the brain, said method comprising:
(a) determining in a sample from the subject a level of expression of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX4*/*1,CTSC, CTSL1, TlMP2, CANX, SLPI, ANXAS, PSMD2* and *CTSB;* and
(b) predicting that the subject will develop metastasis to the brain if expression of one or more of said genes is increased over control.

3. A method of determining the risk that a subject with breast cancer will develop metastasis of said cancer to the bone, said method comprising:
(a) determining in a sample from the subject the level of expression of genes *MME, PSMB3, SNRNP200, SLPI, PSMD10* and *PSMD11; and*
(b) predicting that the subject will develop metastasis to the bone if expression of one or more of said genes is increased over control.

4. A method of determining the risk that a subject with breast cancer will develop metastasis to the lung, said method comprising:
(a) determining in a sample from the subject the level of expression of genes *MME, SERPINB3, PSMB3, PI3, PSMA7, TMPRSS5, F12, PSMA6, SPINK2, PSMA3, ADAM9, PLAU, CAPN3, ZNF146, ANXA1, PSMC2, COPS7B, PSMB5, CTSB, PSMD10, PSMC3, ANXA3, PSMA4, ADAMDEC1, USP1, PSMB4, KIFAP3, PSMD4, HSP90AB1, PCSK1N, APP, CANX, CSTB, PSMB7, PSMC1, ILF2, ANXA5, PSMD1, CTSC, GDI2, CTSL1, SERPINE2, SLPI, PSMD2,* and *S100A10;* and
(b) predicting that the subject will develop metastasis to the lung if expression of one or more of said genes is increased over control.

5. The method of claim 1, wherein the sample comprises cells or tissue from a tumor from the subject.

6. A kit for determining in a sample from a breast cancer subject expression levels of genes indicative of metastasis of cancer in the subject to brain, bone or lung, the kit comprising (i) one or more components for determining the expression levels of said genes, wherein said one or more components are selected from the group consisting of: a DNA array chip, an oligonucleotide array chip, a protein array chip, an antibody, a plurality of probes; and (ii) a set of primers consisting of primers for genes SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2 and PSME1.

7. Use of the kit of claim 6 for determining the risk of metastasis of breast cancer to the brain in a breast cancer patient.

8. The kit of claim 6, further comprising one or more reagents for RNA extraction; one or more enzymes for syntheses of cDNA and cRNA; one or more reagents for hybridization for DNA chip, oligonucleotide chip, protein chip, western blot, probes, or primers; one or more reagents for binding of said antibodies to proteins indicative of recurrence of cancer; or DNA fragments of control genes

9. A set of primers consisting of at least one primer pair for each of genes *SERPINB3, PI3, SPOCK2, PSMB6, PRSS22, CTSS, KLK10, GZMK, ADAMDEC1, ELANE, ILF2, PSMD11*, *PSMB4, S100A10*, *APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* and *PSME1.*

10. An array consisting of a substrate or solid support and consisting of one probe for each of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELT2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* and *PSME1.*

11. A method of predicting the likelihood of metastasis-free survival (MFS) of a subject with breast cancer, the method comprising:
(a) detecting the level of expression of genes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTS8, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, TPSG1, PSMD2* and *PSME1* in a sample from the subject; and
(b) predicting decreased likelihood of metastasis-free survival if any of said genes is increased over control.

12. The method of claim 11, further comprising normalizing said expression levels to obtain a normalized expression level of said genes, wherein an increased normalized expression level of at least one of said genes indicates a decreased likelihood of metastasis-free survival without metastasis to the brain, bone or lung.

13. The method of any of claims 11 or 12, wherein said sample cancer cells or tissue from the subject with breast cancer.

## Patentansprüche

1. Verfahren zum Bestimmen des Risikos, dass ein Individuum mit Brustkrebs eine Metastasierung des Krebses im Gehirn, Knochen und/oder in der Lunge entwickeln wird, wobei das Verfahren Folgendes umfasst:
(a) Nachweisen in einer Probe des Individuums des Expressionsspiegels der Gene *SERPINB3, P13, SPOCK2, PSMB6, PRSS22, CTSS, KLK10, GZMK, ADAMDEC1, ELANE, ILF2, PSMD11, PSMB4, S100A10*, *APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, CSTB, PSMB7, PSMC1, PSMD1, GDI2, SERPINE2, TPSG1, PSMD2* und *PSME1;* und
(b)
(i) Vorhersagen, dass das Individuum eine Metastasierung im Gehirn, Knochen und in der Lunge entwickeln wird, wenn die Expression von *SLPI* gegenüber der Kontrolle erhöht ist;
(ii) Vorhersagen, dass das Individuum eine Metastasierung im Gehirn und Knochen entwickeln wird, wenn die Expression von *PSMD11* gegenüber der Kontrolle erhöht ist;
(iii) Vorhersagen, dass das Individuum eine Metastasierung im Gehirn und in der Lunge entwickeln wird, wenn die Expression von einem oder mehreren von *SERPINB3, PI3, ADAMDEC1, ILF2, PSMB4, APP, S100A10*, *CTSC, CTSL1, CANX, ANXA5, PSMD2* und *CTSB* gegenüber der Kontrolle erhöht ist, aber sich keine Metastasierung im Gehirn entwickeln wird, wenn *TPSG1* gegenüber der Kontrolle erhöht ist;
(iv) Vorhersagen, dass das Individuum eine Metastasierung im Knochen und in der Lunge entwickeln wird, wenn die Expression von einem oder mehreren von *MME, PSMB3* und *PSMD10* gegenüber der Kontrolle erhöht ist;
(v) Vorhersagen, dass das Individuum eine Metastasierung im Gehirn entwickeln wird, nur wenn die Expression von einem oder mehreren von *SPOCK2, PSMB6, PRSS22, CTSS, KLK10, GZMK, ELANE, COX4I1* und *TIMP2* gegenüber der Kontrolle erhöht ist, aber sich keine Metastasierung im Gehirn entwickeln wird, wenn *HNRPNPC* und/oder *SEPT2* gegenüber der Kontrolle erhöht ist;
(vi) Vorhersagen, dass das Individuum eine Metastasierung im Knochen entwickeln wird, wenn die Expression von SNRNP200 gegenüber der Kontrolle erhöht ist, aber sich keine Metastasierung im Knochen entwickeln wird, wenn eines oder mehrere von *EIF3F, RPS6, GZMB, RPS13*, *RPS10, RPL21, RPL30, OAZ1, SerpinF2, RPL27, PRTN3, RPS5, F2, RPL14, PSMD13, RPL28, RPS27A, TIMP1, RPS11, USP4, 1RPS24, CELA2B* und *RPL11* gegenüber der Kontrolle erhöht ist;
(vii) Vorhersagen, dass das Individuum eine Metastasierung in der Lunge entwickeln wird, nur wenn die Expression von einem oder mehreren von *PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, PSMB7, PSMC1, ILF2, PSMD1, GDI2,* und *SERPINE2* gegenüber der Kontrolle erhöht ist, aber sich keine Metastasierung in der Lunge entwickeln wird, wenn eines oder mehrere von *SPINK4, ANXA9, PLAT, MMP24, CST3, EEF2, F7, F10, RPL9, PRSS23, MMP26* und *HTRA1* gegenüber der Kontrolle erhöht ist.

2. Verfahren zum Bestimmen des Risikos, dass ein Individuum mit Brustkrebs eine Metastasierung des Krebses im Gehirn entwickeln wird, wobei das Verfahren Folgendes umfasst:
(a) Bestimmen in einer Probe des Individuums des Expressionsspiegels der Gene *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5*, *PSMD2* und *CTSB*; und
(b) Vorhersagen, dass das Individuum eine Metastasierung im Gehirn entwickeln wird, wenn die Expression von einem oder mehreren der Gene gegenüber der Kontrolle erhöht ist.

3. Verfahren zum Bestimmen des Risikos, dass ein Individuum mit Brustkrebs eine Metastasierung des Krebses im Knochen entwickeln wird, wobei das Verfahren Folgendes umfasst:
(a) Bestimmen in einer Probe des Individuums des Expressionsspiegels der Gene *MME, PSMB3, SNRNP200, SLPI, PSMD10* and *PSMD11;* und
(b) Vorhersagen, dass das Individuum eine Metastasierung im Knochen entwickeln wird, wenn die Expression von einem oder mehreren der Gene gegenüber der Kontrolle erhöht ist.

4. Verfahren zum Bestimmen des Risikos, dass ein Individuum mit Brustkrebs eine Metastasierung des Krebses in der Lunge entwickeln wird, wobei das Verfahren Folgendes umfasst:
(a) Bestimmen in einer Probe des Individuums des Expressionsspiegels der Gene *MME, SERPINB3, PSMB3, PI3, PSMA7, TMPRSS5, F12, PSMA6, SPINK2, PSMA3, ADAM9, PLAU, CAPN3, ZNF146, ANXA1, PSMC2, COPS7B, PSMB5, CTSB, PSMD10, PSMC3, ANXA3, PSMA4, ADAMDEC1, USP1, PSMB4, KIFAP3, PSMD4, HSP90AB1, PCSK1N, APP, CANX, CSTB, PSMB7, PSMC1, ILF2, ANXA5, PSMD1, CTSC, GDI2, CTSL1, SERPINE2, SLPI, PSMD2* und *S100A10;* und
(b) Vorhersagen, dass das Individuum eine Metastasierung in der Lunge entwickeln wird, wenn die Expression von einem oder mehreren der Gene gegenüber der Kontrolle erhöht ist.

5. Verfahren nach Anspruch 1, wobei die Probe Zellen oder Gewebe von einem Tumor des Individuums umfasst.

6. Kit zum Bestimmen in einer Probe eines Individuums mit Brustkrebs des Expressionsspiegels der Gene, die indikativ für eine Metastasierung von Krebs in dem Individuum im Gehirn, Knochen oder in der Lunge sind, wobei das Kit (i)
eine oder mehrere Komponenten zum Bestimmen des Expressionsspiegels der Gene umfasst, wobei die eine oder mehreren Komponenten aus der Gruppe bestehend aus einem DNA-Array-Chip, einem Oligonukleotid-Array-Chip, einem Protein-Array-Chip, einem Antikörper, einer Vielzahl von Proben ausgewählt werden; und (ii)
einen Satz von Primern umfasst, bestehend aus Primern für die Gene SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, T1MP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2 und PSME1.

7. Verwendung des Kits nach Anspruch 6 zum Bestimmen des Risikos von Metastasierung des Brustkrebses im Gehirn in einem Patienten mit Brustkrebs.

8. Kit nach Anspruch 6, ferner umfassend ein oder mehrere Reagenzien für RNA Extraktion; ein oder mehrere Enzyme zur Synthese von cDNA und cRNA; ein oder mehrere Reagenzien zur Hybridisierung für DNA-Chip, Oligonukleotid-Chip, Protein-Chip, Western-Blot, Sonden oder Primern; ein oder mehrere Reagenzien zum Binden der Antikörper an Proteine, die indikativ für eine Wiederkehr von Krebs sind; oder DNA-Fragmente von Kontrollgenen.

9. Satz von Primern, bestehend aus zumindest einem Primerpaar für jedes der Gene *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANX43, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* und *PSME1*.

10. Array, bestehend aus einem Substrat oder festen Träger und bestehend aus einer Sonde für jedes der Gene *SERPINB3, PI3, SPOCK2, PSMB6, PRSS22, CTSS, KLK10, GZMK, ADAMDEC1, ELANE, ILF2, PSMD11, PSMB4, S100A10, APP, COX4H, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* und *PSME1.*

11. Verfahren zum Voraussagen der Wahrscheinlichkeit eines Metastasen-freies Überlebens eines Individuums mit Brustkrebs, wobei das Verfahren Folgendes umfasst:
(a) Bestimmen des Expressionsspiegels der Gene *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, TPSG1, PSMD2* und *PSME1* in einer Probe des Individuums; und
(b) Vorhersagen der verminderten Wahrscheinlichkeit eines Metastasen-freien Überlebens, wenn eines der Gene gegenüber der Kontrolle erhöht ist.

12. Verfahren nach Anspruch 11, ferner umfassend das Normalisieren des Expressionsspiegels, um einen normalisierten Expressionsspiegel der Gene zu erhalten, wobei ein erhöhter normalisierter Expressionsspiegel von zumindest einem der Gene eine verminderte Wahrscheinlichkeit eines Metastasen-freies Überlebens ohne Metastasierung im Gehirn, Knochen oder in der Lunge anzeigt.

13. Verfahren nach einem der Ansprüche 11 oder 12, wobei die Probe Krebszellen oder -gewebe von einem Individuum mit Brustkrebs ist.

## Revendications

1. Méthode de détermination du risque pour un sujet atteint d'un cancer du sein de développer des métastases dudit cancer dans le cerveau, les os et/ou les poumons, ladite méthode comprenant :
(a) la détection dans un échantillon provenant du sujet du niveau d'expression des gènes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, CSTB, PSMB7, PSMC1, PSMD1, GDI2, SERPINE2, TPSG1, PSMD2* et *PSME1* ; et
(b)
(i) la prédiction que le sujet développera des métastases dans le cerveau, les os et les poumons si l'expression de *SLPI* est accrue par rapport à un témoin ;
(ii) la prédiction que le sujet développera des métastases dans le cerveau et les os si l'expression de *PSMD11* est accrue par rapport à un témoin ;
(iii) la prédiction que le sujet développera des métastases dans le cerveau et les poumons si l'expression d'un ou plusieurs des gènes *SERPINB3, PI3, ADAMDEC1, ILF2, PSMB4, APP, S100A10, CTSC, CTSL1, CANX, ANXA5, PSMD2* et *CTSB* est accrue par rapport à un témoin, mais ne développera pas de métastases dans le cerveau si *TPSG1* est accru par rapport à un témoin ;
(iv) la prédiction que le sujet développera des métastases dans les os et les poumons si l'expression d'un ou plusieurs des gènes *MME, PSMB3,* and *PSMD10* est accrue par rapport à un témoin ;
(v) la prédiction que le sujet développera des métastases dans le cerveau uniquement si l'expression d'un ou plusieurs des gènes *SPOCK2, PSMB6, PRSS22, CTSS, KLK10, GZMK, ELANE, COX4I1* et *TIMP2* est accrue par rapport à un témoin, mais ne développera pas de métastases dans le cerveau si *HNRPNPC* et/ou *SEPT2* est accrue par rapport à un témoin ;
(vi) la prédiction que le sujet développera des métastases dans les os si l'expression de SNRNP200 est accrue par rapport à un témoin, mais ne développera pas de métastases dans les os si l'un ou plusieurs des gènes *EIF3F, RPS6, GZMB, RPS13, RPS10, RPL21, RPL30, OAZ1, SerpinF2, RPL27, PRTN3, RPS5, F2, RPL14, PSMD13, RPL28, RPS27A, TIMPI, RPS11, USP4, IRPS24, CELA2B* et *RPL11* est accrue par rapport à un témoin ;
(vii) la prédiction que le sujet développera des métastases dans les poumons uniquement si l'expression d'un ou plusieurs des gènes *PSMA7, TMPRSS5, F12, PSMA6, SPINK2, PSMA3, ADAM9, PLAU, AAPN3, ZNF146, ANXA1, PSMC2, COPS7B, PSMB5, PSMC3, ANXA3, PSMA4, USP1, KIFAP3, PSMD4, HSP90AB1, PCSK1N, PSMB7, PSMC1, ILF2, PSMD1, GDI2* et *SERPINE2* est accrue par rapport à un témoin, mais ne développera pas de métastases dans les poumons si l'un ou plusieurs des gènes *SPINK4, ANXA9, PLAT, MMP24, CST3, EEF2, F7, F10, RPL9, PRSS23, MMP26* et *HTRA1* est accrue par rapport à un témoin.

2. Méthode de détermination du risque pour un sujet atteint d'un cancer du sein de développer des métastases dudit cancer dans le cerveau, ladite méthode comprenant :
(a) la détermination dans un échantillon provenant du sujet du niveau d'expression des gènes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2* et *CTSB* ; et
(b) la prédiction que le sujet développera des métastases dans le cerveau si l'expression de l'un ou plusieurs desdits gènes est accrue par rapport à un témoin.

3. Méthode de détermination du risque pour un sujet atteint d'un cancer du sein de développer des métastases dudit cancer dans les os, ladite méthode comprenant :
(a) la détermination dans un échantillon provenant du sujet du niveau d'expression des gènes *MME, PSMB3, SNRNP200, SLPI, PSMD10 et PSMD11* ; et
(b) la prédiction que le sujet développera des métastases dans les os si l'expression de l'un ou plusieurs desdits gènes est accrue par rapport à un témoin.

4. Méthode de détermination du risque pour un sujet atteint d'un cancer du sein de développer des métastases dans les poumons, ladite méthode comprenant :
(a) la détermination dans un échantillon provenant du sujet du niveau d'expression des gènes *MME, SERPINB3, PSMB3, PI3, PSMA7, TMPRSS5, F12, PSMA6, SPINK2, PSMA3, ADAM9, PLAU, CAPN3, ZNF146, ANXA1, PSMC2, COPS7B, PSMB5, CTSB, PSMD10, PSMC3, ANXA3, PSMA4, ADAMDEC1, USP1, PSMB4, KIFAP3, PSMD4, HSP90AB1, PCSK1N, APP, CANX, CSTB, PSMB7, PSMC1, ILF2, ANXA5*, *PSMD1, CTSC, GDI2, CTSL1, SERPINE2, SLPI, PSMD2* et *S100A10* ; et
(b) la prédiction que le sujet développera des métastases dans les poumons si l'expression de l'un ou plusieurs desdits gènes est accrue par rapport à un témoin.

5. Méthode selon la revendication 1, ledit échantillon comprenant des cellules ou un tissu d'une tumeur provenant du sujet.

6. Kit de détermination dans un échantillon à partir des niveaux d'expression d'un sujet atteint d'un cancer du sein de gènes indicateurs de métastases d'un cancer chez le sujet dans le cerveau, les os ou les poumons, le kit comprenant (i) un ou plusieurs composants pour déterminer les niveaux d'expression desdits gènes, lesdits un ou plusieurs composants étant choisis dans le groupe constitué par : une puce à ADN, une puce à ensemble oligonucléotidique, une puce à ensemble protéique, un anticorps, une pluralité de sondes ; et (ii) une série d'amorces se composant d'amorces pour les gènes SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, T1MP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2 et PSME1.

7. Utilisation du kit selon la revendication 6 pour déterminer le risque de métastases du cancer du sein dans le cerveau chez un patient atteint du cancer du sein.

8. Kit selon la revendication 6, comprenant en outre un ou plusieurs réactifs pour l'extraction d'ARN ; une ou plusieurs enzymes pour les synthèses d'ADNc et d'ARNc ; un ou plusieurs réactifs pour l'hybridation pour une puce à ADN, une puce à oligonucléotides, une puce à protéines, un transfert Western, des sondes ou des amorces ; un ou plusieurs réactifs pour la liaison desdits anticorps à des protéines indicatrices de la récidive d'un cancer ; ou des fragments d'ADN de gènes témoins.

9. Série d'amorces se composant d'au moins une paire d'amorces pour chacun des gènes *SERPINB3, PI3, SPOCK2, PSMB6, PRSS22, CTSS, KLK10, GZMK, ADAMDEC1, ELANE, ILF2, PSMD11, PSMB4, S100A100, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* et *PSME1*.

10. Matrice se composant d'un substrat ou d'un support solide et se composant d'une sonde pour chacun des gènes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, HNRPNPC, SEPT2, EIF3F, RPS5, GZMB, RPS13, RPS10, RPL21, RPL30, OAZ1, SerpinF2, RPL27, PRTN3, RPS6, F2, RPL14, PSMD13, RPL28, RPS27A, TIMP1, RPS11, USP4, RPS24, CELA2B, RPL11, SPINK4, ANXA9, PLAT, MMP24, CST3, EEF2, F7, F10, RPL9, PRSS23, MMP26, HTRA1, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, PSMD2* et *PSME1.*

11. Méthode permettant de prédire la probabilité de la survie sans métastase (MFS) d'un sujet atteint d'un cancer du sein, la méthode comprenant :
(a) la détection du niveau d'expression des gènes *SERPINB3, PI3, SPOCK2, PSMB6, PRSS22, CTSS, KLK10, GZMK, ADAMDEC1, ELANE, ILF2, PSMD11, PSMB4, S100A10, APP, COX4I1, CTSC, CTSL1, TIMP2, CANX, SLPI, ANXA5, PSMD2, CTSB, MME, PSMB3, SNRNP200, SLPI, PSMD10, SERPINB3, PSMA7, TMPRSS5, F12, PSMA6, SPINK2, PSMA3, ADAM9, PLAU, AAPN3, ZNF146, ANXA1, PSMC2, COPS7B, PSMB5, CTSB, PSMC3, ANXA3, PSMA4, USP1, KIFAP3, PSMD4, HSP90AB1, PCSK1N, CSTB, PSMB7, PSMC1, PSMD1, CTSC, GDI2, CTSL1, SERPINE2, TPSG1, PSMD2* et *PSME1* dans un échantillon provenant du sujet ; et
(b) la prédiction d'une probabilité réduite de survie sans métastase si l'un quelconque desdits gènes est accru par rapport à un témoin.

12. Méthode selon la revendication 11, comprenant en outre la normalisation desdits niveaux d'expression pour obtenir un niveau d'expression normalisé desdits gènes, un niveau d'expression normalisé accru d'au moins l'un desdits gènes indiquant une probabilité réduite de survie sans métastase sans métastases dans le cerveau, les os ou les poumons.

13. Méthode selon l'une quelconque des revendications 11 ou 12, ledit tissu cancéreux ou lesdites cellules cancéreuses échantillons provenant du sujet atteint d'un cancer du sein.
